(19) Europäisches Patentamt
European Patent Office
Office européen des brevets

(11) EP 2 215 253 B1

(12) **EUROPEAN PATENT SPECIFICATION**

(45) Date of publication and mention of the grant of the patent:
**27.04.2016 Bulletin 2016/17**

(21) Application number: **08834363.7**

(22) Date of filing: **26.09.2008**

(51) Int Cl.:
*C12Q 1/68* (2006.01) *G06F 19/18* (2011.01)

(86) International application number:
**PCT/US2008/078035**

(87) International publication number:
**WO 2009/042975 (02.04.2009 Gazette 2009/14)**

(54) **METHOD AND COMPUTER SYSTEM FOR CORRELATING GENOTYPE TO PHENOTYPE USING POPULATION DATA**

VERFAHREN UND KOMPUTERSYSTEM ZUR KORRELATION VON GENTOYPEN UND PHENOTYPEN UNTER VERWENDUNG VON POPULATIONSDATEN

PROCÉDÉ ET SYSTÈME INFORMATIQUE POUR LA CORRELATION DES GENOTYPES À UN PHENOTYP À L'AIDE DE DONNÉES DES POPULATIONS

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **26.09.2007 US 975495 P**

(43) Date of publication of application:
**11.08.2010 Bulletin 2010/32**

(73) Proprietor: **Navigenics, Inc.**
**Redwood Shores, CA 94065 (US)**

(72) Inventors:
- **STEPHAN, Dietrich, A.**
  **Phoenix, AZ 85012 (US)**
- **WESSEL, Jennifer**
  **San Francisco, CA 94107 (US)**
- **CARGILL, Michele**
  **Orinda, CA 94563 (US)**
- **HALPERIN, Eran**
  **Berkeley, CA 94708 (US)**

(74) Representative: **Vossius & Partner**
**Patentanwälte Rechtsanwälte mbB**
**Siebertstrasse 3**
**81675 München (DE)**

(56) References cited:
**WO-A1-96/32502** **US-A1- 2002 187 474**
**US-A1- 2003 135 096** **US-A1- 2003 219 776**
**US-A1- 2005 272 054** **US-A1- 2006 051 763**

- **LO ET AL: "GABRB2 Association with Schizophrenia: Commonalities and Differences Between Ethnic Groups and Clinical Subtypes", BIOLOGICAL PSYCHIATRY, ELSEVIER SCIENCE, NEW YORK, NY, US, vol. 61, no. 5, 16 February 2007 (2007-02-16), pages 653-660, XP005894531, ISSN: 0006-3223, DOI: DOI:10.1016/J.BIOPSYCH.2006.05.003**
- **V. R. RAO ET AL: "Single nucleotide polymorphisms in alcohol dehydrogenase genes among some Indian populations", AMERICAN JOURNAL OF HUMAN BIOLOGY, vol. 19, no. 3, 1 May 2007 (2007-05-01), pages 338-344, XP55003384, ISSN: 1042-0533, DOI: 10.1002/ajhb.20589**
- **Katrina A.B. Goddard ET AL: "Linkage Disequilibrium and Allele-Frequency Distributions for 114 Single-Nucleotide Polymorphisms in Five Populations", Am. J. Hum. Genet., 1 January 2000 (2000-01-01), pages 216-234, XP55003379, DOI: 10.1086/302727 Retrieved from the Internet: URL:http://www.sciencedirect.com/science?_ob=MImg&_imagekey=B8JDD-4RH3CKX-W-F&_cdi=4 3612&_user=987766&_pii=S0002929707622496&_ origin=&_coverDate=01%2F31%2F2000&_sk=99 93 39998&view=c&wchp=dGLbVzW-zSkzV&md5=1 668a5 90cb9a8e313d3b410ac53bfc4f&ie=/sdarticle.p df [retrieved on 2011-07-21]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- ROSS LAZARUS ET AL: "Single-Nucleotide Polymorphisms in the Interleukin-10 Gene: Differences in Frequencies, Linkage Disequilibrium Patterns, and Haplotypes in Three United States Ethnic Groups", GENOMICS, vol. 80, no. 2, 1 August 2002 (2002-08-01) , pages 223-228, XP55003703, ISSN: 0888-7543, DOI: 10.1006/geno.2002.6820
- PORNLADA NUCHNOI ET AL: "Linkage disequilibrium structure of the 5q31-33 region in a Thai population", JOURNAL OF HUMAN GENETICS, SPRINGER-VERLAG, TO, vol. 53, no. 9, 24 June 2008 (2008-06-24), pages 850-856, XP019593585, ISSN: 1435-232X

## Description

### BACKGROUND

Sequencing of the human genome and other recent developments in human genomics has revealed that the genomic makeup between any two humans can be over 99.9% similarity. The relatively small number of variations in DNA between individuals gives rise to differences in phenotypic traits, and is related to many human diseases, susceptibility to various diseases, and response to treatment of disease. Variations in DNA between individuals occur in both coding and non-coding regions, and include changes in bases at a particular locus in genomic DNA sequences, as well as insertions and deletions of DNA. Changes that occur at single base positions in the genome are referred to as single nucleotide polymorphisms, or "SNPs."

While SNPs are relatively rare in the human genome, they account for a majority of DNA sequence variations between individuals, occurring approximately once every 1,200 base pairs in the human genome (see International HapMap Project, www.hapmap.org). As more human genetic information becomes available, the complexity of SNPs is beginning to be understood. In turn, the occurrences of SNPs in the genome are becoming correlated to the presence of and/or susceptibility to various diseases and conditions.

As these correlations and other advances in human genetics are being made, medicine and personal health in general are moving toward a customized approach in which a patient will make appropriate medical and other choices in consideration of his or her genomic information, among other factors. An important factor that may affect considerations is an individual's ancestral data (ancestry) or ethnicity. For example, different populations may have different linkage disequilibrium patterns due to various possible reasons such as variation in recombination rates, selection pressure, or population bottleneck. Thus, if a study has been done on population A, yielding a specific odds ratio in that population for a genetic variation correlated with a phenotype, the same odds ratio cannot be assumed in population B. Thus, there is a need to provide individuals and their care-givers with information specific to the individual's personal genome, incorporating ancestral data, toward providing personalized medical and other decisions.

US 2006/051763 discloses that three genes which are associated with prostate cancer are sequenced in people from different populations and that haplotypes (i.e. LD patterns) are estimated with major ethnic groups using the software PHASE.

LO ET AL: "GABRB2 Association with Schizophrenia: Commonalities and Differences Between Ethnic Groups and Clinical Subtypes", BIOLOGICAL PSYCHIATRY, vol. 61(5), (2007-02-16), pages 653-660 discloses a study of determining the association of the gene GABRB2 with schizophrenia. The study encompasses determination of LD patterns using the Tagger program in both a German and a Japanese population. Differences and similarities between the LD patterns are found. The tagging SNPs are allocated to each LD bin. The similarity in LD blocks allows the authors to predict that the SNPs in GABRB2 are associated with schizophrenia.

### SUMMARY

The present disclosure provides a method of assessing genotype correlations to a phenotype of an individual comprising: a) obtaining a genetic sample of the individual, b) generating a genomic profile for the individual, c) determining the individual's genotype correlations with phenotypes by comparing the individual's genomic profile to a current database of human genotype correlations with phenotypes, d) reporting the results from step c) to the individual or a health care manager of the individual, e) updating the database of human genotype correlations with an additional human genotype correlation as the additional human genotype correlation becomes known, f) updating the individual's genotype correlations by comparing the individual's genomic profile from step c) or a portion thereof to the additional human genotype correlation and determining an additional genotype correlation of the individual, and g) reporting the results from step f) to the individual or the health care manager of the individual.

The present disclosure further provides a business method of assessing genotype correlations of an individual comprising: a) obtaining a genetic sample of the individual; b) generating a genomic profile for the individual; c) determining the individual's genotype correlations by comparing the individual's genomic profile to a database of human genotype correlations; d) providing results of the determining of the individual's genotype correlations to the individual in a secure manner; e) updating the database of human genotype correlations with an additional human genotype correlation as the additional human genotype correlation becomes known; f) updating the individual's genotype correlations by comparing the individual's genomic profile or a portion thereof to the additional human genotype correlation and determining an additional genotype correlation of the individual; and g) providing results of the updating of the individual's genotype correlations to the individual of the health care manager of the individual.

Another aspect of the present disclosure is a method generating a phenotype profile for an individual comprising: a) providing a rule set comprising rules, each rule indicating a correlation between at least one genotype and at least one phenotype, b) providing a data set comprising genomic profiles of each of a plurality of individuals, wherein each genomic profile comprises a plurality of genotypes; c) periodically updating the rule set with at least one new rule, wherein the at least one new rule indicates a correlation between a genotype and a phenotype not previously correlated with each other in the rule set; d) applying each new rule to the genomic profile of at least one of the individuals, thereby correlating at least one genotype with at least one phenotype for the individual, and optionally, e) generating a report comprising the phenotype profile of the individual.

The present disclosure also provides a system comprising a) a rule set comprising rules, each rule indicating a correlation between at least one genotype and at least one phenotype; b) code that periodically updates the rule set with at least one new rule, wherein the at least one new rule indicates a correlation between a genotype and a phenotype not previously correlated with each other in the rule set; c) a database comprising genomic profiles of a plurality of individuals; d) code that applies the rule set to the genomic profiles of individuals to determine phenotype profiles for the individuals; and e) code that generates reports for each individual.

The present disclosure further provides a method of assessing genotype correlations of an individual comprising: (a) comparing (i) a first linkage disequilibrium (LD) pattern comprising a genetic variation correlated with a phenotype, wherein the first LD pattern is of a first population of individuals; and, (ii) a second LD pattern comprising the genetic variation, wherein the second LD pattern is of a second population of individuals; (b) determining a probability of the genetic variation being correlated with the phenotype in said second population from said comparing in (a); (c) assessing a genotype correlation of said phenotype from a genomic profile of the individual comprising using the probability of step (b); and, (d) reporting results comprising the genotype correlation from step c) to the individual or a health care manager of the individual. In some embodiments, the methods further comprise (e) updating said results with additional genetic variations.

The probability can be an odds ratio (OR), wherein the OR can be derived from a known OR. For example, the known OR can be for the genetic variation correlated with the phenotype for the first population, such as an OR published for a genetic variation, such as a SNP, in a scientific journal. In some embodiments, the first population and the second population have similar LD patterns. Also provided herein is a method of assessing genotype correlations of an individual comprising: (a) determining a causal genetic variation probability for each of a plurality of genetic variations in a first population of individuals; (b) identifying each of said probability in step (a) as a probability for each of said plurality of genetic variations in a second population of individuals; (c) assessing a genotype correlation from a genomic profile of the individual comprising using the probability of step (b); and, (d) reporting results comprising the genotype correlation from step (c) to the individual or a health care manager of the individual. In some embodiments, the methods further comprise (e) updating said results with additional genetic variations.

The known genetic variation, such as a SNP, can be a genetic variation with an OR published in a scientific journal. The probability can be an odds ratio (OR) and each of the genetic variations of step (a) can be proximal to a known genetic variation correlated to a phenotype in the first population. For example, each of the genetic variations can be in linkage disequilibrium to the known genetic variation.

In some embodiments of the methods and systems disclosed herein, the genotype correlation is reported as a GCI score. The second population is typically of an ancestry different from the first population, and the individual is of an ancestry of the second population. In some embodiments, the causal genetic variation is unknown. The genetic variation can be a single nucleotide polymorphism (SNP).

Another aspect of the present disclosure is transmission over a network, in a secure or non-secure manner, the methods and systems described above. The reporting can be through an on-line portal, by paper or by e-mail. The genomic profile used can be generated and from a genetic sample. A third party can generate the genomic profile, obtain the genetic sample, or both obtain the sample and generate the genomic profile. The genetic sample can be DNA or RNA and obtained from a biological sample selected from the group consisting of: blood, hair, skin, saliva, semen, urine, fecal material, sweat, and buccal sample. The genomic profile can be deposited into a secure database or vault. Furthermore the genomic profile can be a single nucleotide polymorphism profile, and in some embodiments, the genomic profile can comprise truncations, insertions, deletions, or repeats. The genomic profile can be generated by using a high density DNA microarray, RT-PCR, DNA sequencing, or a combination of techniques.

The method relate to the populations comprising anv of the HapMap populations (YRI,CEU,CHB,JPT,ASW,CHD,GIH,LWK,MEX,MKK,TSI), or to any other population such as, but not limited to African American, Caucasian, Ashkenazi Jewish, Sepharadic Jewish, Indian, Pacific islanders, middle eastern, Druze, Bedouins, south Europeans, Scandinavians, eastern Europeans, North Africans, Basques, West Africans, or East Africans.

**BRIEF DESCRIPTION OF THE DRAWINGS**

FIG. 1 is a flow chart illustrating aspects of the method herein.
FIG. 2 is an example of a genomic DNA quality control measure.

FIG. 3 is an example of a hybridization quality control measure.

FIG. 4 are tables of representative genotype correlations from published literature with test SNPs and effect estimates. A-I) represents single locus genotype correlations; J) respresents a two locus genotype correlation; K) represents a three locus genotype correlation; L) is an index of the ethnicity and country abbreviations used in A-K; M) is an index of the abbreviations of the Short Phenotype Names in A-K, the heritability, and the references for the heritability.

FIG. 5A-J are tables of representative genotype correlations with effect estimates.

FIG. 6A-F are tables of representative genotype correlations and estimated relative risks.

FIG. 7 is a sample report.

FIG. 8 is a schematic of a system for the analysis and transmission of genomic and phenotype profiles over a network.

FIG. 9 is a flow chart illustrating aspects of the business method herein.

FIG. 10 is a schematic of a published SNP in CEU (Caucasian ancestry/ethnicity) with a specific odds ratio cannot be assumed to be the same in a different population of a different ancestral background, YRI (Yoruban ancestry/ethnicity see HapMap project (http://hapmap.org/hapmappopulations.html.en)).

## DETAILED DESCRIPTION

The present disclosure provides methods and systems for generating phenotype profiles based on a stored genomic profile of an individual or group of individuals, and for readily generating original and updated phenotype profiles based on the stored genomic profiles. Genomic profiles are generated by determining genotypes from biological samples obtained from individuals. Biological samples obtained from individuals may be any sample from which a genetic sample may be derived. Samples may be from buccal swabs, saliva, blood, hair, or any other type of tissue sample. Genotypes may then be determined from the biological samples. Genotypes may be any genetic variant or biological marker, for example, single nucleotide polymorphisms (SNPs), haplotypes, or sequences of the genome. The genotype may be the entire genomic sequence of an individual. The genotypes may result from high-throughput analysis that generates thousands or millions of data points, for example, microarray analysis for most or all of the known SNPs. In other embodiments, genotypes may also be determined by high throughput sequencing.

The genotypes form a genomic profile for an individual. The genomic profile is stored digitally and is readily accessed at any point of time to generate phenotype profiles. Phenotype profiles are generated by applying rules that correlate or associate genotypes with phenotypes. Rules can be made based on scientific research that demonstrates a correlation between a genotype and a phenotype. The correlations may be curated or validated by a committee of one or more experts. By applying the rules to a genomic profile of an individual, the association between an individual's genotype and a phenotype may be determined. The phenotype profile for an individual will have this determination. The determination may be a positive association between an individual's genotype and a given phenotype, such that the individual has the given phenotype, or will develop the phenotype. Alternatively, it may be determined that the individual does not have, or will not develop, a given phenotype. In other embodiments, the determination may be a risk factor, estimate, or a probability that an individual has, or will develop a phenotype.

The determinations may be made based on a number of rules, for example, a plurality of rules may be applied to a genomic profile to determine the association of an individual's genotype with a specific phenotype. The determinations may also incorporate factors that are specific to an individual, such as ethnicity, gender, lifestyle, age, environment, family medical history, personal medical history, and other known phenotypes. The incorporation of the specific factors may be by modifying existing rules to encompass these factors. Alternatively, separate rules may be generated by these factors and applied to a phenotype determination for an individual after an existing rule has been applied.

Phenotypes may include any measurable trait or characteristic, such as susceptibility to a certain disease or response to a drug treatment. Other phenotypes that may be included are physical and mental traits, such as height, weight, hair color, eye color, sunburn susceptibility, size, memory, intelligence, level of optimism, and general disposition. Phenotypes may also include genetic comparisons to other individuals or organisms. For example, an individual may be interested in the similarity between their genomic profile and that of a celebrity. They may also have their genomic profile compared to other organisms such as bacteria, plants, or other animals.

In another aspect of the disclosure information about the association of multiple genetic markers with one or more diseases or conditions is combined and analyzed to produce a Genetic Composite Index (GCI) score (such as described in PCT Publication No. WO2008/067551). This score incorporates known risk factors, as well as other information and assumptions such as the allele frequencies and the prevalence of a disease. The GCI can be used to qualitatively estimate the association of a disease or a condition with the combined effect of a set of Genetic markers. The GCI score can be used to provide people not trained in genetics with a reliable (i.e., robust), understandable, and/or intuitive sense of what their individual risk of a disease is compared to a relevant population based on current scientific research. The GCI score may be used to generate GCI Plus scores, as described in PCT Publication No. WO2008/067551. The GCI Plus score may contain all the GCI assumptions, including risk (such as lifetime risk), age-defined prevalence, and/or age-defined incidence of the condition. The lifetime risk for the individual may then be calculated as a GCI Plus score which is proportional to the individual's GCI score divided by the average GCI score. The average GCI score may be determined from a group of individuals of similar ancestral background, for example a group of Caucasians, Asians, East Indians, or other group with a common ancestral background. Groups may comprise of at least 5, 10, 15, 20, 25, 30, 35, 40, 45, 50, 55, or 60 individuals. In some embodiments, the average may be determined from at least 75, 80, 95, or 100 individuals. The GCI Plus score may be determined by determining the GCI score for an individual, dividing the GCI score by the average relative risk and multiplying by the lifetime risk for a condition or phenotype. For example, using data from PCT Publication No. WO2008/067551, such as FIG. 22 and/or FIG. 25 with information in FIG. 24 to calculate GCI Plus scores such as in FIG. 19.

The present disclosure encompasses using the GCI score as described herein, and one of ordinary skill in the art will readily recognize the use of GCI Plus scores or variations thereof, in place of GCI scores as described herein. In one embodiment a GCI score is generated for each disease or condition of interest. These GCI scores may be collected to form a risk profile for an individual. The GCI scores may be stored digitally so that they are readily accessible at any point of time to generate risk profiles. Risk profiles may be broken down by broad disease classes, such as cancer, heart disease, metabolic disorders, psychiatric disorders, bone disease, or age on-set disorders. Broad disease classes may be further broken down into subcategories. For example for a broad class such as a cancer, sub-categories of cancer may be listed such as by type (sarcoma, carcinoma or leukemia, etc.) or by tissue specificity (neural, breast, ovaries, testes, prostate, bone, lymph nodes, pancreas, esophagus, stomach, liver, brain, lung, kidneys, etc.).

In another embodiment a GCI score is generated for an individual, which provides them with easily comprehended information about the individual's risk of acquiring or susceptibility to at least one disease or condition. In one embodiment multiple GCI scores are generated for different diseases or conditions. In another embodiment at least one GCI score is accessible by an on-line portal. Alternatively, at least one GCI score may be provided in paper form, with subsequent updates also provided in paper form. In one embodiment access to at least one GCI score is provided to a subscriber, which is an individual who subscribes to the service. In an alternative embodiment access is provided to non-subscribers, wherein they may have limited access to at least one of their GCI scores, or they may have an initial report on at least one of their GCI scores generated, but updated reports will be generated only with purchase of a subscription. In another embodiment health care managers and providers, such as caregivers, physicians, and genetic counselors may also have access to at least one of an individual's GCI scores.

Together, the collection of correlated phenotypes determined for an individual comprises the phenotype profile for the individual. The phenotype profile may be accessible by an on-line portal. Alternatively, the phenotype profile as it exists at a certain time may be provided in paper form, with subsequent updates also provided in paper form. The phenotype profile may also be provided by an on-line portal. The on-line portal may optionally be a secure on-line portal. Access to the phenotype profile may be provided to a subscriber, which is an individual who subscribes to the service that generates rules on correlations between phenotypes and genotypes, determines the genomic profile of an individual, applies the rules to the genomic profile, and generates a phenotype profile of the individual. Access may also be provided to non-subscribers, wherein they may have limited access to their phenotype profile and/or reports, or may have an initial report or phenotype profile generated, but updated reports will be generated only with purchase of a subscription. Health care managers and providers, such as caregivers, physicians, and genetic counselors may also have access to the phenotype profile.

In another aspect of the disclosure the genomic profile may be generated for subscribers and non-subscribers and stored digitally but access to the phenotype profile and reports may be limited to subscribers. In another variation, both subscribers and non-subscribers may access their genotype and phenotype profiles, but have limited access, or have a limited report generated for non-subscribers, whereas subscribers have full access and may have a full report generated. In another embodiment, both subscribers and non-subscribers may have full access initially, or full initial reports, but only subscribers may access updated reports based on their stored genomic profile.

There may also be a basic subscription model. A basic subscription may provide a phenotype profile where the subscriber may choose to apply all existing rules to their genomic profile, or a subset of the existing rules, to their genomic profile. For example, they may choose to apply only the rules for disease phenotypes that are actionable. The basic subscription may have different levels within the subscription class. For example, different levels may be dependent on the number of phenotypes a subscriber wants correlated to their genomic profile, or the number of people that may access their phenotype profile. Another level of basic subscription may be to incorporate factors specific to an individual, such as already known phenotypes such as age, gender, or medical history, to their phenotype profile.

Still another level of the basic subscription may allow an individual to generate at least one GCI score for a disease or condition. A variation of this level may further allow an individual to specify for an automatic update of at least one GCI score for a disease or condition to be generated if their is any change in at least one GCI score due to changes in the analysis used to generate at least one GCI score. In some embodiments the individual may be notified of the automatic update by email, voice message, text message, mail delivery, or fax.

Subscribers may also generate reports that have their phenotype profile as well as information about the phenotypes, such as genetic and medical information about the phenotype. For example, the prevalence of the phenotype in the population, the genetic variant that was used for the correlation, the molecular mechanism that causes the phenotype, therapies for the phenotype, treatment options for the phenotype, and preventative actions, may be included in the report. In other embodiments, the reports may also include information such as the similarity between an individual's genotype and that of other individuals, such as celebrities or other famous people. The information on similarity may be, but are not limited to, percentage homology, number of identical variants, and phenotypes that may be similar. These reports may further contain at least one GCI score.

The report may also provide links to other sites with further information on the phenotypes, links to on-line support groups and message boards of people with the same phenotype or one or more similar phenotypes, links to an on-line genetic counselor or physician, or links to schedule telephonic or in-person appointments with a genetic counselor or physician, if the report is accessed on-line. If the report is in paper form, the information may be the website location of the aforementioned links, or the telephone number and address of the genetic counselor or physician. The subscriber may also choose which phenotypes to include in their phenotype profile and what information to include in their report. The phenotype profile and reports may also be accessible by an individual's health care manager or provider, such as a caregiver, physician, psychiatrist, psychologist, therapist, or genetic counselor. The subscriber may be able to choose whether the phenotype profile and reports, or portions thereof, are accessible by such individual's health care manager or provider.

The present disclosure may also include a premium level of subscription. The premium level of subscription maintains their genomic profile digitally after generation of an initial phenotype profile and report, and provides subscribers the opportunity to generate phenotype profiles and reports with updated correlations from the latest research. In another embodiment, subscribers have the opportunity to generate risk profile and reports with updated correlations from the latest research. As research reveals new correlations between genotypes and phenotypes, disease or conditions, new rules will be developed based on these new correlations and can be applied to the genomic profile that is already stored and being maintained. The new rules may correlate genotypes not previously correlated with any phenotype, correlate genotypes with new phenotypes, or modify existing correlations, or provide the basis for adjustment of a GCI score based on a newly discovered association between a genotype and disease or condition. Subscribers may be informed of new correlations via e-mail or other electronic means, and if the phenotype is of interest, they may choose to update their phenotype profile with the new correlation. Subscribers may choose a subscription where they pay for each update, or for a number of updates or an unlimited number of updates for a designated time period (e.g. three months, six months, or one year). Another subscription level may be where a subscriber has their phenotype profile or risk profile automatically updated, instead of where the individual chooses when to update their phenotype profile or risk profile, whenever a new rule is generated based on a new correlation.

In another aspect of the subscription, subscribers may refer non-subscribers to the service that generates rules on correlations between phenotypes and genotypes, determines the genomic profile of an individual, applies the rules to the genomic profile, and generates a phenotype profile of the individual. Referral by a subscriber may give the subscriber a reduced price on subscription to the service, or upgrades to their existing subscriptions. Referred individuals may have free access for a limited time or have a discounted subscription price.

Phenotype profiles and reports as well as risk profiles and reports may be generated for individuals that are human and non-human. For example, individuals may include other mammals, such as bovines, equines, ovines, canines, or felines. Subscribers, as used herein, are human individuals who subscribe to a service by purchase or payment for one or more services. Services may include, but are not limited to, one or more of the following: having their or another individual's, such as the subscriber's child or pet, genomic profile determined, obtaining a phenotype profile, having the phenotype profile updated, and obtaining reports based on their genomic and phenotype profile.

In another aspect of the disclosure, "field-deployed" mechanisms may be gathered from individuals to generate phenotype profiles for individuals. In preferred embodiments, an individual may have an initial phenotype profile generated based on genetic information. For example, an initial phenotype profile is generated that includes risk factors for different phenotypes as well as suggested treatments or preventative measures. For example, the profile may include information on available medication for a certain condition, and/or suggestions on dietary changes or exercise regimens. The individual may choose to see, or contact via a web portal or phone call, a physician or genetic counselor, to discuss their phenotype profile. The individual may decide to take a certain course of action, for example, take specific medications, change their diet, etc.

The individual may then subsequently submit biological samples to assess changes in their physical condition and possible change in risk factors. Individuals may have the changes determined by directly submitting biological samples to the facility (or associated facility, such as a facility contracted by the entity generating the genetic profiles and phenotype profiles us) that generates the genomic profiles and phenotype profiles. Alternatively, the individuals may use a "field-deployed" mechanism, wherein the individual may submit their saliva, blood, or other biological sample into a detection device at their home, analyzed by a third party, and the data transmitted to be incorporated into another phenotype profile. For example, an individual may have received an initial phenotype report based on their genetic data reporting the individual having an increased lifetime risk of myocardial infarction (MI). The report may also have suggestions on preventative measures to reduce the risk of MI, such as cholesterol lowering drugs and change in diet. The individual may choose to contact a genetic counselor or physician to discuss the report and the preventative measures and decides to change their diet. After a period of being on the new diet, the individual may see their personal physician to have their cholesterol level measured. The new information (cholesterol level) may be transmitted (for example, via the Internet) to the entity with the genomic information, and the new information used to generate a new phenotype profile for the individual, with a new risk factor for myocardial infarction, and/or other conditions.

The individual may also use a "field-deployed" mechanism, or direct mechanism, to determine their individual response to specific medications. For example, an individual may have their response to a drug measured, and the information may be used to determine more effective treatments. Measurable information include, but are not limited to, metabolite levels, glucose levels, ion levels (for example, calcium, sodium, potassium, iron), vitamins, blood cell counts, body mass index (BMI), protein levels, transcript levels, heart rate, etc., can be determined by methods readily available and can be factored into an algorithm to combine with initial genomic profiles to determine a modified overall risk estimate score.

The term "biological sample" refers to any biological sample from which a genetic sample of an individual can be isolated.

As used herein, a "genetic sample" refers to DNA and/or RNA obtained or derived from an individual.

As used herein, the term "genome" is intended to mean the full complement of chromosomal DNA found within the nucleus of a human cell. The term "genomic DNA" refers to one or more chromosomal DNA molecules occurring naturally in the nucleus of a human cell, or a portion of the chromosomal DNA molecules.

The term "genomic profile" refers to a set of information about an individual's genes, such as the presence or absence of specific SNPs or mutations. Genomic profiles include the genotypes of individuals. Genomic profiles may also be substantially the complete genomic sequence of an individual. In some embodiments, the genomic profile may be at least 60%, 80%, or 95% of the complete genomic sequence of an individual. The genomic profile may be approximately 100% of the complete genomic sequence of an individual. In reference to a genomic profile, "a portion thereof" refers to the genomic profile of a subset of the genomic profile of an entire genome.

The term "genotype" refers to the specific genetic makeup of an individual's DNA. The genotype may include the genetic variants and markers of an individual. Genetic markers and variants may include nucleotide repeats, nucleotide insertions, nucleotide deletions, chromosomal translocations, chromosomal duplications, or copy number variations. Copy number variation may include microsatellite repeats, nucleotide repeats, centromeric repeats, or telomeric repeats. The genotypes may also be SNPs, haplotypes, or diplotypes. A haplotype may refer to a locus or an allele. A haplotype is also referred to as a set of single nucleotide polymorphisms (SNPs) on a single chromatid that are statistically associated. A diplotype is a set of haplotypes.

The term single nucleotide polymorphism or "SNP" refers to a particular locus on a chromosome which exhibits variability such as at least one percent (1%) with respect to the identity of the nitrogenous base present at such locus within the human population For example, where one individual might have adenosine (A) at a particular nucleotide position of a given gene, another might have cytosine (C), guanine (G), or thymine (T) at this position, such that there is a SNP at that particular position.

As used herein, the terminology "SNP genomic profile" refers to the base content of a given individual's DNA at SNP sites throughout the individual's entire genomic DNA sequence. A "SNP profile" can refer to an entire genomic profile, or may refer to a portion thereof, such as a more localized SNP profile which can be associated with a particular gene or set of genes.

The term "phenotype" is used to describe a quantitative trait or characteristic of an individual. Phenotypes include, but are not limited to, medical and non-medical conditions. Medical conditions include diseases and disorders. Phenotypes may also include physical traits, such as hair color, physiological traits, such as lung capacity, mental traits, such as memory retention, emotional traits, such as ability to control anger, ethnicity, such as ethnic background, ancestry, such as an individual's place of origin, and age, such as age expectancy or age of onset of different phenotypes. Phenotypes may also be monogenic, wherein it is thought that one gene may be correlated with a phenotype, or multigenic, wherein more than one gene is correlated with a phenotype.

A "rule" is used to define the correlation between a genotype and a phenotype. The rules may define the correlations by a numerical value, for example by a percentage, risk factor, or confidence score. A rule may incorporate the correlations of a plurality of genotypes with a phenotype. A "rule set" comprises more than one rule. A "new rule" may be a rule that indicates a correlation between a genotype and a phenotype for which a rule does not currently exist. A new rule may correlate an uncorrelated genotype with a phenotype. A new rule may also correlate a genotype that is already correlated with a phenotype to a phenotype it had not been previously correlated to. A "new rule" may also be an existing rule that is modified by other factors, including another rule. An existing rule may be modified due to an individual's known characteristics, such as ethnicity, ancestry, geography, gender, age, family history, or other previously determined phenotypes.

Use of "genotype correlation" herein refers to the statistical correlation between an individual's genotype, such as presence of a certain mutation or mutations, and the likelihood of being predisposed to a phenotype, such as a particular disease, condition, physical state, and/or mental state. The frequency with which a certain phenotype is observed in the presence of a specific genotype determines the degree of genotype correlation or likelihood of a particular phenotype. For example, as detailed herein, SNPs giving rise to the apolipoprotein E4 isoform are correlated with being predisposed to early onset Alzheimer's disease. Genotype correlations may also refer to correlations wherein there is not a predisposition to a phenotype, or a negative correlation. The genotype correlations may also represent an estimate of an individual to have a phenotype or be predisposed to have a phenotype. The genotype correlation may be indicated by a numerical value, such as a percentage, a relative risk factor, an effects estimate, or confidence score.

The term "phenotype profile" refers to a collection of a plurality of phenotypes correlated with a genotype or genotypes of an individual. Phenotype profiles may include information generated by applying one or more rules to a genomic profile, or information about genotype correlations that are applied to a genomic profile. Phenotype profiles may be generated by applying rules that correlate a plurality of genotypes with a phenotype. The probability or estimate may be expressed as a numerical value, such as a percentage, a numerical risk factor or a numerical confidence interval. The probability may also be expressed as high, moderate, or low. The phenotype profiles may also indicate the presence or absence of a phenotype or the risk of developing a phenotype. For example, a phenotype profile may indicate the presence of blue eyes, or a high risk of developing diabetes. The phenotype profiles may also indicate a predicted prognosis, effectiveness of a treatment, or response to a treatment of a medical condition.

The term risk profile refers to a collection of GCI scores for more than one disease or condition. GCI scores are based on analysis of the association between an individual's genotype with one or more diseases or conditions. Risk profiles may display GCI scores grouped into categories of disease. Further the Risk profiles may display information on how the GCI scores are predicted to change as the individual ages or various risk factors are adjusted. For example, the GCI scores for particular diseases may take into account the effect of changes in diet or preventative measures taken (smoking cessation, drug intake, double radical mastectomies, hysterectomies). The GCI scores may be displayed as a numerical measure, a graphical display, auditory feedback or any combination of the preceding.

As used herein, the term "on-line portal" refers to a source of information which can be readily accessed by an individual through use of a computer and internet website, telephone, or other means that allow similar access to information. The on-line portal may be a secure website. The website may provide links to other secure and non-secure websites, for example links to a secure website with the individual's phenotype profile, or to non-secure websites such as a message board for individuals sharing a specific phenotype.

The practice of the present disclosure may employ, unless otherwise indicated, conventional techniques and descriptions of molecular biology, cell biology, biochemistry, and immunology, which are within the skill of the art. Such conventional techniques include nucleic acid isolation, polymer array synthesis, hybridization, ligation, and detection of hybridization using a label. Specific illustrations of suitable techniques are exemplified and referenced herein. However, other equivalent conventional procedures can also be used. Other conventional techniques and descriptions can be found in standard laboratory manuals and texts such as Genome Analysis: A Laboratory Manual Series (Vols. I-IV), PCR Primer: A Laboratory Manual, Molecular Cloning: A Laboratory Manual (all from Cold Spring Harbor Laboratory Press); Stryer, L. (1995) Biochemistry (4th Ed.) Freeman, New York; Gait, "Oligonucleotide Synthesis: A Practical Approach" 1984, IRL Press, London, Nelson and Cox (2000); Lehninger, Principles of Biochemistry 3rd Ed., W.H. Freeman Pub., New York, N.Y.; and Berg et al. (2002) Biochemistry, 5th Ed., W.H. Freeman Pub., New York, N.Y..

The methods of the present disclosure involve analysis of an individual's genomic profile to provide the individual with molecular information relating to a phenotype. As detailed herein, the individual provides a genetic sample, from which a personal genomic profile is generated. The data of the individual's genomic profile is queried for genotype correlations by comparing the profile against a database of established and validated human genotype correlations. The database of established and validated genotype correlations may be from peer-reviewed literature and further judged by a committee of one or more experts in the field, such as geneticists, epidemiologists, or statisticians, and curated. In preferred embodiments, rules are made based on curated genotype correlations and are applied to an individual's genomic profile to generate a phenotype profile. Results of the analysis of the individual's genomic profile, phenotype profile, along with interpretation and supportive information, are provided to the individual of the individual's health care manager, to empower personalized choices for the individual's health care.

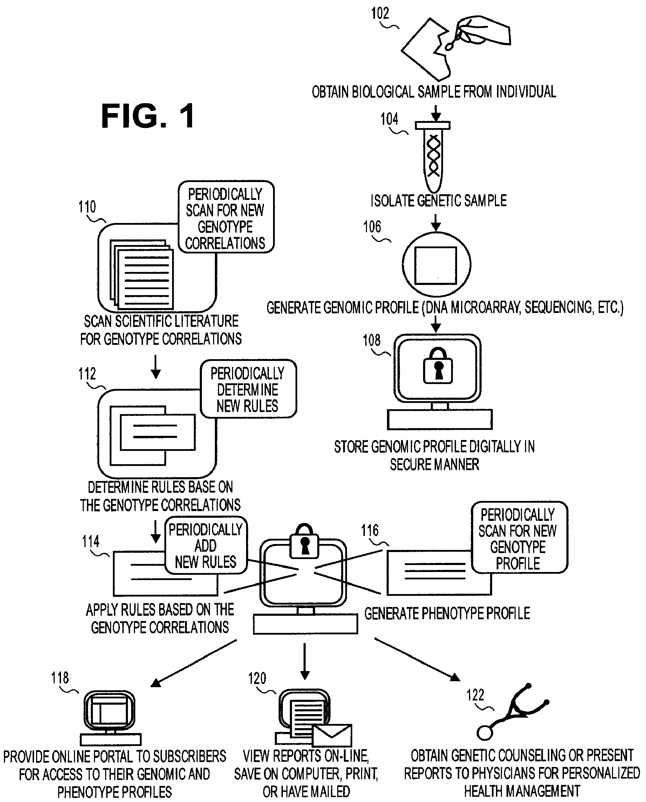

The method of the disclosure is detailed as in **FIG. 1,** where an individual's genomic profile is first generated. An individual's genomic profile will contain information about an individual's genes based on genetic variations or markers. Genetic variations are genotypes, which make up genomic profiles. Such genetic variations or markers include, but are not limited to, single nucleotide polymorphisms, single and/or multiple nucleotide repeats, single and/or multiple nucleotide deletions, microsatellite repeats (small numbers of nucleotide repeats with a typical 5-1,000 repeat units), di-nucleotide repeats, tri-nucleotide repeats, sequence rearrangements (including translocation and duplication), copy number variations (both loss and gains at specific loci), and the like. Other genetic variations include chromosomal duplications and translocations as well as centromeric and telomeric repeats.

Genotypes may also include haplotypes and diplotypes. In some embodiments, genomic profiles may have at least 100,000, 300,000, 500,000, or 1,000,000 genotypes. In some embodiments, the genomic profile may be substantially the complete genomic sequence of an individual. In other embodiments, the genomic profile is at least 60%, 80%, or 95% of the complete genomic sequence of an individual. The genomic profile may be approximately 100% of the complete genomic sequence of an individual. Genetic samples that contain the targets include, but are not limited to, unamplified genomic DNA or RNA samples or amplified DNA (or cDNA). The targets may be particular regions of genomic DNA that contain genetic markers of particular interest.

In step **102** of **FIG. 1,** a genetic sample of an individual is isolated from a biological sample of an individual. Such biological samples include, but are not limited to, blood, hair, skin, saliva, semen, urine, fecal material, sweat, buccal, and various bodily tissues. In some embodiments, tissues samples may be directly collected by the individual, for example, a buccal sample may be obtained by the individual taking a swab against the inside of their cheek. Other samples such as saliva, semen, urine, fecal material, or sweat, may also be supplied by the individual themselves. Other biological samples may be taken by a health care specialist, such as a phlebotomist, nurse or physician. For example, blood samples may be withdrawn from an individual by a nurse. Tissue biopsies may be performed by a health care specialist, and kits are also available to health care specialists to efficiently obtain samples. A small cylinder of skin may be removed or a needle may be used to remove a small sample of tissue or fluids.

Kits may be provided to individuals with sample collection containers for the individual's biological sample. The kit may also provide instructions for an individual to directly collect their own sample, such as how much hair, urine, sweat, or saliva to provide. The kit may also contain instructions for an individual to request tissue samples to be taken by a health care specialist. The kit may include locations where samples may be taken by a third party, for example kits may be provided to health care facilities who in turn collect samples from individuals. The kit may also provide return packaging for the sample to be sent to a sample processing facility, where genetic material is isolated from the biological sample in step **104.**

A genetic sample of DNA or RNA may be isolated from a biological sample according to any of several well-known biochemical and molecular biological methods, see, e.g., Sambrook, et al., Molecular Cloning: A Laboratory Manual (Cold Spring Harbor Laboratory, New York) (1989). There are also several commercially available kits and reagents for isolating DNA or RNA from biological samples, such as those available from DNA Genotek, Gentra Systems, Qiagen, Ambion, and other suppliers. Buccal sample kits are readily available commercially, such as the MasterAmp™ Buccal Swab DNA extraction kit from Epicentre Biotechnologies, as are kits for DNA extraction from blood samples such as Extract-N-Amp™ from Sigma Aldrich. DNA from other tissues may be obtained by digesting the tissue with proteases and heat, centrifuging the sample, and using phenolchloroform to extract the unwanted materials, leaving the DNA in the aqueous phase. The DNA can then be further isolated by ethanol precipitation.

In a preferred embodiment, genomic DNA is isolated from saliva. For example, using DNA self collection kit technology available from DNA Genotek, an individual collects a specimen of saliva for clinical processing. The sample conveniently can be stored and shipped at room temperature. After delivery of the sample to an appropriate laboratory for processing, DNA is isolated by heat denaturing and protease digesting the sample, typically using reagents supplied by the collection kit supplier at 50°C for at least one hour. The sample is next centrifuged, and the supernatant is ethanol precipitated. The DNA pellet is suspended in a buffer appropriate for subsequent analysis.

In another embodiment, RNA may be used as the genetic sample. In particular, genetic variations that are expressed can be identified from mRNA. The term "messenger RNA" or "mRNA" includes, but is not limited to pre-mRNA transcript(s), transcript processing intermediates, mature mRNA(s) ready for translation and transcripts of the gene or genes, or nucleic acids derived from the mRNA transcript(s). Transcript processing may include splicing, editing and degradation. As used herein, a nucleic acid derived from an mRNA transcript refers to a nucleic acid for whose synthesis the mRNA transcript or a subsequence thereof has ultimately served as a template. Thus, a cDNA reverse transcribed from an mRNA, a DNA amplified from the cDNA, an RNA transcribed from the amplified DNA, etc., are all derived from the mRNA transcript. RNA can be isolated from any of several bodily tissues using methods known in the art, such as isolation of RNA from unfractionated whole blood using the PAXgene™ Blood RNA System available from PreAnalytiX. Typically, mRNA will be used to reverse transcribe cDNA, which will then be used or amplified for gene variation analysis.

Prior to genomic profile analysis, a genetic sample will typically be amplified, either from DNA or cDNA reverse transcribed from RNA. DNA can be amplified by a number of methods, many of which employ PCR. See, for example, PCR Technology: Principles and Applications for DNA Amplification (Ed. H. A. Erlich, Freeman Press, NY, N.Y., 1992); PCR Protocols: A Guide to Methods and Applications (Eds. Innis, et al., Academic Press, San Diego, Calif., 1990); Mattila et al., Nucleic Acids Res. 19,4967 (1991); Eckert et al., PCR Methods and Applications 1, 17 (1991); PCR (Eds. McPherson et al., IRL Press, Oxford); and U.S. Pat. Nos. 4,683,202, 4,683,195, 4,800,159 4,965,188, and 5,333,675.

Other suitable amplification methods include the ligase chain reaction (LCR) (for example, Wu and Wallace, Genomics 4, 560 (1989*),* Landegren et al., Science 241, 1077 (1988*) and* Barringer et al. Gene 89:117 (1990*)*), transcription amplification (Kwoh et al., Proc. Natl. Acad. Sci. USA 86:1173-1177 (1989*)* and WO88/10315), self-sustained sequence replication (Guatelli et al., Proc. Nat. Acad. Sci. USA, 87:1874-1878 (1990*)* and WO90/06995), selective amplification of target polynucleotide sequences (U.S. Pat. No. 6,410,276), consensus sequence primed polymerase chain reaction (CP-PCR) (U.S. Pat. No. 4,437,975), arbitrarily primed polymerase chain reaction (AP-PCR) (U.S. Pat. Nos. 5,413,909, 5,861,245) nucleic acid based sequence amplification (NABSA), rolling circle amplification (RCA), multiple displacement amplification (MDA) (U.S. Pat. Nos. 6,124,120 and 6,323,009) and circle-to-circle amplification (C2CA) *(*Dahl et al. Proc. Natl. Acad. Sci 101:4548-4553 (2004*)*). (See, U.S. Pat. Nos. 5,409,818, 5,554,517, and 6,063,603. Other amplification methods that may be used are described in, U.S. Pat. Nos. 5,242,794, 5,494,810, 5,409,818, 4,988,617, 6,063,603 and 5,554,517 and in U.S. Ser. No. 09/854,317.

Generation of a genomic profile in step 106 is performed using any of several methods. Generation of a genomic profile can be performed using any of several methods. Several methods are known in the art to identify genetic variations, and include, but are not limited to, DNA sequencing by any of several methodologies, PCR based methods, fragment length polymorphism assays (restriction fragment length polymorphism (RFLP), cleavage fragment length polymorphism (CFLP)) hybridization methods using an allele-specific oligonucleotide as a template (e.g., TaqMan assays and microarrays, further described herein), methods using a primer extension reaction, mass spectrometry (such as, MALDI-TOF/MS method), and the like, such as described in Kwok, Pharmocogenomics 1:95-100 (2000*).* Other methods include invader methods, such as monoplex and biplex invader assays (e.g. available from Third Wave Technologies, Madison, WI and described in Olivier et al., Nucl. Acids Res. 30: e53 (2002*)).*

In one embodiment, a high density DNA array is used for SNP identification and profile generation. Such arrays are commercially available from Affymetrix and Illumina (see Affymetrix GeneChip® 500K Assay Manual, Affymetrix, Santa Clara, CA; Sentrix® humanHap650Y genotyping beadchip, Illumina, San Diego, CA).

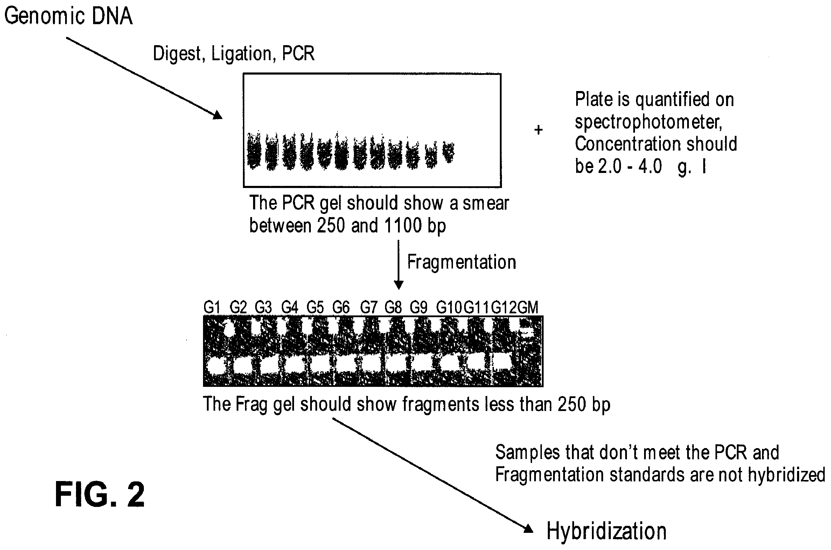

For example, a SNP profile can be generated by genotyping more than 900,000 SNPs using the Affymetrix Genome Wide Human SNP Array 6.0. Alternatively, more than 500,000 SNPs through whole-genome sampling analysis may be determined by using the Affymetrix GeneChip Human Mapping 500K Array Set. In these assays, a subset of the human genome is amplified through a single primer amplification reaction using restriction enzyme digested, adaptor-ligated human genomic DNA. As shown in **FIG. 2,** the concentration of the ligated DNA may then be determined. The amplified DNA is then fragmented and the quality of the sample determined prior to continuing with step **106.** If the samples meet the PCR and fragmentation standards, the sample is denatured, labeled, and then hybridized to a microarray consisting of small DNA probes at specific locations on a coated quartz surface. The amount of label that hybridizes to each probe as a function of the amplified DNA sequence is monitored, thereby yielding sequence information and resultant SNP genotyping.

Use of the Affymetrix GeneChip 500K Assay is carried out according to the manufacturer's directions. Briefly, isolated genomic DNA is first digested with either a NspI or StyI restriction endonuclease. The digested DNA is then ligated with a NspI or StyI adaptor oligonucleotide that respectively anneals to either the NspI or StyI restricted DNA. The adaptor-containing DNA following ligation is then amplified by PCR to yield amplified DNA fragments between about 200 and 1100 base pairs, as confirmed by gel electrophoresis. PCR products that meet the amplification standard are purified and quantified for fragmentation. The PCR products are fragmented with DNase I for optimal DNA chip hybridization. Following fragmentation, DNA fragments should be less than 250 base pairs, and on average, about 180 base pairs, as confirmed by gel electrophoresis. Samples that meet the fragmentation standard are then labeled with a biotin compound using terminal deoxynucleotidyl transferase. The labeled fragments are next denatured and then hybridized into a GeneChip 250K array. Following hybridization, the array is stained prior to scanning in a three step process consisting of a streptavidin phycoerythin (SAPE) stain, followed by an antibody amplification step with a biotinylated, anti-streptavidin antibody (goat), and final stain with streptavidin phycoerythin (SAPE). After labeling, the array is covered with an array holding buffer and then scanned with a scanner such as the Affymetrix GeneChip Scanner 3000.

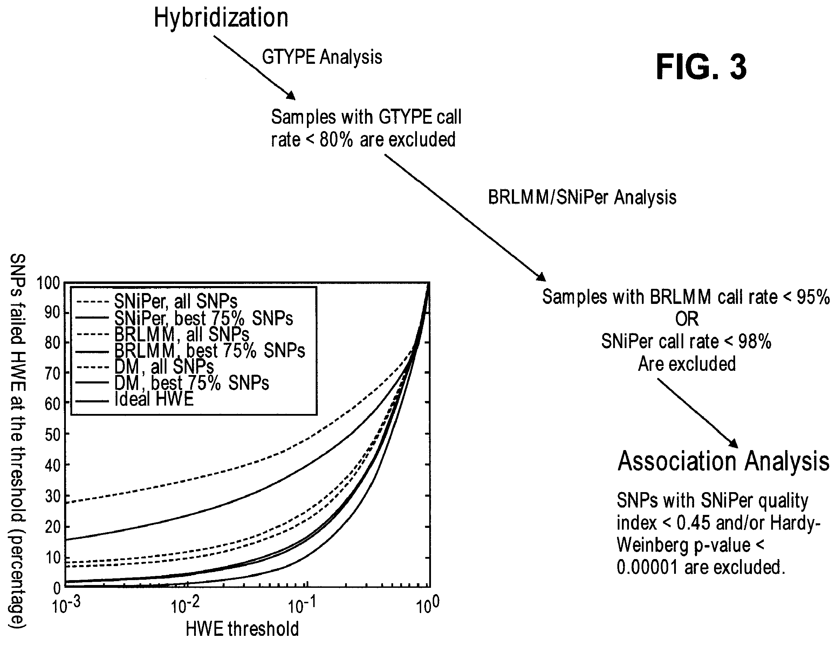

Analysis of data following scanning of an Affymetrix GeneChip Human Mapping 500K Array Set is performed according to the manufacturer's guidelines, as shown in **FIG. 3.** Briefly, acquisition of raw data using GeneChip Operating Software (GCOS) occurs. Data may also be aquired using Affymetrix GeneChip Command Console™. The aquisition of raw data is followed by analysis with GeneChip Genotyping Analysis Software (GTYPE). For purposes of the present disclosure, samples with a GTYPE call rate of less than 80% are excluded. Samples are then examined with BRLMM and/or SNiPer algorithm analyses. Samples with a BRLMM call rate of less than 95% or a SNiPer call rate of less than 98% are excluded. Finally, an association analysis is performed, and samples with a SNiPer quality index of less than 0.45 and/or a Hardy-Weinberg p-value of less than 0.00001 are excluded.

As an alternative to or in addition to DNA microarray analysis, genetic variations such as SNPs and mutations can be detected by other hybridization based methods, such as the use of TaqMan methods and variations thereof. TaqMan PCR, iterative TaqMan, and other variations of real time PCR (RT-PCR), such as those described in Livak et al., Nature Genet., 9, 341-32 (1995*)* and Ranade et al. Genome Res., 11, 1262-1268 (2001*)* can be used in the methods disclosed herein. In some embodiments, probes for specific genetic variations, such as SNPs, are labeled to form TaqMan probes. The probes are typically approximately at least 12, 15, 18 or 20 base pairs in length. They may be between approximately 10 and 70, 15 and 60, 20 and 60, or 18 and 22 base pairs in length. The probe is labeled with a reporter label, such as a fluorophore, at the 5' end and a quencher of the label at the 3'end. The reporter label may be any fluorescent molecule that has its fluorescence inhibited or quenched when in close proximity, such as the length of the probe, to the quencher. For example, the reporter label can be a fluorophore such as 6-carboxyfluorescein (FAM), tetracholorfluorescin (TET), or derivatives thereof, and the quencher tetramethylrhodamine (TAMRA), dihydrocyclopyrroloindole tripeptide (MGB), or derivatives thereof.

As the reporter fluorophore and quencher are in close proximity, separated by the length of the probe, the fluorescence is quenched. When the probe anneals to a target sequence, such as a sequence comprising a SNP in a sample, DNA polymerase with 5' to 3' exonuclease activity, such as Taq polymerase, can extend the primer and the exonuclease activity cleaves the probe, separating the reporter from the quencher, and thus the reporter can fluoresce. The process can be repeated, such as in RT-PCR. The TaqMan probe is typically complementary to a target sequence that is located between two primers that are designed to amplify a sequence. Thus, the accumulation of PCR product can be correlated to the accumulation of released fluorophore, as each probe can hybridize to newly generated PCR product. The released fluorophore can be measured and the amount of target sequence present can be determined. RT-PCR methods for high througput genotyping, can be employed.

Genetic variations can also be identified by DNA sequencing. DNA sequencing may be used to sequence a substantial portion, or the entire, genomic sequence of an individual. Traditionally, common DNA sequencing has been based on polyacrylamide gel fractionation to resolve a population of chain-terminated fragments (Sanger et al., Proc. Natl. Acad. Sci. USA 74:5463-5467 (1977)). Alternative methods have been and continue to be developed to increase the speed and ease of DNA sequencing. For example, high throughput and single molecule sequencing platforms are commercially available or under development from 454 Life Sciences (Branford, CT) (Margulies et al., Nature 437:376-380 (2005)); Solexa (Hayward, CA); Helicos BioSciences Corporation (Cambridge, MA) (U.S. application Ser. No. 11/167046, filed June 23, 2005), and Li-Cor Biosciences (Lincoln, NE) (U.S. application Ser. No. 11/118031, filed April 29, 2005).

After an individual's genomic profile is generated in step **106,** the profile is stored digitally in step **108,** such profile may be stored digitally in a secure manner. The genomic profile is encoded in a computer readable format to be stored as part of a data set and may be stored as a database, where the genomic profile may be "banked", and can be accessed again later. The data set comprises a plurality of data points, wherein each data point relates to an individual. Each data point may have a plurality of data elements. One data element is the unique identifier, used to identify the individual's genomic profile. It may be a bar code. Another data element is genotype information, such as the SNPs or nucleotide sequence of the individual's genome. Data elements corresponding to the genotype information may also be included in the data point. For example, if the genotype information includes SNPs identified by microarray analysis, other data elements may include the microarray SNP identification number, the SNP rs number, and the polymorphic nucleotide. Other data elements may be chromosome position of the genotype information, quality metrics of the data, raw data files, images of the data, and extracted intensity scores.

The individual's specific factors such as physical data, medical data, ethnicity, ancestry, geography, gender, age, family history, known phenotypes, demographic data, exposure data, lifestyle data, behavior data, and other known phenotypes may also be incorporated as data elements. For example, factors may include, but are not limited to, individual's: birthplace, parents and/or grandparents, relatives' ancestry, location of residence, ancestors' location of residence, environmental conditions, known health conditions, known drug interactions, family health conditions, lifestyle conditions, diet, exercise habits, marital status, and physical measurements, such as weight, height, cholesterol level, heart rate, blood pressure, glucose level and other measurements known in the art The above mentioned factors for an individual's relatives or ancestors, such as parents and grandparents, may also be incorporated as data elements and used to determine an individual's risk for a phenotype or condition.

The specific factors may be obtained from a questionnaire or from a health care manager of the individual. Information from the "banked" profile can then be accessed and utilized as desired. For example, in the initial assessment of an individual's genotype correlations, the individual's entire information (typically SNPs or other genomic sequences across, or taken from an entire genome) will be analyzed for genotype correlations. In subsequent analyses, either the entire information can be accessed, or a portion thereof, from the stored, or banked genomic profile, as desired or appropriate.

Comparison of genomic profile with database of genotype correlations.

In step **110,** genotype correlations are obtained from scientific literature. Genotype correlations for genetic variations are determined from analysis of a population of individuals who have been tested for the presence or absence of one or more phenotypic traits of interest and for genotype profile. The alleles of each genetic variation or polymorphism in the profile are then reviewed to determine whether the presence or absence of a particular allele is associated with a trait of interest. Correlation can be performed by standard statistical methods and statistically significant correlations between genetic variations and phenotypic characteristics are noted. For example, it may be determined that the presence of allele A1 at polymorphism A correlates with heart disease. As a further example, it might be found that the combined presence of allele A1 at polymorphism A and allele **B1** at polymorphism B correlates with increased risk of cancer. The results of the analyses may be published in peer-reviewed literature, validated by other research groups, and/or analyzed by a committee of experts, such as geneticists, statisticians, epidemiologists, and physicians, and may also be curated.

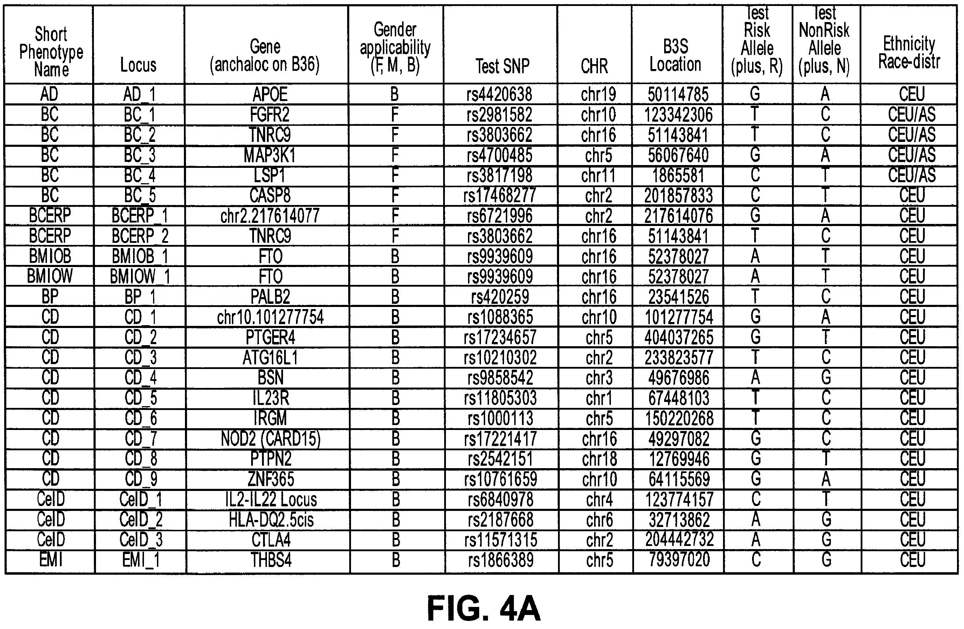

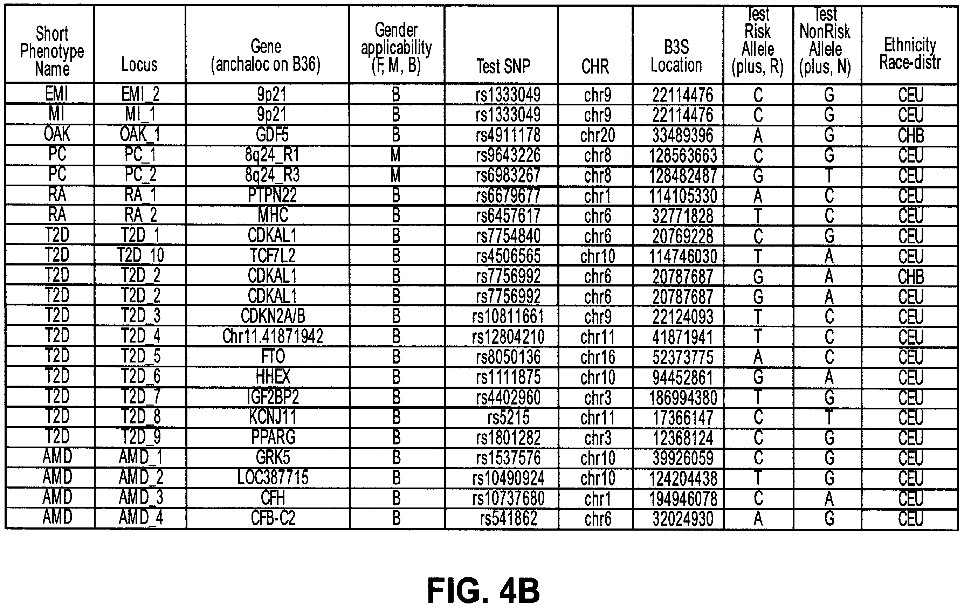

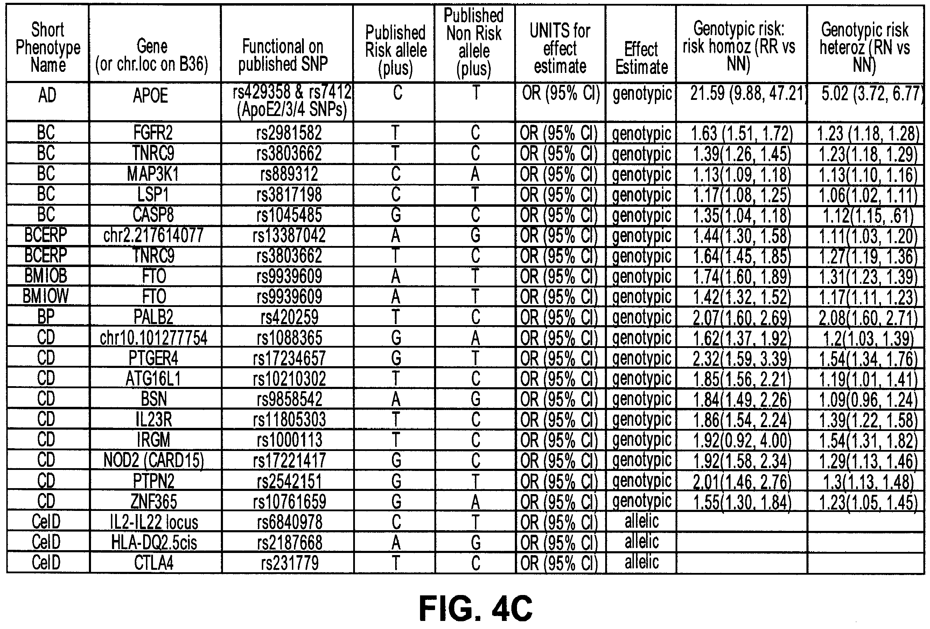

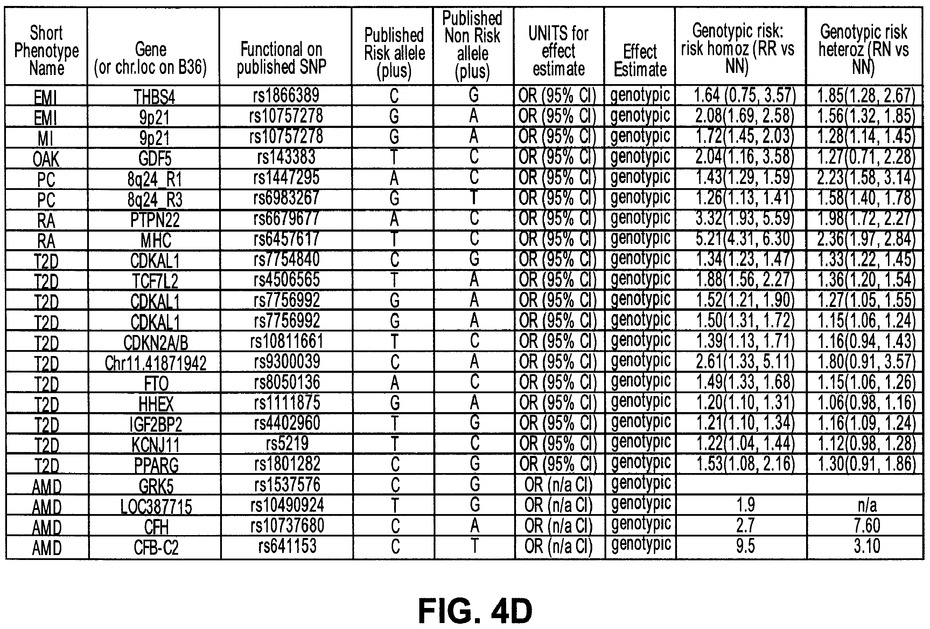

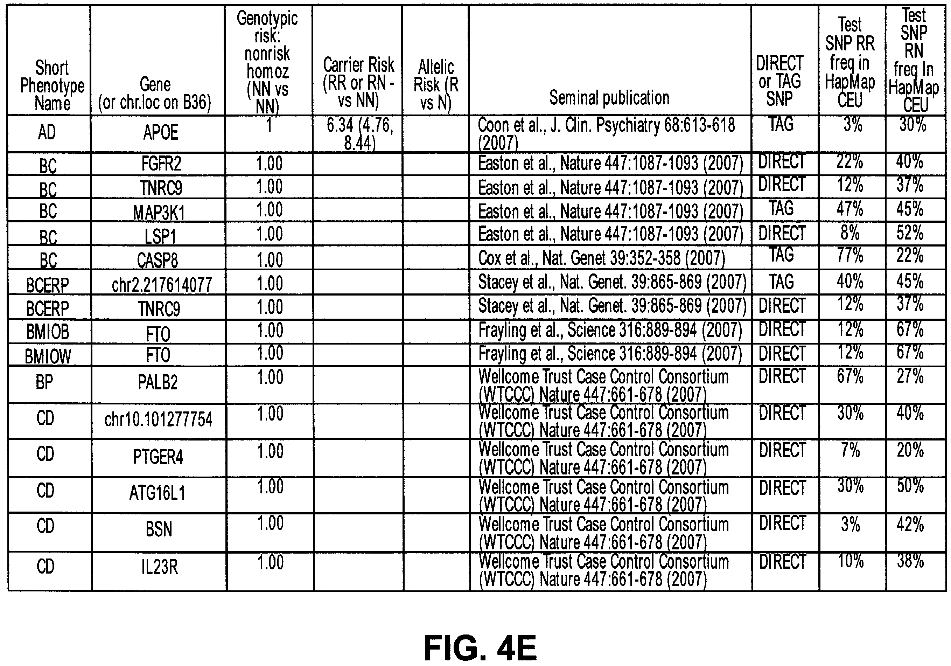

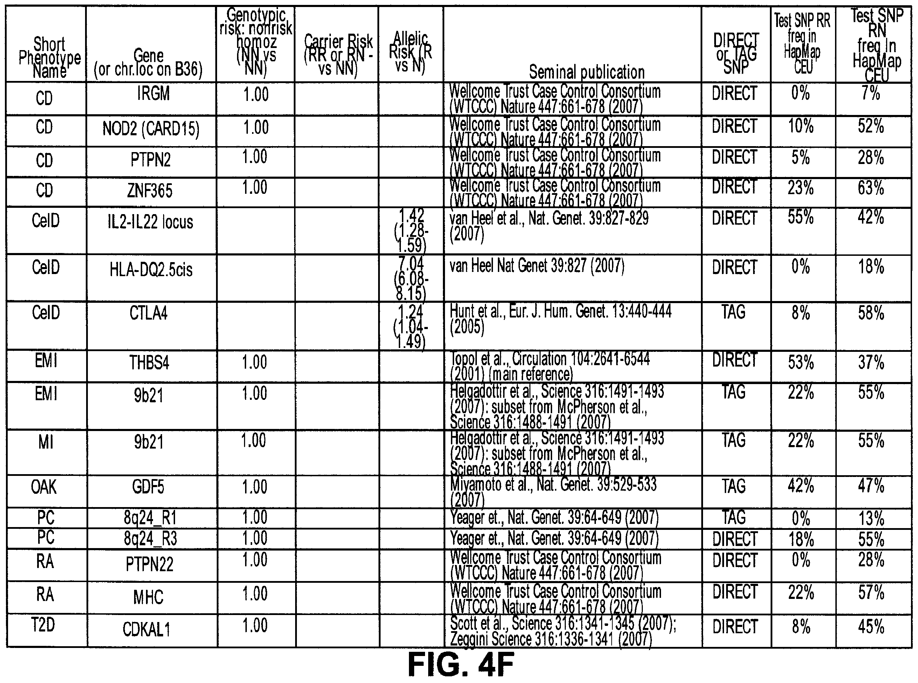

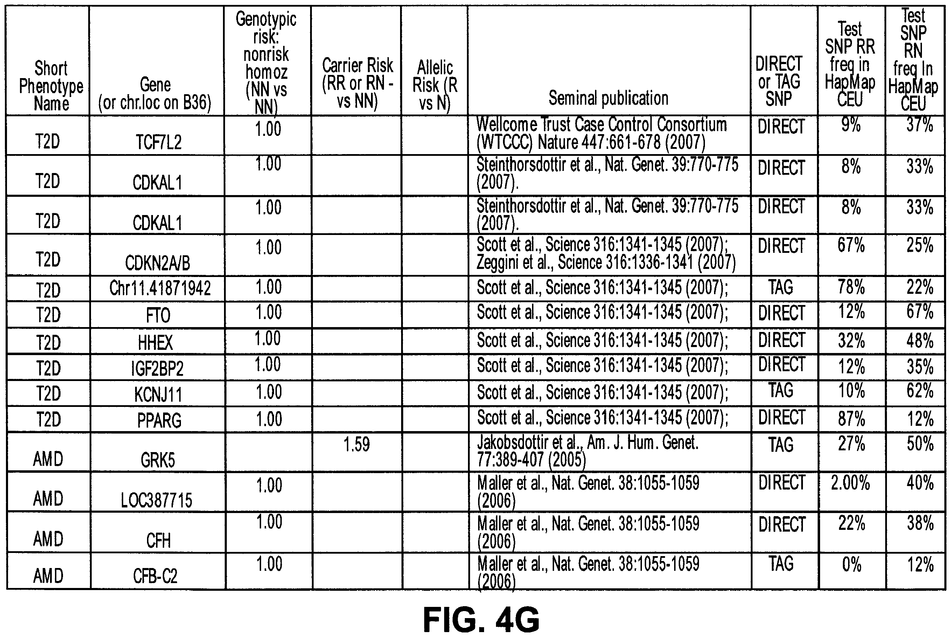

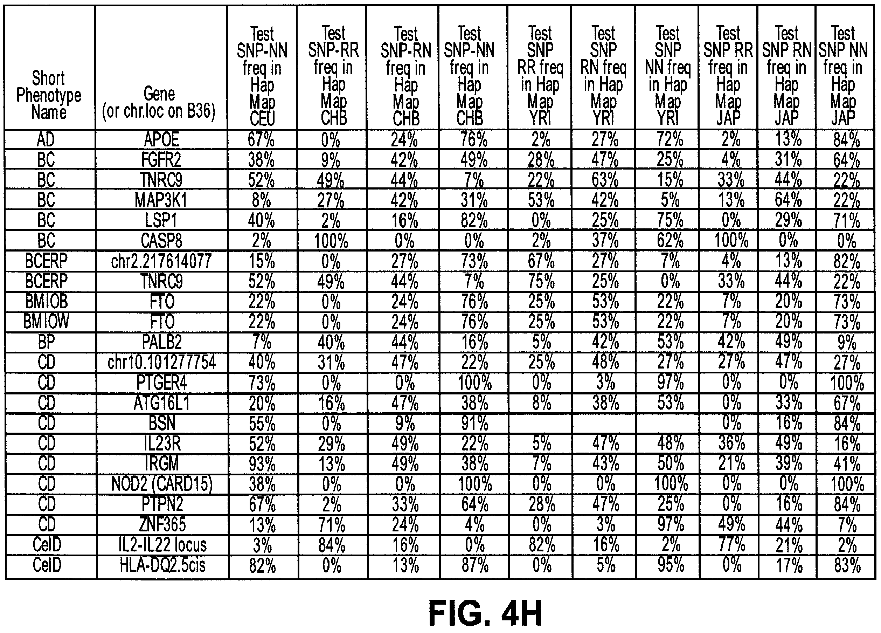

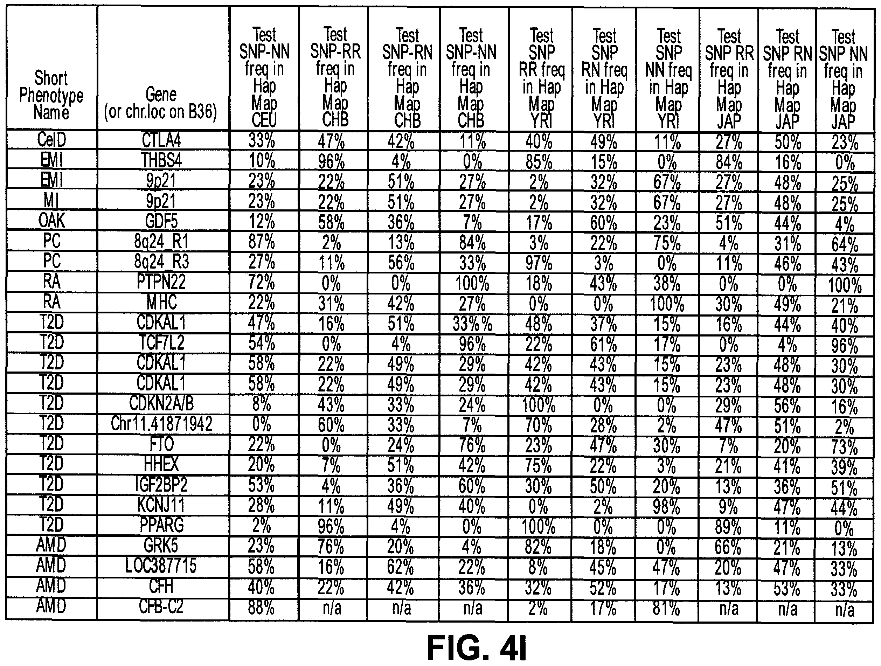

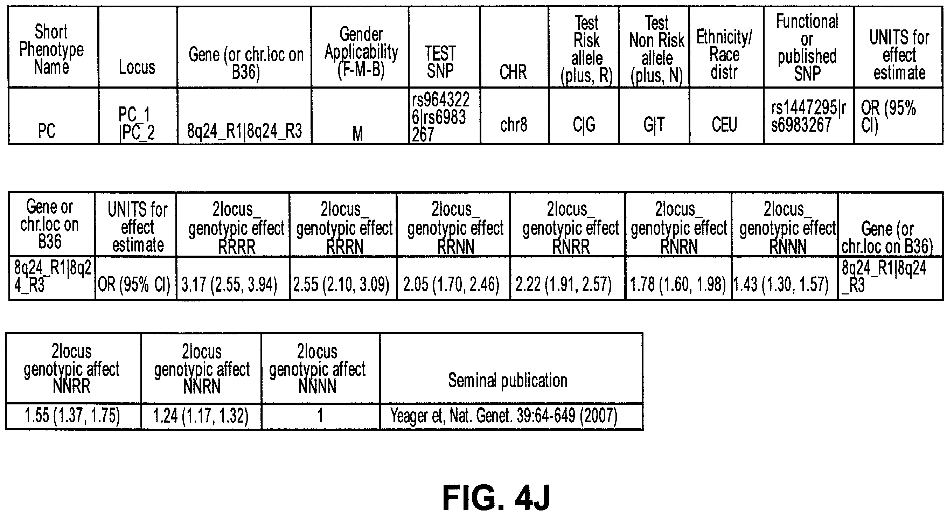

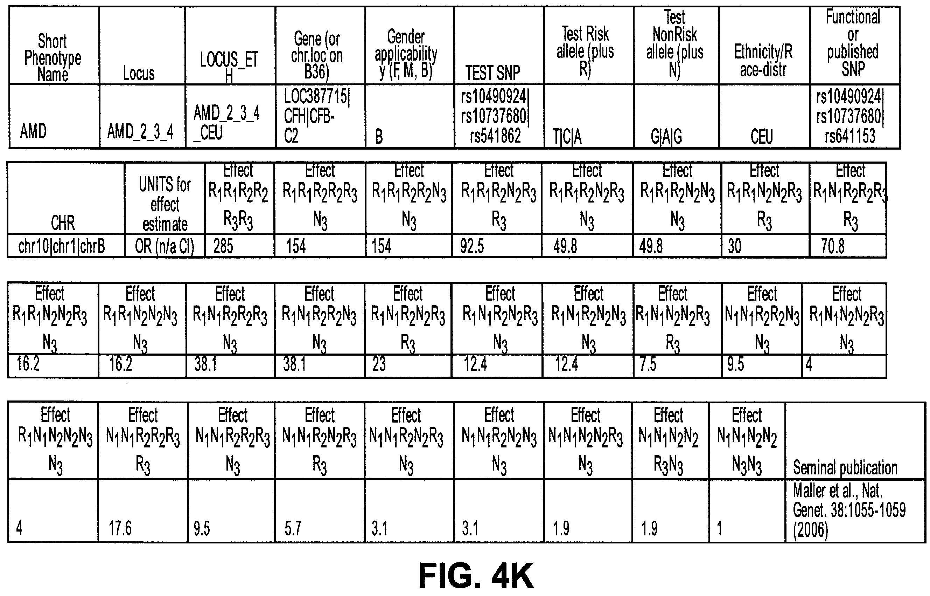

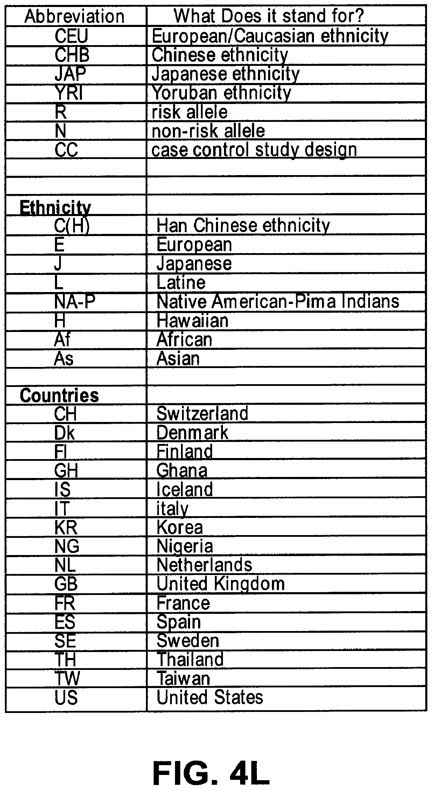

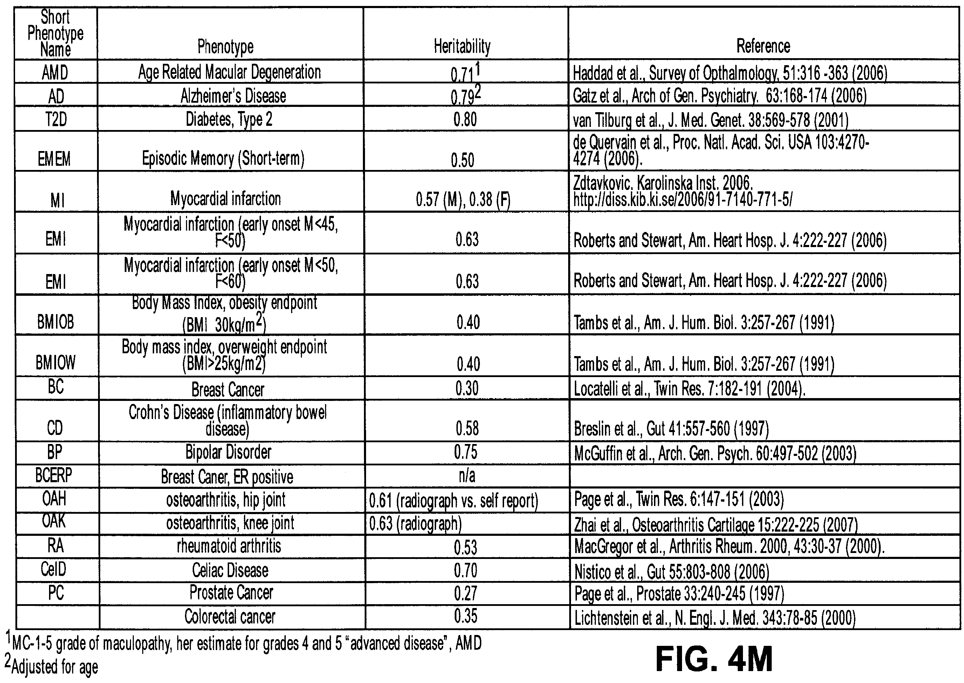

In **FIGS. 4, 5,** and **6** are examples of correlations between genotypes and phenotypes from which rules to be applied to genomic profiles may be based. For example, in FIGS. 4A and B, each row corresponds to a phenotype/locus/ethnicity, wherein FIGS. 4C through I contains further information about the correlations for each of these rows. As an example, in **FIG. 4A,** the "Short Phenotype Name" of BC, as noted in **FIG. 4M,** an index for the names of the short phenotypes, is an abbreviation for breast cancer. In row BC_4, which is the generic name for the locus, the gene LSP1 is correlated to breast cancer. The published or functional SNP identified with this correlation is rs3817198, as shown in **FIG. 4C,** with the published risk allele being C, the nonrisk allele being T. The published SNP and alleles are identified through publications such as seminal publications as in FIGS. 4E-G. In the example of LSP1 in **FIG. 4E,** the seminal publication is Easton et al., Nature 447:713-720 (2007).

Alternatively, the correlations may be generated from the stored genomic profiles. For example, individuals with stored genomic profiles may also have known phenotype information stored as well. Analysis of the stored genomic profiles and known phenotypes may generate a genotype correlation. As an example, 250 individuals with stored genomic profiles also have stored information that they have previously been diagnosed with diabetes. Analysis of their genomic profiles is performed and compared to a control group of individuals without diabetes. It is then determined that the individuals previously diagnosed with diabetes have a higher rate of having a particular genetic variant compared to the control group, and a genotype correlation may be made between that particular genetic variant and diabetes.

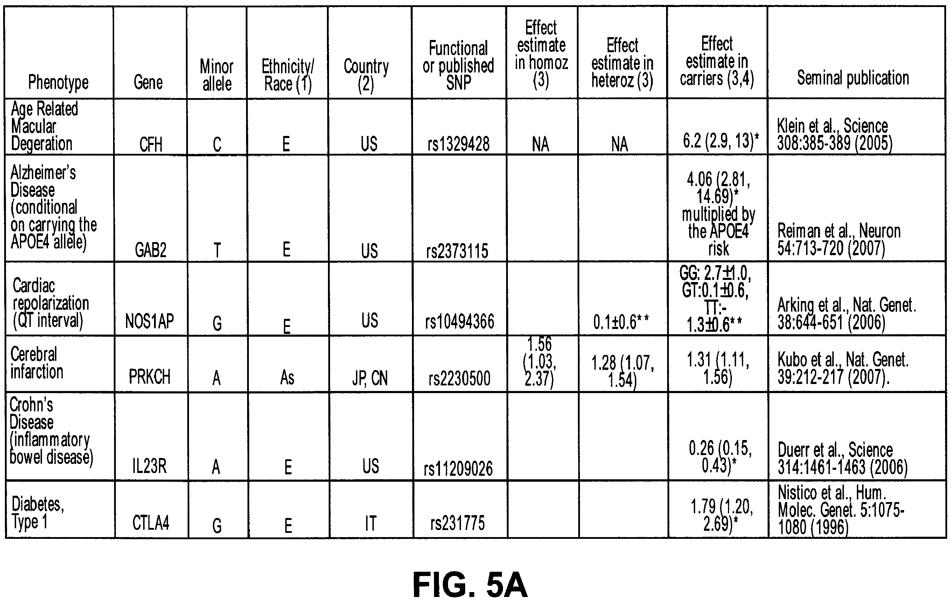

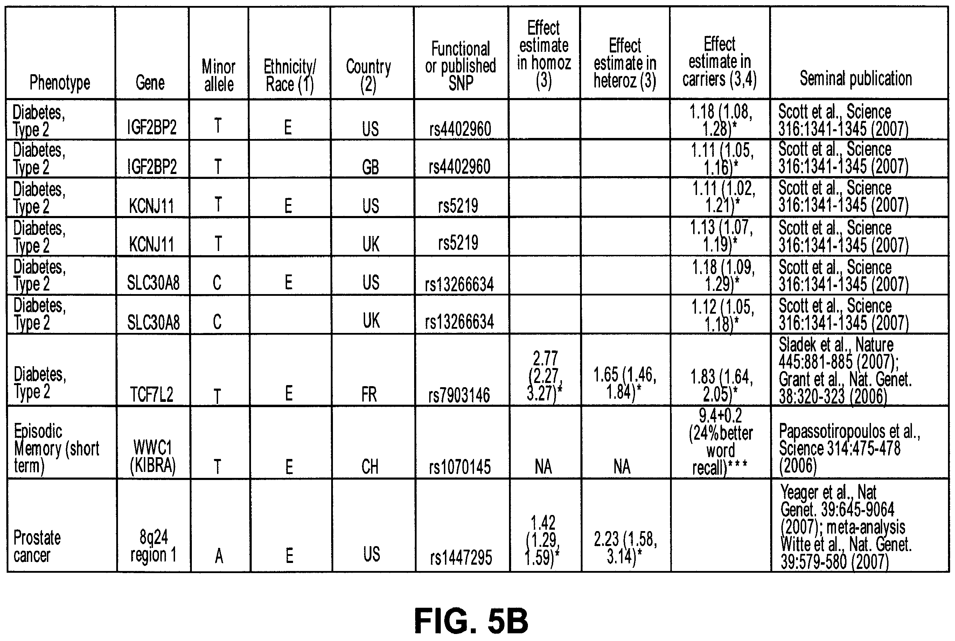

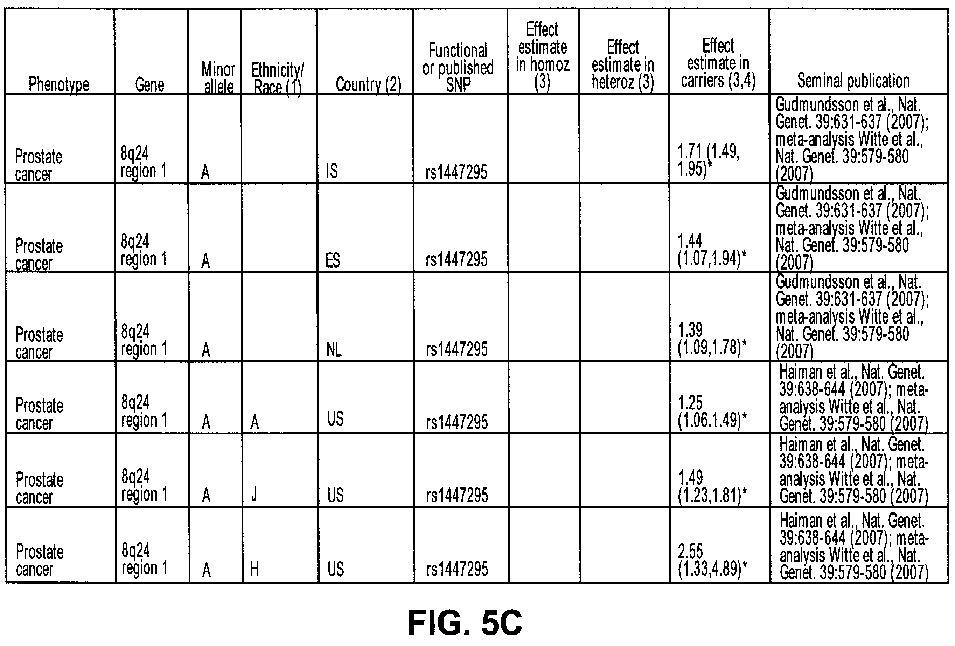

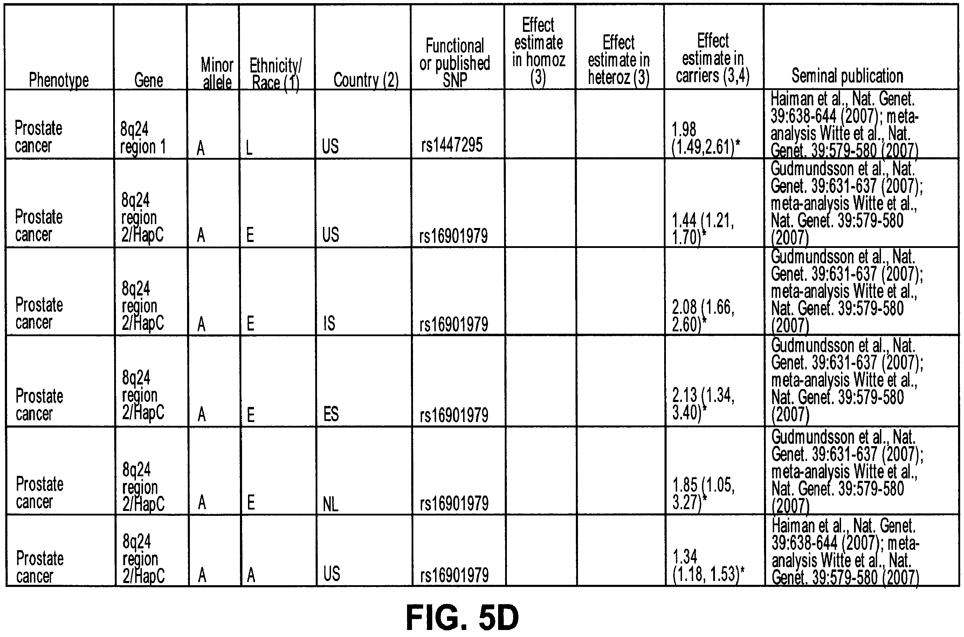

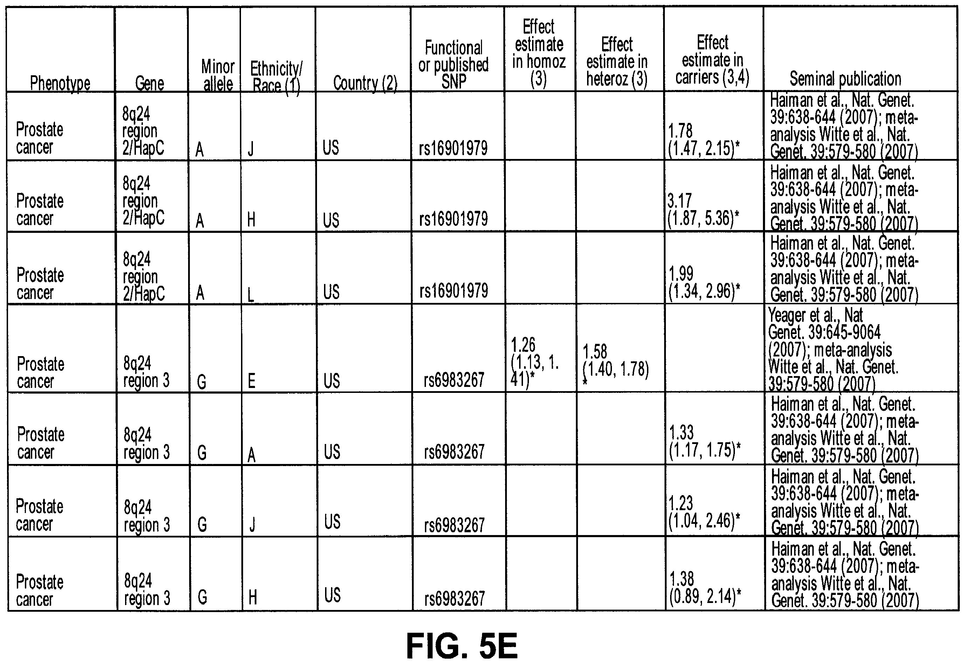

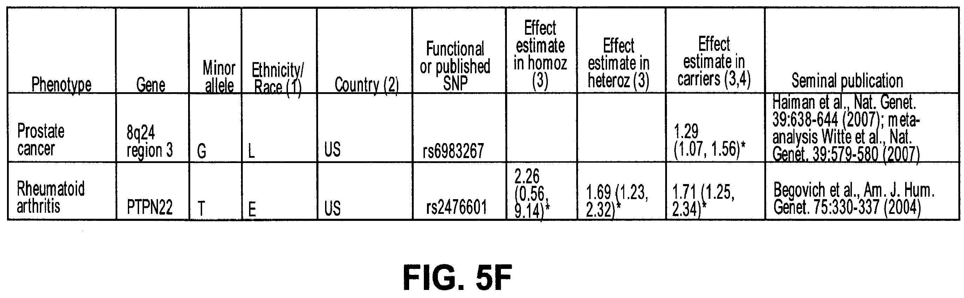

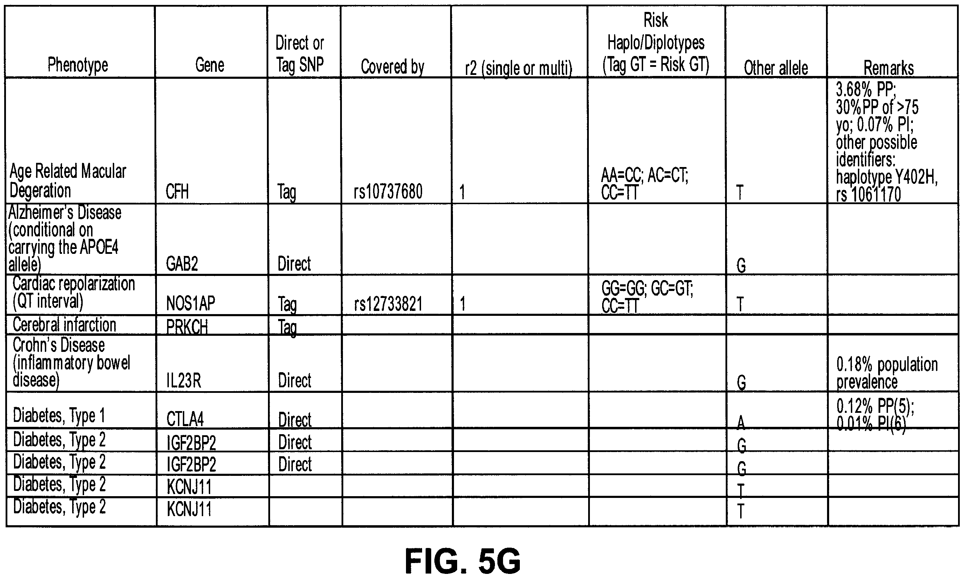

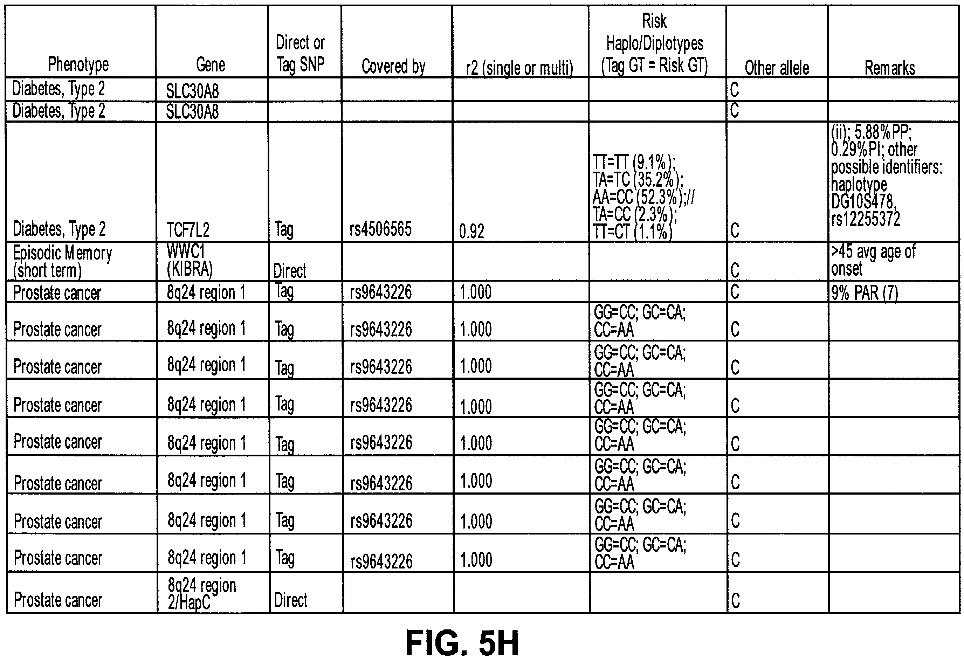

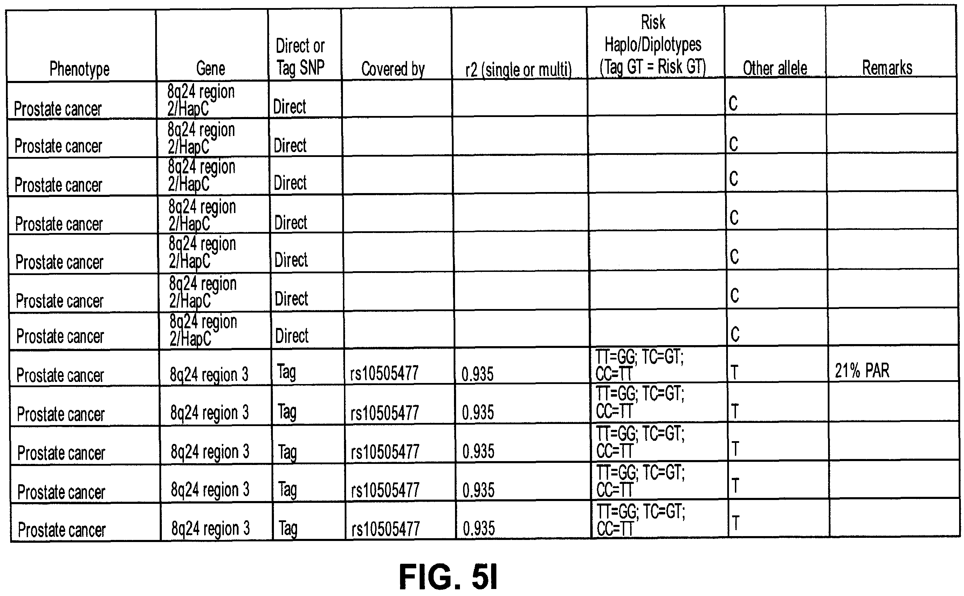

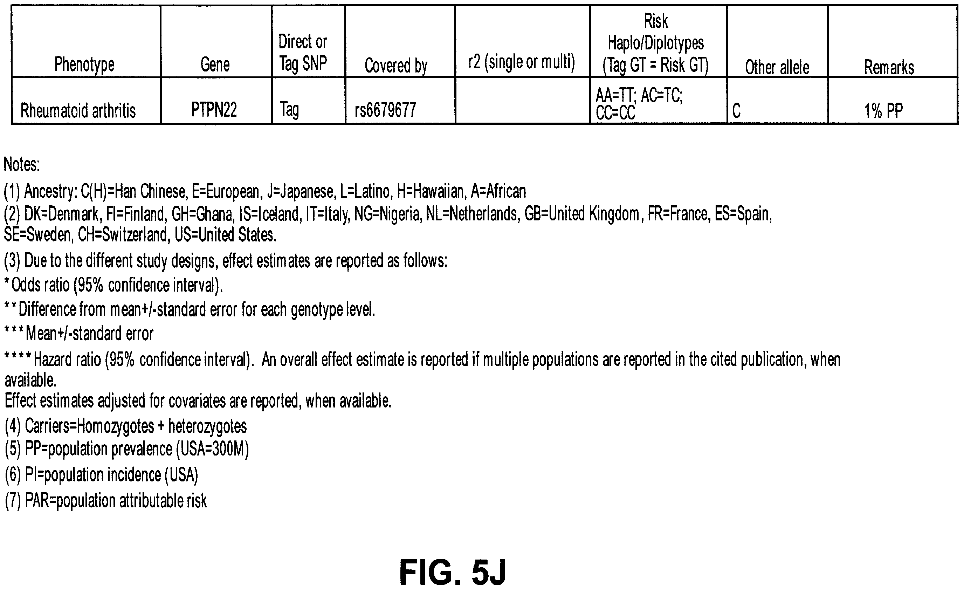

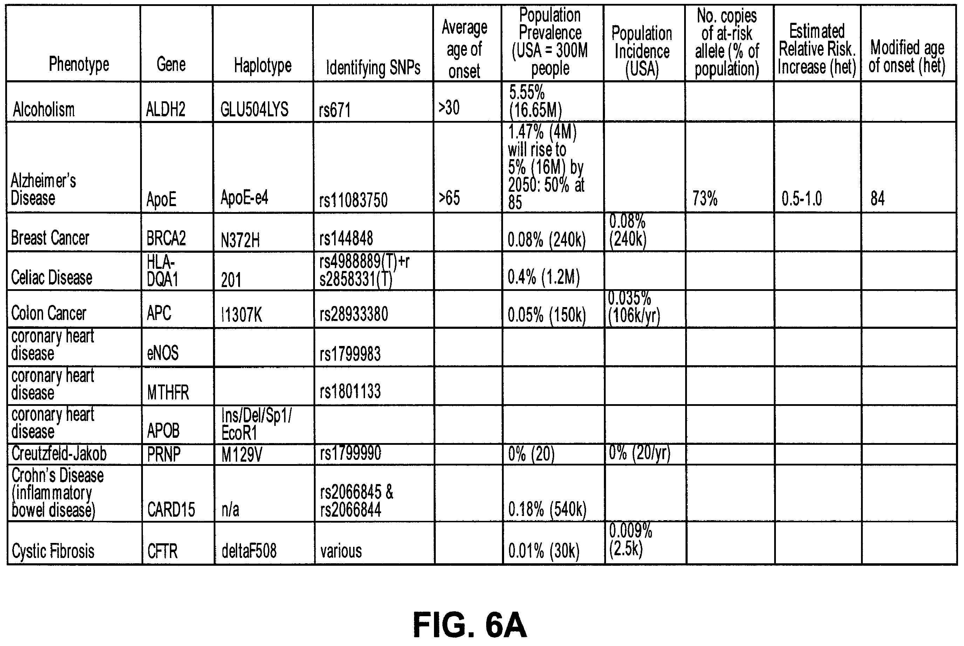

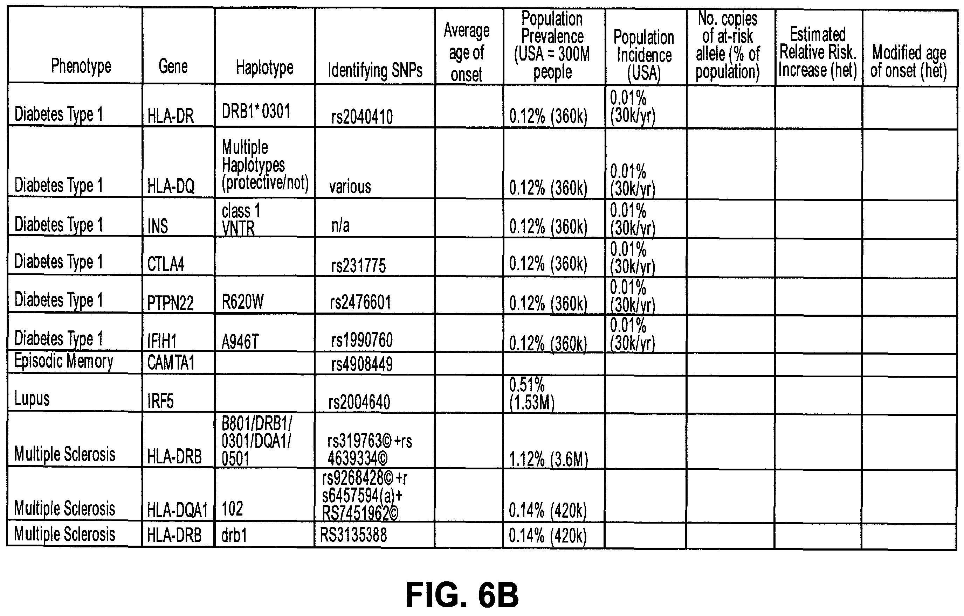

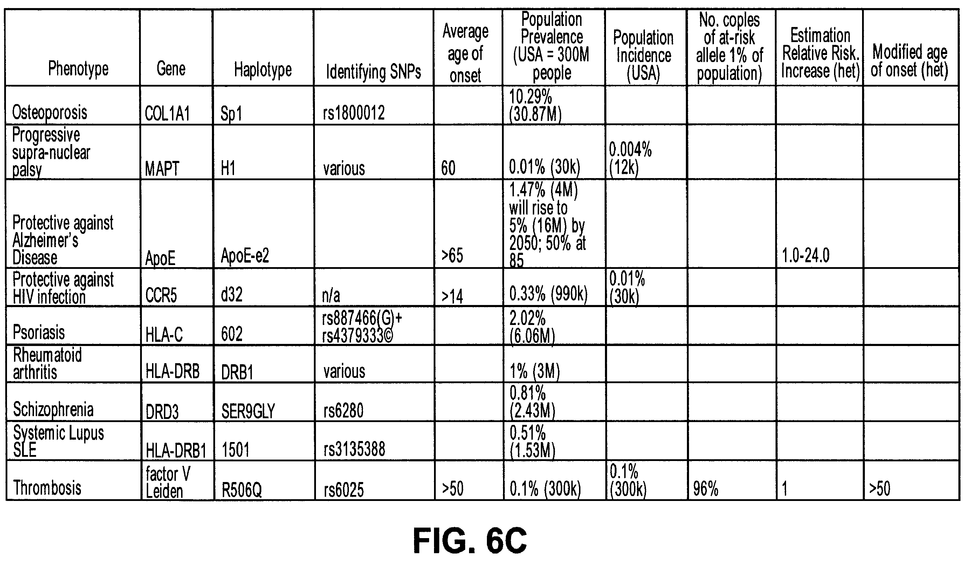

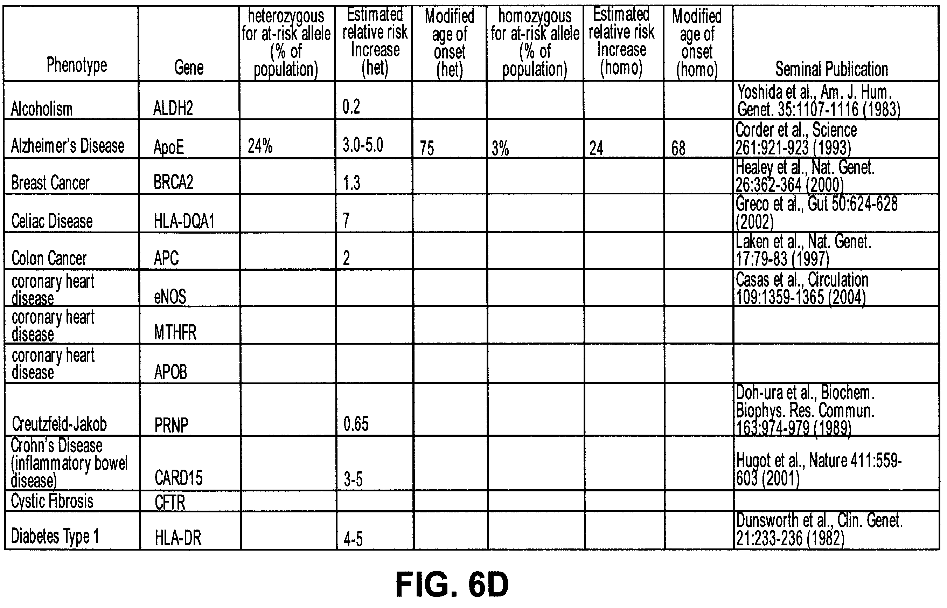

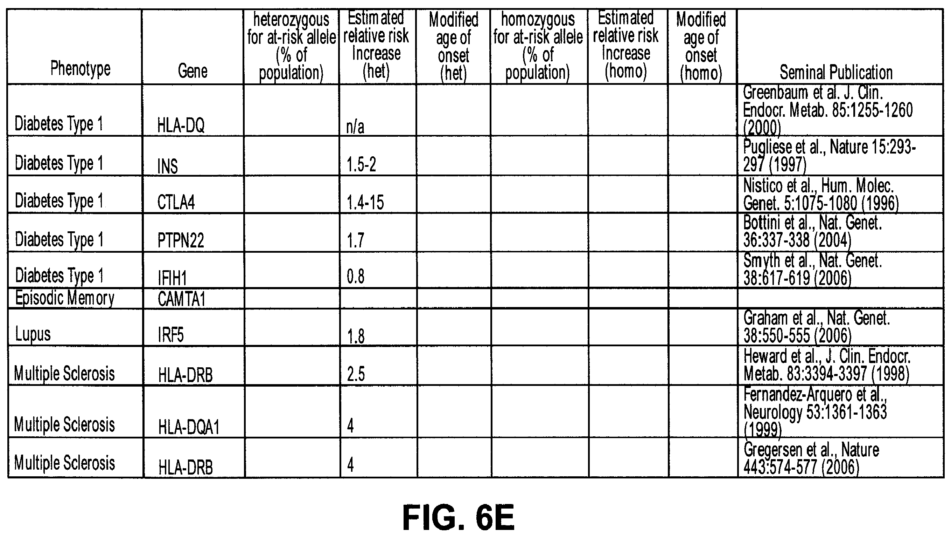

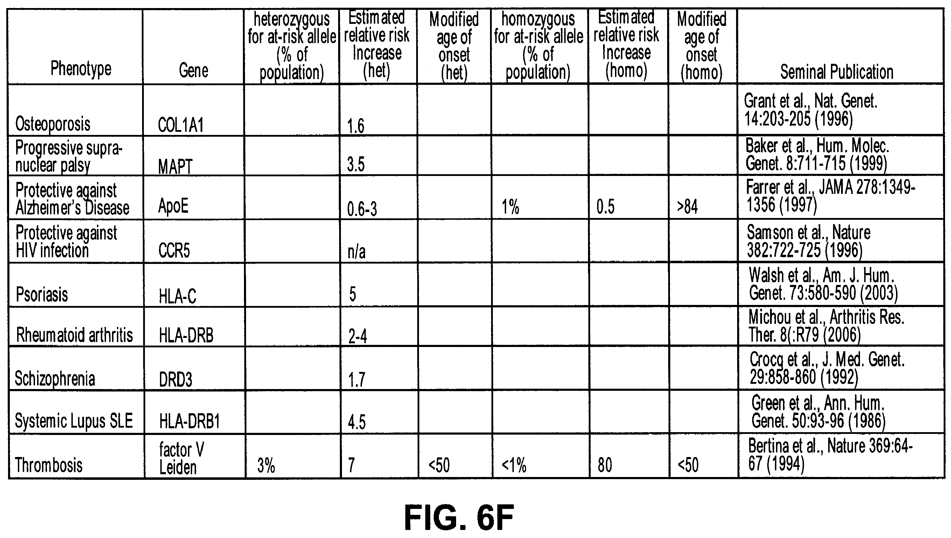

In step **112,** rules are made based on the validated correlations of genetic variants to particular phenotypes. Rules may be generated based on the genotypes and phenotypes correlated as listed in Table 1, for example. Rules based on correlations may incorporate other factors such as gender (e.g. **FIG. 4)** or ethnicity **(FIGS. 4** and **5),** to generate effects estimates, such as those in **FIGS. 4** and **5.** Other measures resulting from rules may be estimated relative risk increase such as in **FIG. 6.** The effects estimates and estimated relative risk increase may be from the published literature, or calculated from the published literature. Alternatively, the rules may be based on correlations generated from stored genomic profiles and previously known phenotypes.

In a preferred embodiment, the genetic variants will be SNPs. While SNPs occur at a single site, individuals who carry a particular SNP allele at one site often predictably carry specific SNP alleles at other sites. A correlation of SNPs and an allele predisposing an individual to disease or condition occurs through linkage disequilibrium, in which the non-random association of alleles at two or more loci occur more or less frequently in a population than would be expected from random formation through recombination.

Other genetic markers or variants, such as nucleotide repeats or insertions, may also be in linkage disequilibrium with genetic markers that have been shown to be associated with specific phenotypes. For example, a nucleotide insertion is correlated with a phenotype and a SNP is in linkage disequilibrium with the nucleotide insertion. A rule is made based on the correlation between the SNP and the phenotype. A rule based on the correlation between the nucleotide insertion and the phenotype may also be made. Either rules or both rules may be applied to a genomic profile, as the presence of one SNP may give a certain risk factor, the other may give another risk factor, and when combined may increase the risk.

Through linkage disequilibrium, a disease predisposing allele cosegregates with a particular allele of a SNP or a combination of particular alleles of SNPs. A particular combination of SNP alleles along a chromosome is termed a haplotype, and the DNA region in which they occur in combination can be referred to as a haplotype block. While a haplotype block can consist of one SNP, typically a haplotype block represents a contiguous series of 2 or more SNPs exhibiting low haplotype diversity across individuals and with generally low recombination frequencies. An identification of a haplotype can be made by identification of one or more SNPs that lie in a haplotype block. Thus, a SNP profile typically can be used to identify haplotype blocks without necessarily requiring identification of all SNPs in a given haplotype block.

Genotype correlations between SNP haplotype patterns and diseases, conditions or physical states are increasingly becoming known. For a given disease, the haplotype patterns of a group of people known to have the disease are compared to a group of people without the disease. By analyzing many individuals, frequencies of polymorphisms in a population can be determined, and in turn these frequencies or genotypes can be associated with a particular phenotype, such as a disease or a condition. Examples of known SNP-disease correlations include polymorphisms in Complement Factor H in age-related macular degeneration (Klein et al., Science: 308:385-389, (2005*)*) and a variant near the INSIG2 gene associated with obesity (Herbert et al., Science: 312:279-283 (2006*)*). Other known SNP correlations include polymorphisms in the 9p21 region that includes CDKN2A and B, such as) such as rs10757274, rs2383206, rs13333040, rs2383207, and rs10116277 correlated to myocardial infarction *(*Helgadottir et al., Science 316:1491-1493 (2007*);* McPherson et al., Science 316:1488-1491 (2007*)*)

The SNPs may be functional or non-functional. For example, a functional SNP has an effect on a cellular function, thereby resulting in a phenotype, whereas a non-functional SNP is silent in function, but may be in linkage disequilibrium with a functional SNP. The SNPs may also be synonymous or non-synonymous. SNPs that are synonymous are SNPs in which the different forms lead to the same polypeptide sequence, and are non-functional SNPs. If the SNPs lead to different polypetides, the SNP is non-synonymous and may or may not be functional. SNPs, or other genetic markers, used to identify haplotypes in a diplotype, which is 2 or more haplotypes, may also be used to correlate phenotypes associated with a diplotype. Information about an individual's haplotypes, diplotypes, and SNP profiles may be in the genomic profile of the individual.

In preferred embodiments, for a rule to be generated based on a genetic marker in linkage disequilibrium with another genetic marker that is correlated with a phenotype, the genetic marker may have a $r^2$ or D' score, scores commonly used in the art to determine linkage disequilibrium, of greater than 0.5. In preferred embodiments, the score is greater than 0.6, 0.7, 0.8, 0.90, 0.95 or 0.99. As a result, in the present disclosure, the genetic marker used to correlate a phenotype to an individual's genomic profile may be the same as the functional or published SNP correlated to a phenotype, or different. For example, using BC_4, the test SNP and published SNP are the same, as are the test risk and nonrisk alleles are the same as the published risk and nonrisk alleles **(**FIGS. 4A and C). However, for BC_5, CASP8 and its correlation to breast cancer, the test SNP is different from its functional or published SNP, as are the test risk and nonrisk alleles to the published risk and nonrisk alleles. The test and published alleles are oriented relative to the plus strand of the genome, and from these columns, it can be inferred the homozygous risk or nonrisk genotype, which may generate a rule to be applied to the genomic profile of individuals such as subscribers.

The test SNPs may be "DIRECT" or "TAG" SNPs **(**FIGS. 4E-G, FIG. 5). Direct SNPs are the test SNPs that are the same as the published or functional SNP, such as for BC_4. Direct SNPs may also be used for FGFR2 correlation with breast cancer, using the SNP rs1073640 in Europeans and Asians, where the minor allele is A and the other allele is G *(*Easton et al., Nature 447:1087-1093 (2007*)*). Another published or functional SNP for FGFR2 correlation to breast cancer is rs1219648, also in Europeans and Asians *(*Hunter et al., Nat. Genet. 39:870-874 (2007*)).* Tag SNPs are where the test SNP is different from that of the functional or published SNP, as in for BC_5. Tag SNPs may also be used for other genetic variants such as SNPs for CAMTA1 (rs4908449), 9p21 (rs10757274, rs2383206, rs13333040, rs2383207, rs10116277), COL1A1 (rs1800012), FVL (rs6025), HLA-DQA1 (rs4988889, rs2588331), eNOS (rs1799983), MTHFR (rs1801133), and APC (rs28933380).

Databases of SNPs are publicly available from, for example, the International HapMap Project (see www.hapmap.org, The International HapMap Consortium, Nature 426:789-796 (2003*),* and The International HapMap Consortium, Nature 437:1299-1320 (2005*)*), the Human Gene Mutation Database (HGMD) public database (see www.hgmd.org), and the Single Nucleotide Polymorphism database (dbSNP) (see www.ncbi.nlm.nih.gov/SNP/). These databases provide SNP haplotypes, or enable the determination of SNP haplotype patterns. Accordingly, these SNP databases enable examination of the genetic risk factors underlying a wide range of diseases and conditions, such as cancer, inflammatory diseases, cardiovascular diseases, neurodegenerative diseases, and infectious diseases. The diseases or conditions may be actionable, in which treatments and therapies currently exist. Treatments may include prophylactic treatments as well as treatments that ameliorate symptoms and conditions, including lifestyle changes.

Many other phenotypes such as physical traits, physiological traits, mental traits, emotional traits, ethnicity, ancestry, and age may also be examined. Physical traits may include height, hair color, eye color, body, or traits such as stamina, endurance, and agility. Mental traits may include intelligence, memory performance, or learning performance. Ethnicity and ancestry may include identification of ancestors or ethnicity, or where an individual's ancestors originated from. The age may be a determination of an individual's real age, or the age in which an individual's genetics places them in relation to the general population. For example, an individual's real age is 38 years of age, however their genetics may determine their memory capacity or physical well-being may be of the average 28 year old. Another age trait may be a projected longevity for an individual.

Other phenotypes may also include non-medical conditions, such as "fun" phenotypes. These phenotypes may include comparisons to well known individuals, such as foreign dignitaries, politicians, celebrities, inventors, athletes, musicians, artists, business people, and infamous individuals, such as convicts. Other "fun" phenotypes may include comparisons to other organisms, such as bacteria, insects, plants, or non-human animals. For example, an individual may be interested to see how their genomic profile compares to that of their pet dog, or to a former president.

At step **114,** the rules are applied to the stored genomic profile to generate a phenotype profile of step **116.** For example, information in **FIGS. 4, 5,** or **6** may form the basis of rules, or tests, to apply to an individual's genomic profile. The rules may encompass the information on test SNP and alleles, and the effect estimates of **FIG. 4,** where the UNITS for effect estimate is the units of the effect estimate, such as OR, or odds-ratio (95% confidence interval) or mean. The effects estimate may be a genotypic risk **(**FIGS. 4C-G) in preferred embodiments, such as the risk for homozygotes (homoz or RR), risk heterozygotes (heteroz or RN), and nonrisk homozygotes (homoz or NN). In other embodiments, the effect estimate may be carrier risk, which is RR or RN vs NN. In yet other embodiments, the effect estimate may be based on the allele, an allelic risk such as R vs. N. There may also be two locus **(FIG. 4J)** or three locus **(FIG. 4K)** genotypic effect estimate (e.g. RRRR, RRNN, etc for the 9 possible genotype combinations for a two locus effect estimate). The test SNP frequency in the public HapMap is also noted in FIGS. 4H and I.

The estimated risk for a condition may be based on the SNPs as listed in US Patent Application Publication No. 20080131887 and PCT Publication No. WO2008/067551. In some embodiments, the risk for a condition may be based on at least one SNP. For example, assessment of an individual's risk for Alzheimers **(AD),** colorectal cancer (CRC), osteoarthritis (OA) or exfoliation glaucoma (XFG), maybe based on 1 SNP (for example, rs4420638 for AD, rs6983267 for CRC, rs4911178 for OA and rs2165241 for XFG). For other conditions, such as obesity (BMIOB), Graves' disease (GD), or hemochromatosis (HEM), an individual's estimated risk may be based on at least 1 or 2 SNPs (for example, rs9939609 and/or rs9291171 for BMIOB; DRB1*0301 DQA1*0501 and/or rs3087243 for GD; rs1800562 and/or rs129128 for HEM). For conditions such as, but not limited to, myocardial infarction (MI), multiple sclerosis (MS), or psoriasis (PS), 1, 2, or 3 SNPs may be used to assess an individual's risk for the condition (for example, rs1866389, rs1333049, and/or rs6922269 for MI; rs6897932, rs12722489, and/or DRB1*1501 for MS; rs6859018, rs11209026, and/or HLAC*0602 for PS). For estimating an individual's risk of restless legs syndrome (RLS) or celiac disease (CelD), 1, 2, 3, or 4 SNPs (for example, rs6904723, rs2300478, rs1026732, and/or rs9296249 for RLS; rs6840978, rs11571315, rs2187668, and/or DQA1*0301 DQB1*0302 for CelD). For prostate cancer (PC) or lupus (SLE), 1, 2, 3, 4, or 5 SNPs may be used to estimate an individual's risk for PC or SLE (for example, rs4242384, rs6983267, rs16901979, rs17765344, and/or rs4430796 for PC; rs12531711, rs10954213, rs2004640, DRB1*0301, and/or DRB1*1501 for SLE). For estimating an individual's lifetime risk of macular degeneration (AMD) or rheumatoid arthritis (RA), 1, 2, 3, 4, 5, or 6 SNPs, may be used (for example, rs10737680, rs10490924, rs541862, rs2230199, rs1061170, and/or rs9332739 for AMD; rs6679677, rs11203367, rs6457617, DRB*0101, DRB1*0401, and/or DRB1*0404 for RA). For estimating an individual's lifetime risk of breast cancer (BC), 1, 2, 3, 4, 5, 6 or 7 SNPs maybe used (for example, rs3803662, rs2981582, rs4700485, rs3817198, rs17468277, rs6721996, and/or rs3803662). For estimating an individual's lifetime risk of Crohn's disease (CD) or Type 2 diabetes (T2D), 1, 2, 3, 4, 5, 6, 7, 8, 9, 10, or 11 SNPs may be used (for example, rs2066845, rs5743293, rs10883365, rs17234657, rs10210302, rs9858542, rs11805303, rs1000113, rs17221417, rs2542151, and/or rs10761659 for CD; rs13266634, rs4506565, rs10012946, rs7756992, rs10811661, rs12288738, rs8050136, rs1111875, rs4402960, rs5215, and/or rs1801282 for T2D). In some embodiments, the SNPs used as a basis for determining risk may be in linkage disequilibrium with the SNPs as mentioned above, or other SNPs, such as in US Patent Publication No. 20080131887 and PCT Publication No. WO2008/067551.

The phenotype profile of an individual may comprise a number of phenotypes. In particular, the assessment of a patient's risk of disease or other conditions such as likely drug response including metabolism, efficacy and/or safety, by the methods of the present disclosure allows for prognostic or diagnostic analysis of susceptibility to multiple, unrelated diseases and conditions, whether in symptomatic, presymptomatic or asymptomatic individuals, including carriers of one or more disease/condition predisposing alleles. Accordingly, these methods provide for general assessment of an individual's susceptibility to disease or condition without any preconceived notion of testing for a specific disease or condition. For example, the methods of the present disclosure allow for assessment of an individual's susceptibility to any of the several conditions listed in Tables 1, FIG. 4, 5, or 6, based on the individual's genomic profile. The assessment preferably provides information for 2 or more of these conditions, and more preferably, 3, 4, 5, 10, 20, 50, 100 or even more of these conditions. In preferred embodiments, the phenotype profile results from the application of at least 20 rules to the genomic profile of an individual. In other embodiments, at least 50 rules are applied to the genomic profile of an individual. A single rule for a phenotype may be applied for monogenic phenotypes. More than one rule may also be applied for a single phenotype, such as a multigenic phenotype or a monogenic phenotype wherein multiple genetic variants within a single gene affects the probability of having the phenotype.

Following an initial screening of an individual patient's genomic profile, updates of an individual's genotype correlations are made (or are available) through comparisons to additional nucleotide variants, such as SNPs, when such additional nucleotide variants become known. For example, step **110** may be performed periodically, for example, daily, weekly, or monthly by one or more people of ordinary skill in the field of genetics, who scan scientific literature for new genotype correlations. The new genotype correlations may then be further validated by a committee of one or more experts in the field. Step **112** may then also be periodically updated with new rules based on the new validated correlations.

The new rule may encompass a genotype or phenotype without an existing rule. For example, a genotype not correlated with any phenotype is discovered to correlate with a new or existing phenotype. A new rule may also be for a correlation between a phenotype for which no genotype has previously been correlated to. New rules may also be determined for genotypes and phenotypes that have existing rules. For example, a rule based on the correlation between genotype A and phenotype A exists. New research reveals genotype B correlates with phenotype A, and a new rule based on this correlation is made. Another example is phenotype B is discovered to be associated with genotype A, and thus a new rule may be made.

Rules may also be made on discoveries based on known correlations but not initially identified in published scientific literature. For example, it may be reported genotype C is correlated with phenotype C. Another publication reports genotype D is correlated with phenotype D. Phenotype C and D are related symptoms, for example phenotype C may be shortness of breath, and phenotype D is small lung capacity. A correlation between genotype C and phenotype D, or genotype D with phenotype C, may be discovered and validated through statistical means with existing stored genomic profiles of individuals with genotypes C and D, and phenotypes C and D, or by further research. A new rule may then be generated based on the newly discovered and validated correlation. In another embodiment, stored genomic profiles of a number of individuals with a specific or related phenotype may be studied to determine a genotype common to the individuals, and a correlation may be determined. A new rule may be generated based on this correlation.

Rules may also be made to modify existing rules. For example, correlations between genotypes and phenotypes may be partly determined by a known individual characteristic, such as ethnicity, ancestry, geography, gender, age, family history, or any other known phenotypes of the individual. Rules based on these known individual characteristics may be made and incorporated into an existing rule, to provide a modified rule. The choice of modified rule to be applied will be dependent on the specific individual factor of an individual. For example, a rule may be based on the probability an individual who has phenotype E is 35% when the individual has genotype E. However, if an individual is of a particular ethnicity, the probability is 5%. A new rule may be generated based on this result and applied to individuals with that particular ethnicity. Alternatively, the existing rule with a determination of 35% may be applied, and then another rule based on ethnicity for that phenotype is applied. The rules based on known individual characteristics may be determined from scientific literature or determined based on studies of stored genomic profiles. New rules may be added and applied to genomic profiles in step 114, as the new rules are developed, or they may be applied periodically, such as at least once a year.

Information of an individual's risk of disease can also be expanded as technology advances allow for finer resolution SNP genomic profiles. As indicated above, an initial SNP genomic profile readily can be generated using microarray technology for scanning of 500,000 SNPs. Given the nature of haplotype blocks, this number allows for a representative profile of all SNPs in an individual's genome. Nonetheless, there are approximately 10 million SNPs estimated to occur commonly in the human genome (the International HapMap Project; www.hapmap.org). As technological advances allow for practical, cost-efficient resolution of SNPs at a finer level of detail, such as microarrays of 1,000,000, 1,500,000, 2,000,000, 3,000,000, or more SNPs, or whole genomic sequencing, more detailed SNP genomic profiles can be generated. Likewise, cost-efficient analysis of finer SNP genomic profiles and updates to the master database of SNP-disease correlations will be enabled by advances in computational analytical methodology.

After generation of phenotype profile at step **116,** a subscriber or their health care manager may access their genomic or phenotype profiles via an on-line portal or website as in step **118.** Reports containing phenotype profiles and other information related to the phenotype and genomic profiles may also be provided to the subscriber or their health care manager, as in steps **120** and **122.** The reports may be printed, saved on the subscriber's computer, or viewed on-line.

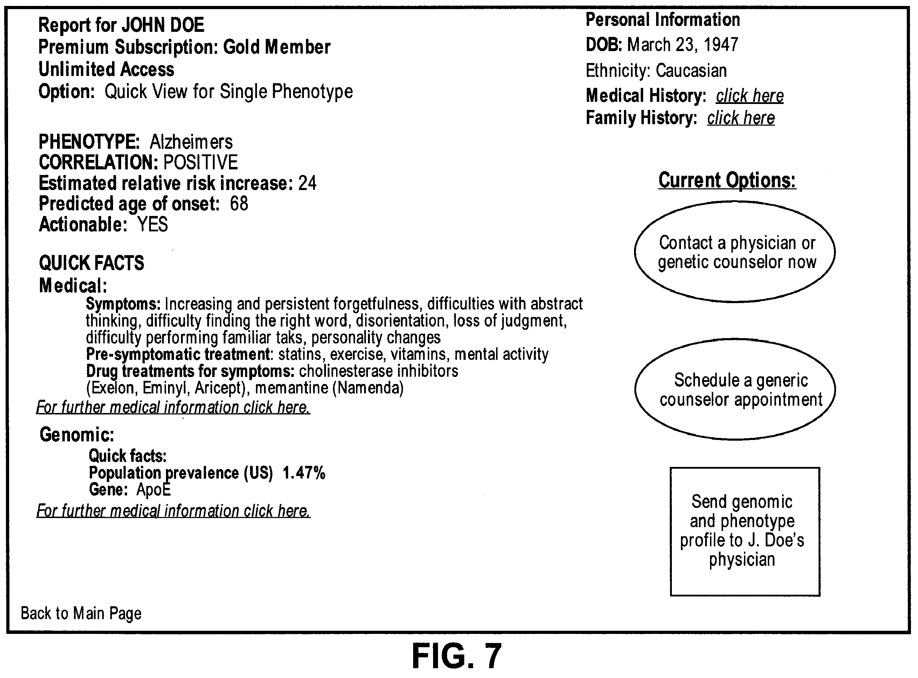

A sample on-line report is shown in **FIG. 7.** The subscriber may choose to display a single phenotype, or more than one phenotype. The subscriber may also have different viewing options, for example, as shown in **FIG. 7,** a "Quick View" option. The phenotype may be a medical condition and different treatments and symptoms in the quick report may link to other web pages that contain further information about the treatment. For example, by clicking on a drug, it will lead to website that contains information about dosages, costs, side effects, and effectiveness. It may also compare the drug to other treatments. The website may also contain a link leading to the drug manufacturer's website. Another link may provide an option for the subscriber to have a pharmacogenomic profile generated, which would include information such as their likely response to the drug based on their genomic profile. Links to alternatives to the drug may also be provided, such as preventative action such as fitness and weight loss, and links to diet supplements, diet plans, and to nearby health clubs, health clinics, health and wellness providers, day spas and the like may also be provided. Educational and informational videos, summaries of available treatments, possible remedies, and general recommendations may also be provided.

The on-line report may also provide links to schedule in-person physician or genetic counseling appointments or to access an on-line genetic counselor or physician, providing the opportunity for a subscriber to ask for more information regarding their phenotype profile. Links to on-line genetic counseling and physician questions may also be provided on the on-line report.

Reports may also be viewed in other formats such as a comprehensive view for a single phenotype, wherein more detail for each category is provided. For example, there may be more detailed statistics about the likelihood of the subscriber developing the phenotype, more information about the typical symptoms or phenotypes, such as sample symptoms for a medical condition, or the range of a physical non-medical condition such as height, or more information about the gene and genetic variant, such as the population incidence, for example in the world, or in different countries, or in different age ranges or genders. In another embodiment, the report may be of a "fun" phenotype, such as the similarity of an individual's genomic profile to that of a famous individual, such as Albert Einstein. The report may display a percentage similarity between the individual's genomic profile to that of Einstein's, and may further display a predicted IQ of Einstein and that of the individual's. Further information may include how the genomic profile of the general population and their IQ compares to that of the individual's and Einstein's.

In another embodiment, the report may display all phenotypes that have been correlated to the subscriber's genomic profile. In other embodiments, the report may display only the phenotypes that are positively correlated with an individual's genomic profile. In other formats, the individual may choose to display certain subgroups of phenotypes, such as only medical phenotypes, or only actionable medical phenotypes. For example, actionable phenotypes and their correlated genotypes, may include Crohn's disease (correlated with IL23R and CARD 15), Type 1 diabetes (correlated with HLA-DR/DQ), lupus (correlated HLA-DRB1), psoriasis (HLA-C), multiple sclerosis (HLA-DQA1), Graves disease (HLA-DRB1), rheumatoid arthritis (HLA-DRB1), Type 2 diabetes (TCF7L2), breast cancer (BRCA2), colon cancer (APC), episodic memory (KIBRA), and osteoporosis (COL1A1). The individual may also choose to display subcategories of phenotypes in their report, such as only inflammatory diseases for medical conditions, or only physical traits for non-medical conditions.

Information submitted by and conveyed to an individual may be secure and confidential, and access to such information may be controlled by the individual. Information derived from the complex genomic profile may be supplied to the individual as regulatory agency approved, understandable, medically relevant and/or high impact data. Information may also be of general interest, and not medically relevant. Information can be securely conveyed to the individual by several means including, but not restricted to, a portal interface and/or mailing. More preferably, information is securely (if so elected by the individual) provided to the individual by a portal interface, to which the individual has secure and confidential access. Such an interface is preferably provided by on-line, internet website access, or in the alternative, telephone or other means that allow private, secure, and readily available access. The genomic profiles, phenotype profiles, and reports are provided to an individual or their health care manager by transmission of the data over a network.

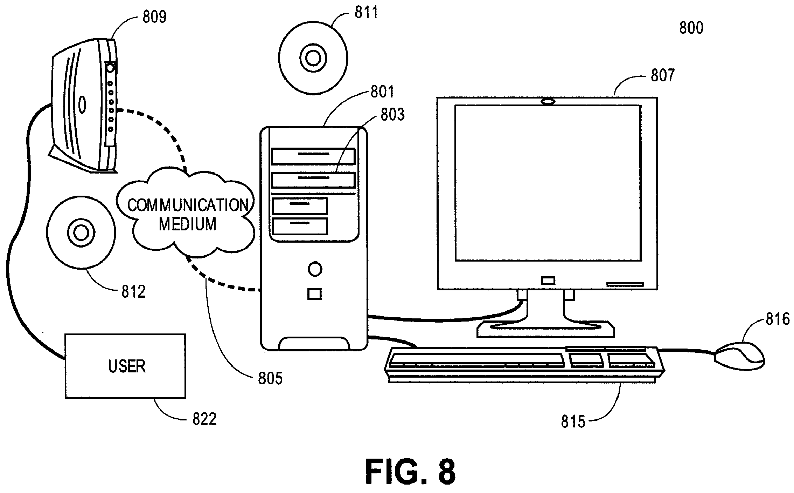

Accordingly, **FIG. 8** is a block diagram showing a representative example logic device through which a phenotype profile and report may be generated. **FIG. 8** shows a computer system (or digital device) **800** to receive and store genomic profiles, analyze genotype correlations, generate rules based on the analysis of genotype correlations, apply the rules to the genomic profiles, and produce a phenotype profile and report. The computer system **800** may be understood as a logical apparatus that can read instructions from media **811** and/or network port **805,** which can optionally be connected to server **809** having fixed media **812.** The system shown in **FIG.** 8 includes CPU **801,** disk drives **803,** optional input devices such as keyboard **815** and/or mouse **816** and optional monitor **807.** Data communication can be achieved through the indicated communication medium to a server **809** at a local or a remote location. The communication medium can include any means of transmitting and/or receiving data. For example, the communication medium can be a network connection, a wireless connection or an internet connection. Such a connection can provide for communication over the World Wide Web. It is envisioned that data relating to the present disclosure can be transmitted over such networks or connections for reception and/or review by a party **822.** The receiving party **822** can be but is not limited to an individual, a subscriber, a health care provider or a health care manager. In one embodiment, a computer-readable medium includes a medium suitable for transmission of a result of an analysis of a biological sample or a genotype correlation. The medium can include a result regarding a phenotype profile of an individual subject, wherein such a result is derived using the methods described herein.

A personal portal will preferably serve as the primary interface with an individual for receiving and evaluating genomic data. A portal will enable individuals to track the progress of their sample from collection through testing and results. Through portal access, individuals are introduced to relative risks for common genetic disorders based on their genomic profile. The subscriber may choose which rules to apply to their genomic profile through the portal.

In one embodiment, one or more web pages will have a list of phenotypes and next to each phenotype a box in which a subscriber may select to include in their phenotype profile. The phenotypes may be linked to information on the phenotype, to help the subscriber make an informed choice about the phenotype they want included in their phenotype profile. The webpage may also have phenotypes organized by disease groups, for example as actionable diseases or not. For example, a subscriber may choose actionable phenotypes only, such as HLA-DQA1 and celiac disease. The subscriber may also choose to display pre or post symptomatic treatments for the phenotypes. For example, the individual may choose actionable phenotypes with pre-symptomatic treatments (outside of increased screening), for celiac disease, a pre-symptomatic treatment of gluten free diet. Another example may be Alzheimer's, the pre-symptomatic treatment of statins, exercise, vitamins, and mental activity. Thrombosis is another example, with a pre-symptomatic treatment of avoid oral contraceptives and avoid sitting still for long periods of time. An example of a phenotype with an approved post symptomatic treatment is wet AMD, correlated with CFH, wherein individuals may obtain laser treatment for their condition.

The phenotypes may also be organized by type or class of disease or conditions, for example neurological, cardiovascular, endocrine, immunological, and so forth. Phenotypes may also be grouped as medical and non-medical phenotypes. Other groupings of phenotypes on the webpage may be by physical traits, physiological traits, mental traits, or emotional traits. The webpage may further provide a section in which a group of phenotypes are chosen by selection of one box. For example, a selection for all phenotypes, only medically relevant phenotypes, only non-medically relevant phenotypes, only actionable phenotypes, only non-actionable phenotypes, different disease group, or "fun" phenotypes. "Fun" phenotypes may include comparisons to celebrities or other famous individuals, or to other animals or even other organisms. A list of genomic profiles available for comparison may also be provided on the webpage for selection by the subscriber to compare to the subscriber's genomic profile.

The on-line portal may also provide a search engine, to help the subscriber navigate the portal, search for a specific phenotype, or search for specific terms or information revealed by their phenotype profile or report. Links to access partner services and product offerings may also be provided by the portal. Additional links to support groups, message boards, and chat rooms for individuals with a common or similar phenotype may also be provided. The on-line portal may also provide links to other sites with more information on the phenotypes in a subscriber's phenotype profile. The on-line portal may also provide a service to allow subscribers to share their phenotype profile and reports with friends, families, or health care managers. Subscribers may choose which phenotypes to show in the phenotype profile they want shared with their friends, families, or health care managers.

The phenotype profiles and reports provide a personalized genotype correlation to an individual. The genotype correlations provided to an individual can be used in determining personal health care and lifestyle choices. If a strong correlation is found between a genetic variant and a disease for which treatment is available, detection of the genetic variant may assist in deciding to begin treatment of the disease and/or monitoring of the individual. In the case where a statistically significant correlation exists but is not regarded as a strong correlation, an individual can review the information with a personal physician and decide an appropriate, beneficial course of action. Potential courses of action that could be beneficial to an individual in view of a particular genotype correlation include administration of therapeutic treatment, monitoring for potential need of treatment or effects of treatment, or making life-style changes in diet, exercise, and other personal habits/activities. For example, an actionable phenotype such as celiac disease may have a pre-symptomatic treatment of a gluten-free diet. Likewise, genotype correlation information could be applied through pharmacogenomics to predict the likely response an individual would have to treatment with a particular drug or regimen of drugs, such as the likely efficacy or safety of a particular drug treatment.

Subscribers may choose to provide the genomic and phenotype profiles to their health care managers, such as a physician or genetic counselor. The genomic and phenotype profiles may be directly accessed by the healthcare manager, by the subscriber printing out a copy to be given to the healthcare manager, or have it directly sent to the healthcare manager through the on-line portal, such as through a link on the on-line report.

Delivery of this pertinent information will empower patients to act in concert with their physician. In particular, discussions between patients and their physicians can be empowered through an individual's portal and links to medical information, and the ability to tie patient's genomic information into their medical records. Medical information may include prevention and wellness information. The information provided to the individual patient by the present disclosure will enable patients to make informed choices for their health care. In this manner, patients will be able to make choices that may help them avoid and/or delay diseases that their individual genomic profile (inherited DNA) makes more likely. In addition, patients will be able to employ a treatment regime that personally fits their specific medical needs. Individuals also will have the ability to access their genotype data should they develop an illness and need this information to help their physician form a therapeutic strategy.

Genotype correlation information could also be used in cooperation with genetic counseling to advise couples considering reproduction, and potential genetic concerns to the mother, father and/or child. Genetic counselors may provide information and support to subscribers with phenotype profiles that display an increased risk for specific conditions or diseases. They may interpret information about the disorder, analyze inheritance patterns and risks of recurrence, and review available options with the subscriber. Genetic counselors may also provide supportive counseling refer subscribers to community or state support services. Genetic counseling may be included with specific subscription plans. In some embodiments, genetic counseling may be scheduled within 24 hours of request and available during of hours such as evenings, Saturdays, Sundays, and/or holidays.

An individual's portal will also facilitate delivery of additional information beyond an initial screening. Individuals will be informed about new scientific discoveries that relate to their personal genetic profile, such as information on new treatments or prevention strategies for their current or potential conditions. The new discoveries may also be delivered to their healthcare managers. In preferred embodiments, the subscribers, or their healthcare providers are informed of new genotype correlations and new research about the phenotypes in the subscriber's phenotype profiles, by e-mail. In other embodiments, e-mails of "fun" phenotypes are sent to subscribers, for example, an e-mail may inform them that their genomic profile is 77% identical to that of Abraham Lincoln and that further information is available via an on-line portal.

The present disclosure also provides a system of computer code for generating new rules, modifying rules, combining rules, periodically updating the rule set with new rules, maintaining a database of genomic profile securely, applying the rules to the genomic profiles to determine phenotype profiles, and for generating reports. Computer code for notifying subscribers of new or revised correlations new or revised rules, and new or revised reports, for example with new prevention and wellness information, information about new therapies in development, or new treatments available.

<u>Business method</u>

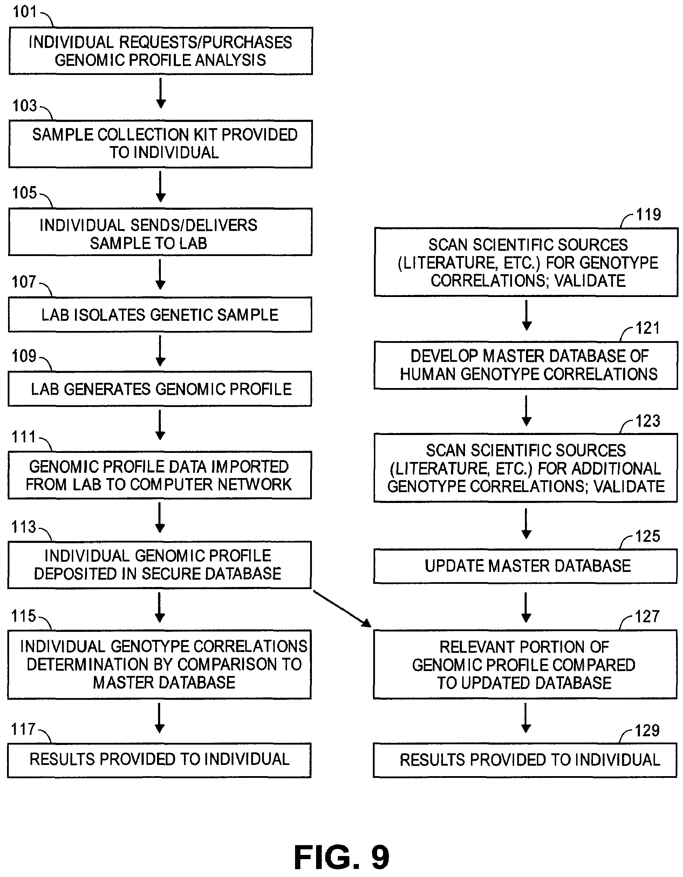

The present disclosure provides a business method of assessing an individual's genotype correlations based on comparison of the patient's genome profile against a clinically-derived database of established, medically relevant nucleotide variants. The present disclosure further provides a business method for using the stored genomic profile of the individual for assessing new correlations that were not initially known, to generate updated phenotype profiles for an individual, without the requirement of the individual submitting another biological sample. A flow chart illustrating the business method is in **FIG. 9.**

A revenue stream for the subject business method is generated in part at step 101, when an individual initially requests and purchases a personalized genomic profile for genotype correlations for a multitude of common human diseases, conditions, and physical states. A request and purchase can be made through any number of sources, including but not limited to, an on-line web portal, an on-line health service, and an individual's personal physician or similar source of personal medical attention. In an alternative embodiment, the genomic profile may be provided free, and the revenue stream is generated at a later step, such as step 103.

A subscriber, or customer, makes a request for purchase of a phenotype profile. In response to a request and purchase, a customer is provided a collection kit for a biological sample used for genetic sample isolation at step 103. When a request is made on-line, by telephone, or other source in which a collection kit is not readily physically available to the customer, a collection kit is provided by expedited delivery, such as courier service that provides same-day or overnight delivery. Included in the collection kit is a container for a sample, as well as packaging materials for expedited delivery of the sample to a laboratory for genomic profile generation. The kit may also include instructions for sending the sample to the sample processing facility, or laboratory, and instructions for accessing their genomic profile and phenotype profile, which may occur through an on-line portal.

As detailed above, genomic DNA can be obtained from any of a number of types of biological samples. Preferably, genomic DNA is isolated from saliva, using a commercially available collection kit such as that available from DNA Genotek. Use of saliva and such a kit allows for a non-invasive sample collection, as the customer conveniently provides a saliva sample in a container from a collection kit and then seals the container. In addition, a saliva sample can be stored and shipped at room temperature.

After depositing a biological sample into a collection or specimen container, a customer will deliver the sample to a laboratory for processing at step **105.** Typically, the customer may use packaging materials provided in the collection kit to deliver/send the sample to a laboratory by expedited delivery, such as same-day or overnight courier service.

The laboratory that processes the sample and generates the genomic profile may adhere to appropriate governmental agency guidelines and requirements. For example, in the United States, a processing laboratory may be regulated by one or more federal agencies such as the Food and Drug Administration (FDA) or the Centers for Medicare and Medicaid Services (CMS), and/or one or more state agencies. In the United States, a clinical laboratory may be accredited or approved under the Clinical Laboratory Improvement Amendments of 1988 (CLIA).

At step **107,** the laboratory processes the sample as previously described to isolate the genetic sample of DNA or RNA. Analysis of the isolated genetic sample and generation of a genomic profile is then performed at step **109.** Preferably, a genomic SNP profile is generated. As described above, several methodologies may be used to generate a SNP profile. Preferably, a high density array, such as the commercially available platforms from Affymetrix or Illumina, is used for SNP identification and profile generation. For example, a SNP profile may be generated using an Affymetrix GeneChip assay, as described above in more detail. As technology evolves, there may be other technology vendors who can generate high density SNP profiles. In another embodiment, a genomic profile for a subscriber will be the genomic sequence of the subscriber.

Following generation of an individual's genomic profile, the genotype data is preferably encrypted, imported at step **111,** and deposited into a secure database or vault at step **113,** where the information is stored for future reference. The genomic profile and related information may be confidential, with access to this proprietary information and the genomic profile limited as directed by the individual and/or his or her personal physician. Others, such as family and the genetic counselor of the individual may also be permitted access by the subscriber.

The database or vault may be located on-site with the processing laboratory. Alternatively, the database may be located at a separate location. In this scenario, the genomic profile data generated by the processing lab can be imported at step **111** to a separate facility that contains the database.

After an individual's genomic profile is generated, the individual's genetic variations are then compared against a clinically-derived database of established, medically relevant genetic variants in step **115.** Alternatively, the genotype correlations may not be medically relevant but still incorporated into the database of genotype correlations, for example, physical traits such as eye color, or "fun" phenotypes such as genomic profile similarity to a celebrity.

The medically relevant SNPs may have been established through the scientific literature and related sources. The non-SNP genetic variants may also be established to be correlated with phenotypes. Generally, the correlation of SNPs to a given disease is established by comparing the haplotype patterns of a group of people known to have the disease to a group of people without the disease. By analyzing many individuals, frequencies of polymorphisms in a population can be determined, and in turn these genotype frequencies can be associated with a particular phenotype, such as a disease or a condition. Alternatively, the phenotype may be a non-medical condition.

The relevant SNPs and non-SNP genetic variants may also be determined through analysis of the stored genomic profiles of individuals rather than determined by available published literature. Individuals with stored genomic profiles may disclose phenotypes that have previously been determined. Analysis of the genotypes and disclosed phenotypes of the individuals may be compared to those without the phenotypes to determine a correlation that may then be applied to other genomic profiles. Individuals that have their genomic profiles determined may fill out questionnaires about phenotypes that have previously been determined. Questionnaires may contain questions about medical and non-medical conditions, such as diseases previously diagnosed, family history of medical conditions, lifestyle, physical traits, mental traits, age, social life, environment and the like.

In one embodiment, an individual may have their genomic profile determined free of charge if they fill out a questionnaire. In some embodiments, the questionnaires are to be filled out periodically by the individuals in order to have free access to their phenotype profile and reports. In other embodiments, the individuals that fill out the questionnaires may be entitled to a subscription upgrade, such that they have more access than their previous subscription level, or they may purchase or renew a subscription at a reduced cost.

All information deposited in the database of medically relevant genetic variants at step **121** is first approved by a research/clinical advisory board for scientific accuracy and importance, coupled with review and oversight by an appropriate governmental agency if warranted at step **119.** For example, in the United States, the FDA may provide oversight through approval of algorithms used for validation of genetic variant (typically SNP, transcript level, or mutation) correlative data. At step **123,** scientific literature and other relevant sources are monitored for additional genetic variant-disease or condition correlations, and following validation of their accuracy and importance, along with governmental agency review and approval, these additional genotype correlations are added to the master database at step **125.**

The database of approved, validated medically-relevant genetic variants, coupled with a genome-wide individual profile, will advantageously allow genetic risk-assessment to be performed for a large number of diseases or conditions. Following compilation of an individual's genomic profile, individual genotype correlations can be determined through comparison of the individual's nucleotide (genetic) variants or markers with a database of human nucleotide variants that have been correlated to a particular phenotype, such as a disease, condition, or physical state. Through comparison of an individual's genomic profile to the master database of genotype correlations, the individual can be informed whether they are found to be positive or negative for a genetic risk factor, and to what degree. An individual will receive relative risk and/or predisposition data on a wide range of scientifically validated disease states (e.g., Alzheimer's, cardiovascular disease, blood clotting). For example, genotype correlations in Table 1 may be included. In addition, SNP disease correlations in the database may include, but are not limited to, those correlations shown in **FIG. 4.** Other correlations from **FIGS. 5** and **6** may also be included. The subject business method therefore provides analysis of risk to a multitude of diseases and conditions without any preconceived notion of what those diseases and conditions might entail.

In other embodiments, the genotype correlations that are coupled to the genome wide individual profile are non-medically relevant phenotypes, such as "fun" phenotypes or physical traits such as hair color. In preferred embodiments, a rule or rule set is applied to the genomic profile or SNP profile of an individual, as described above. Application of the rules to a genomic profile generates a phenotype profile for the individual.

Accordingly, the master database of human genotype correlations is expanded with additional genotype correlations as new correlations become discovered and validated. An update can be made by accessing pertinent information from the individual's genomic profile stored in a database as desired or appropriate. For example, a new genotype correlation that becomes known may be based on a particular gene variant. Determination of whether an individual may be susceptible to that new genotype correlation can then be made by retrieving and comparing just that gene portion of the individual's entire genomic profile.

The results of the genomic query preferably are analyzed and interpreted so as to be presented to the individual in an understandable format. At step **117,** the results of an initial screening are then provided to the patient in a secure, confidential form, either by mailing or through an on-line portal interface, as detailed above.

The report may contain the phenotype profile as well as genomic information about the phenotypes in the phenotype profile, for example basic genetics about the genes involved or the statistics of the genetic variants in different populations. Other information based on the phenotype profile that may be included in the report are prevention strategies, wellness information, therapies, symptom awareness, early detection schemes, intervention schemes, and refined identification and sub-classification of the phenotypes. Following an initial screening of an individual's genomic profile, controlled, moderated updates are or can be made.

Updates of an individual's genomic profile are made or are available in conjunction with updates to the master database as new genotype correlations emerge and are both validated and approved. New rules based on the new genotype correlations may be applied to the initial genomic profile to provide updated phenotype profiles. An updated genotype correlation profile can be generated by comparing the relevant portion of the individual's genomic profile to a new genotype correlation at step **127.** For example, if a new genotype correlation is found based on variation in a particular gene, then that gene portion of the individual's genomic profile can be analyzed for the new genotype correlation. In such a case, one or more new rules may be applied to generate an updated phenotype profile, rather than an entire rule set with rules that had already been applied. The results of the individual's updated genotype correlations are provided in a secure manner at step **129.**

Initial and updated phenotype profiles may be a service provided to subscribers or customers. Varying levels of subscriptions to genomic profile analysis and combinations thereof can be provided. Likewise, subscription levels can vary to provide individuals choices of the amount of service they wish to receive with their genotype correlations. Thus, the level of service provided would vary with the level of service subscription purchased by the individual.

An entry level subscription for a subscriber may include a genomic profile and an initial phenotype profile. This may be a basic subscription level. Within the basic subscription level may be varying levels of service. For example, a particular subscription level could provide references for genetic counseling, physicians with particular expertise in treating or preventing a particular disease, and other service options. Genetic counseling may be obtained on-line or by telephone. In another embodiment, the price of the subscription may depend on the number of phenotypes an individual chooses for their phenotype profile. Another option may be whether the subscriber chooses to access on-line genetic counseling.

In another scenario, a subscription could provide for an initial genome-wide, genotype correlation, with maintenance of the individual's genomic profile in a database; such database may be secure if so elected by the individual. Following this initial analysis, subsequent analyses and additional results could be made upon request and additional payment by the individual. This may be a premium level of subscription.

Updates of an individual's risks may be performed and corresponding information made available to individuals on a subscription basis. The updates may be available to subscribers who purchase the premium level of subscription. Subscription to genotype correlation analysis can provide updates with a particular category or subset of new genotype correlations according to an individual's preferences. For example, an individual might only wish to learn of genotype correlations for which there is a known course of treatment or prevention. To aid an individual in deciding whether to have an additional analysis performed, the individual can be provided with information regarding additional genotype correlations that have become available. Such information can be conveniently mailed or e-mailed to a subscriber.

Within the premium subscription, there may be further levels of service, such as those mentioned in the basic subscription. Other subscription models may be provided within the premium level. For example, the highest level may provide a subscriber to unlimited updates and reports. The subscriber's profile may be updated as new correlations and rules are determined. At this level, subscribers may also permit access to unlimited number of individuals, such as family members and health care managers. The subscribers may also have unlimited access to on-line genetic counselors and physicians.

The next level of subscription within the premium level may provide more limited aspects, for example a limited number of updates. The subscriber may have a limited number of updates for their genomic profile within a subscription period, for example, 4 times a year. In another subscription level, the subscriber may have their stored genomic profile updated once a week, once a month, or once a year. In another embodiment, the subscriber may only have a limited number of phenotypes they may choose to update their genomic profile against.

A personal portal will also conveniently allow an individual to maintain a subscription to risk or correlation updates and information updates or alternatively, make requests for updated risk assessment and information. As described above, varying subscription levels could be provided to allow individuals choices of various levels of genotype correlation results and updates and may different subscription levels may be chosen by the subscriber via their personal portal.

Any of these subscription options will contribute to the revenue stream for the subject business method. The revenue stream for the subject business method will also be added by the addition of new customers and subscribers, wherein the new genomic profiles are added to the database.

Table 1: Representative genes having genetic variants correlated with a phenotype.

| Gene | Phenotype |
|---|---|
| A2M | Alzheimer's Disease |
| ABCA1 | cholesterol, HDL |
| ABCB1 | HIV |
| ABCB1 | epilepsy |
| ABCB1 | kidney transplant complications |
| ABCB1 | digoxin, serum concentration |
| ABCB1 | Crohn's disease; ulcerative colitis |
| ABCB1 | Parkinson's disease |
| ABCC8 | Type 2 diabetes |

(continued)

| Gene | Phenotype |
|---|---|
| ABCC8 | diabetes, type 2 |
| ABO | myocardial infarct |
| ACADM | medium-chain acyl-CoA dehydrogenase deficiency |
| ACDC | Type 2, diabetes |
| ACE | Type 2 diabetes |
| ACE | hypertension |
| ACE | Alzheimer's Disease |
| ACE | myocardial infarction |
| ACE | cardiovascular |
| ACE | left ventricular hypertrophy |
| ACE | coronary artery disease |
| ACE | atherosclerosis, coronary |
| ACE | retinopathy, diabetic |
| ACE | systemic lupus erythematosus |
| ACE | blood pressure, arterial |
| ACE | erectile dysfunction |
| ACE | Lupus |
| ACE | polycystic kidney disease |
| ACE | stroke |
| ACP1 | diabetes, type 1 |
| ACSM1 (LIP)c | cholesterol levels |
| ADAM33 | asthma |
| ADD1 | hypertension |
| ADD1 | blood pressure, arterial |
| ADH1B | alcohol abuse |
| ADH1C | alcohol abuse |
| ADIPOQ | diabetes, type 2 |
| ADIPOQ | obesity |
| ADORA2A | panic disorder |
| ADRB1 | hypertension |
| ADRB1 | heart failure |
| ADRB2 | asthma |
| ADRB2 | hypertension |
| ADRB2 | obesity |
| ADRB2 | blood pressure, arterial |
| ADRB2 | Type 2 Diabetes |
| ADRB3 | obesity |
| ADRB3 | Type 2 Diabetes |

(continued)

| Gene | Phenotype |
|---|---|
| ADRB3 | hypertension |
| AGT | hypertension |
| AGT | Type 2 diabetes |
| AGT | Essential Hypertension |
| AGT | myocardial infarction |
| AGTR1 | hypertension |
| AGTR2 | hypertension |
| AHR | breast cancer |
| ALAD | lead toxicity |
| ALDH2 | alcoholism |
| ALDH2 | alcohol abuse |
| ALDH2 | colorectal cancer |
| ALDRL2 | Type 2 diabetes |
| ALOX5 | asthma |
| ALOX5AP | asthma |
| APBB1 | Alzheimer’s Disease |
| APC | colorectal cancer |
| APEX1 | lung cancer |
| APOA1 | atherosclerosis, coronary |
| APOA1 | cholesterol, HDL |
| APOA1 | coronary artery disease |
| APOA1 | Type 2 diabetes |
| APOA4 | Type 2 diabetes |
| APOA5 | triglycerides |
| APOA5 | atherosclerosis, coronary |
| APOB | hypercholesterolemia |
| APOB | obesity |
| APOB | cardiovascular |
| APOB | coronary artery disease |
| APOB | coronary heart disease |
| APOB | Type 2 diabetes |
| APOC1 | Alzheimer’s Disease |
| APOC3 | triglycerides |
| APOC3 | Type 2 Diabetes |
| APOE | Alzheimer’s Disease |
| APOE | Type 2 diabetes |
| APOE | multiple sclerosis |
| APOE | atherosclerosis, coronary |

(continued)

| Gene | Phenotype |
|---|---|
| APOE | Parkinson's disease |
| APOE | coronary heart disease |
| APOE | myocardial infarction |
| APOE | stroke |
| APOE | Alzheimer's disease |
| APOE | coronary artery disease |
| APP | Alzheimer's Disease |
| AR | prostate cancer |
| AR | breast cancer |
| ATM | breast cancer |
| ATP7B | Wilson disease |
| ATXN8OS | spinocerebellar ataxia |
| BACE1 | Alzheimer's Disease |
| BCHE | Alzheimer's Disease |
| BDKRB2 | hypertension |
| BDNF | Alzheimer's Disease |
| BDNF | bipolar disorder |
| BDNF | Parkinson's disease |
| BDNF | schizophrenia |
| BDNF | memory |
| BGLAP | bone density |
| BRAF | thyroid cancer |
| BRCA1 | breast cancer |
| BRCA1 | breast cancer; ovarian cancer |
| BRCA1 | ovarian cancer |
| BRCA2 | breast cancer |
| BRCA2 | breast cancer; ovarian cancer |
| BRCA2 | ovarian cancer |
| BRIP1 | breast cancer |
| C4A | systemic lupus erythematosus |
| CALCR | bone density |
| CAMTA1 | episodic memory |
| CAPN10 | diabetes, type 2 |
| CAPN10 | Type 2 diabetes |
| CAPN3 | muscular dystrophy |
| CARD15 | Crohn's disease |
| CARD15 | Crohn's disease; ulcerative colitis |
| CARD15 | Inflammatory Bowel Disease |

(continued)

| Gene | Phenotype |
|---|---|
| CART | obesity |
| CASR | bone density |
| CCKAR | schizophrenia |
| CCL2 | systemic lupus erythematosus |
| CCL5 | HIV |
| CCL5 | asthma |
| CCND1 | colorectal cancer |
| CCR2 | HIV |
| CCR2 | HIV infection |
| CCR2 | hepatitis C |
| CCR2 | myocardial infarct |
| CCR3 | Asthma |
| CCR5 | HIV |
| CCR5 | HIV infection |
| CCR5 | hepatitis C |
| CCR5 | asthma |
| CCR5 | multiple sclerosis |
| CD14 | atopy |
| CD14 | asthma |
| CD14 | Crohn's disease |
| CD14 | Crohn's disease; ulcerative colitis |
| CD14 | periodontitis |
| CD14 | Total IgE |
| CDH1 | prostate cancer |
| CDH1 | colorectal cancer |
| CDKN2A | melanoma |
| CDSN | psoriasis |
| CEBPA | leukemia, myeloid |
| CETP | atherosclerosis, coronary |
| CETP | coronary heart disease |
| CETP | hypercholesterolemia |
| CFH | macular degeneration |
| CFTR | cystic fibrosis |
| CFTR | pancreatitis |
| CFTR | Cystic Fibrosis |
| CHAT | Alzheimer's Disease |
| CHEK2 | breast cancer |
| CHRNA7 | schizophrenia |

(continued)

| Gene | Phenotype |
|---|---|
| CMA1 | atopic dermatitis |
| CNR1 | schizophrenia |
| COL1A1 | bone density |
| COL1A1 | osteoporosis |
| COL1A2 | bone density |
| COL2A1 | Osteoarthritis |
| COMT | schizophrenia |
| COMT | breast cancer |
| COMT | Parkinson's disease |
| COMT | bipolar disorder |
| COMT | obsessive compulsive disorder |
| COMT | alcoholism |
| CR1 | systemic lupus erythematosus |
| CRP | C-reactive protein |
| CST3 | Alzheimer's Disease |
| CTLA4 | Type 1 diabetes |
| CTLA4 | Graves' disease |
| CTLA4 | multiple sclerosis |
| CTLA4 | rheumatoid arthritis |
| CTLA4 | systemic lupus erythematosus |
| CTLA4 | lupus erythematosus |
| CTLA4 | celiac disease |
| CTSD | Alzheimer's Disease |
| CX3CR1 | HIV |
| CXCL12 | HIV |
| CXCL12 | HIV infection |
| CYBA | atherosclerosis, coronary |
| CYBA | hypertension |
| CYP11B2 | hypertension |
| CYP11B2 | left ventricular hypertrophy |
| CYP17A1 | breast cancer |
| CYP17A1 | prostate cancer |
| CYP17A1 | endometriosis |
| CYP17A1 | endometrial cancer |
| CYP19A1 | breast cancer |
| CYP19A1 | prostate cancer |
| CYP19A1 | endometriosis |
| CYP1A1 | lung cancer |

(continued)

| Gene | Phenotype |
|---|---|
| CYP1A1 | breast cancer |
| CYP1A1 | Colorectal Cancer |
| CYP1A1 | prostate cancer |
| CYP1A1 | esophageal cancer |
| CYP1A1 | endometriosis |
| CYP1A1 | cytogenetic studies |
| CYP1A2 | schizophrenia |
| CYP1A2 | colorectal cancer |
| CYP1B1 | breast cancer |
| CYP1B1 | glaucoma |
| CYP1B1 | prostate cancer |
| CYP21A2 | 21-hydroxylase deficiency |
| CYP21A2 | congenital adrenal hyperplasia |
| CYP21A2 | adrenal hyperplasia, congenital |
| CYP2A6 | smoking behavior |
| CYP2A6 | nicotine |
| CYP2A6 | lung cancer |
| CYP2C19 | H. pylori infection |
| CYP2C19 | phenytoin |
| CYP2C19 | gastric disease |
| CYP2C8 | malaria, plasmodium falciparum |
| CYP2C9 | anticoagulant complications |
| CYP2C9 | warfarin sensitivity |
| CYP2C9 | warfarin therapy, response to |
| CYP2C9 | colorectal cancer |
| CYP2C9 | phenytoin |
| CYP2C9 | acenocoumarol response |
| CYP2C9 | coagulation disorder |
| CYP2C9 | hypertension |
| CYP2D6 | colorectal cancer |
| CYP2D6 | Parkinson's disease |
| CYP2D6 | CYP2D6 poor metabolizer phenotype |
| CYP2E1 | lung cancer |
| CYP2E1 | colorectal cancer |
| CYP3A4 | prostate cancer |
| CYP3A5 | prostate cancer |
| CYP3A5 | esophageal cancer |
| CYP46A1 | Alzheimer's Disease |

(continued)

| Gene | Phenotype |
|---|---|
| DBH | schizophrenia |
| DHCR7 | Smith-Lemli-Opitz syndrome |
| DISCI | schizophrenia |
| DLST | Alzheimer's Disease |
| DMD | muscular dystrophy |
| DRD2 | alcoholism |
| DRD2 | schizophrenia |
| DRD2 | smoking behavior |
| DRD2 | Parkinson's disease |
| DRD2 | tardive dyskinesia |
| DRD3 | schizophrenia |
| DRD3 | tardive dyskinesia |
| DRD3 | bipolar disorder |
| DRD4 | attention deficit hyperactivity disorder |
| DRD4 | schizophrenia |
| DRD4 | novelty seeking |
| DRD4 | ADHD |
| DRD4 | personality traits |
| DRD4 | heroin abuse |
| DRD4 | alcohol abuse |
| DRD4 | alcoholism |
| DRD4 | personality disorders |
| DTNBP1 | schizophrenia |
| EDN1 | hypertension |
| EGFR | lung cancer |
| ELAC2 | prostate cancer |
| ENPP1 | Type 2 diabetes |
| EPHB2 | prostate cancer |
| EPHX1 | lung cancer |
| EPHX1 | colorectal cancer |
| EPHX1 | cytogenetic studies |
| EPHX1 | chronic obstructive pulmonary disease/COPD |
| ERBB2 | breast cancer |
| ERCC1 | lung cancer |
| ERCC1 | colorectal cancer |
| ERCC2 | lung cancer |
| ERCC2 | cytogenetic studies |
| ERCC2 | bladder cancer |

(continued)

| Gene | Phenotype |
|---|---|
| ERCC2 | colorectal cancer |
| ESR1 | bone density |
| ESR1 | bone mineral density |
| ESR1 | breast cancer |
| ESR1 | endometriosis |
| ESR1 | osteoporosis |
| ESR2 | bone density |
| ESR2 | breast cancer |
| estrogen receptor | bone mineral density |
| F2 | coronary heart disease |
| F2 | stroke |
| F2 | thromboembolism, venous |
| F2 | preeclampsia |
| F2 | thrombosis |
| F5 | thromboembolism, venous |
| F5 | preeclampsia |
| F5 | myocardial infarct |
| F5 | stroke |
| F5 | stroke, ischemic |
| F7 | atherosclerosis, coronary |
| F7 | myocardial infarct |
| F8 | hemophilia |
| F9 | hemophilia |
| FABP2 | Type 2 diabetes |
| FAS | Alzheimer's Disease |
| FASLG | multiple sclerosis |
| FCGR2A | systemic lupus erythematosus |
| FCGR2A | lupus erythematosus |
| FCGR2A | periodontitis |
| FCGR2A | rheumatoid arthritis |
| FCGR2B | lupus erythematosus |
| FCGR2B | systemic lupus erythematosus |
| FCGR3A | systemic lupus erythematosus |
| FCGR3A | lupus erythematosus |
| FCGR3A | periodontitis |
| FCGR3A | arthritis |
| FCGR3A | rheumatoid arthritis |
| FCGR3B | periodontitis |

(continued)

| Gene | Phenotype |
|---|---|
| FCGR3B | periodontal disease |
| FCGR3B | lupus erythematosus |
| FGB | fibrinogen |
| FGB | myocardial infarction |
| FGB | coronary heart disease |
| FLT3 | leukemia, myeloid |
| FLT3 | leukemia |
| FMR1 | Fragile X syndrome |
| FRAXA | Fragile X Syndrome |
| FUT2 | H. pylori infection |
| FVL | Factor V Leiden |
| G6PD | G6PD deficiency |
| G6PD | hyperbilirubinemia |
| GABRA5 | bipolar disorder |
| GBA | Gaucher disease |
| GBA | Parkinson’s disease |
| GCGR (FAAH, ML4R, UCP2) | body mass/obesity |
| GCK | Type 2 diabetes |
| GCLM (F12, TLR4) | atherosclerosis, myocardial infarction |
| GDNF | schizophrenia |
| GHRL | obesity |
| GJB1 | Charcot-Marie-Tooth disease |
| GJB2 | deafness |
| GJB2 | hearing loss, sensorineural nonsyndromic |
| GJB2 | hearing loss, sensorineural |
| GJB2 | hearing loss/deafness |
| GJB6 | hearing loss, sensorineural nonsyndromic |
| GJB6 | hearing loss/deafness |
| GNAS | hypertension |
| GNB3 | hypertension |
| GPX1 | lung cancer |
| GRIN1 | schizophrenia |
| GRIN2B | schizophrenia |
| GSK3B | bipolar disorder |
| GSTM1 | lung cancer |
| GSTM1 | colorectal cancer |
| GSTM1 | breast cancer |
| GSTM1 | prostate cancer |

(continued)

| Gene | Phenotype |
|---|---|
| GSTM1 | cytogenetic studies |
| GSTM1 | bladder cancer |
| GSTM1 | esophageal cancer |
| GSTM1 | head and neck cancer |
| GSTM1 | leukemia |
| GSTM1 | Parkinson's disease |
| GSTM1 | stomach cancer |
| GSTP1 | Lung cancer |
| GSTP1 | colorectal cancer |
| GSTP1 | breast cancer |
| GSTP1 | cytogenetic studies |
| GSTP1 | prostate cancer |
| GSTT1 | lung cancer |
| GSTT1 | colorectal cancer |
| GSTT1 | breast cancer |
| GSTT1 | prostate cancer |
| GSTT1 | Bladder Cancer |
| GSTT1 | cytogenetic studies |
| GSTT1 | asthma |
| GSTT1 | benzene toxicity |
| GSTT1 | esophageal cancer |
| GSTT1 | head and neck cancer |
| GYS1 | Type 2 diabetes |
| HBB | thalassemia |
| HBB | thalassemia, beta |
| HD | Huntington's disease |
| HFE | Hemochromatosis |
| HFE | iron levels |
| HFE | colorectal cancer |
| HK2 | Type 2 diabetes |
| HLA | rheumatoid arthritis |
| HLA | Type 1 diabetes |
| HLA | Behcet's Disease |
| HLA | celiac disease |
| HLA | psoriasis |
| HLA | Graves disease |
| HLA | multiple sclerosis |
| HLA | schizophrenia |

(continued)

| Gene | Phenotype |
|---|---|
| HLA | asthma |
| HLA | diabetes mellitus |
| HLA | Lupus |
| HLA-A | leukemia |
| HLA-A | HIV |
| HLA-A | diabetes, type 1 |
| HLA-A | graft-versus-host disease |
| HLA-A | multiple sclerosis |
| HLA-B | leukemia |
| HLA-B | Behcet's Disease |
| HLA-B | celiac disease |
| HLA-B | diabetes, type 1 |
| HLA-B | graft-versus-host disease |
| HLA-B | sarcoidosis |
| HLA-C | psoriasis |
| HLA-DPA1 | measles |
| HLA-DPB1 | diabetes, type 1 |
| HLA-DPB1 | Asthma |
| HLA-DQA1 | diabetes, type 1 |
| HLA-DQA1 | celiac disease |
| HLA-DQA1 | cervical cancer |
| HLA-DQA1 | asthma |
| HLA-DQA1 | multiple sclerosis |
| HLA-DQA1 | diabetes, type 2; diabetes, type 1 |
| HLA-DQA1 | lupus erythematosus |
| HLA-DQA1 | pregnancy loss, recurrent |
| HLA-DQA1 | psoriasis |
| HLA-DQB1 | diabetes, type 1 |
| HLA-DQB1 | celiac disease |
| HLA-DQB1 | multiple sclerosis |
| HLA-DQB1 | cervical cancer |
| HLA-DQB1 | lupus erythematosus |
| HLA-DQB1 | pregnancy loss, recurrent |
| HLA-DQB1 | arthritis |
| HLA-DQB1 | asthma |
| HLA-DQB1 | HIV |
| HLA-DQB1 | lymphoma |
| HLA-DQB1 | tuberculosis |

(continued)

| Gene | Phenotype |
|---|---|
| HLA-DQB1 | rheumatoid arthritis |
| HLA-DQB1 | diabetes, type 2 |
| HLA-DQB1 | graft-versus-host disease |
| HLA-DQB1 | narcolepsy |
| HLA-DQB1 | arthritis, rheumatoid |
| HLA-DQB1 | cholangitis, sclerosing |
| HLA-DQB1 | diabetes, type 2; diabetes, type 1 |
| HLA-DQB1 | Graves' disease |
| HLA-DQB1 | hepatitis C |
| HLA-DQB1 | hepatitis C, chronic |
| HLA-DQB1 | malaria |
| HLA-DQB1 | malaria, plasmodium falciparum |
| HLA-DQB1 | melanoma |
| HLA-DQB1 | psoriasis |
| HLA-DQB1 | Sjogren's syndrome |
| HLA-DQB1 | systemic lupus erythematosus |
| HLA-DRB1 | diabetes, type 1 |
| HLA-DRB1 | multiple sclerosis |
| HLA-DRB1 | systemic lupus erythematosus |
| HLA-DRB1 | rheumatoid arthritis |
| HLA-DRB1 | cervical cancer |
| HLA-DRB1 | arthritis |
| HLA-DRB1 | celiac disease |
| HLA-DRB1 | lupus erythematosus |
| HLA-DRB1 | sarcoidosis |
| HLA-DRB1 | HIV |
| HLA-DRB1 | tuberculosis |
| HLA-DRB1 | Graves' disease |
| HLA-DRB1 | lymphoma |
| HLA-DRB1 | psoriasis |
| HLA-DRB1 | asthma |
| HLA-DRB1 | Crohn's disease |
| HLA-DRB1 | graft-versus-host disease |
| HLA-DRB1 | hepatitis C, chronic |
| HLA-DRB1 | narcolepsy |
| HLA-DRB1 | sclerosis, systemic |
| HLA-DRB1 | Sjogren's syndrome |
| HLA-DRB1 | Type 1 diabetes |

(continued)

| Gene | Phenotype |
|---|---|
| HLA-DRB1 | arthritis, rheumatoid |
| HLA-DRB1 | cholangitis, sclerosing |
| HLA-DRB1 | diabetes, type 2; diabetes, type 1 |
| HLA-DRB1 | H. pylori infection |
| HLA-DRB1 | hepatitis C |
| HLA-DRB1 | juvenile arthritis |
| HLA-DRB1 | leukemia |
| HLA-DRB1 | malaria |
| HLA-DRB1 | melanoma |
| HLA-DRB1 | pregnancy loss, recurrent |
| HLA-DRB3 | psoriasis |
| HLA-G | pregnancy loss, recurrent |
| HMOX1 | atherosclerosis, coronary |
| HNF4A | diabetes, type 2 |
| HNF4A | type 2 diabetes |
| HSD11B2 | hypertension |
| HSD17B1 | breast cancer |
| HTR1A | depressive disorder, major |
| HTR1B | alcohol dependence |
| HTR1B | alcoholism |
| HTR2A | memory |
| HTR2A | schizophrenia |
| HTR2A | bipolar disorder |
| HTR2A | depression |
| HTR2A | depressive disorder, major |
| HTR2A | suicide |
| HTR2A | Alzheimer's Disease |
| HTR2A | anorexia nervosa |
| HTR2A | hypertension |
| HTR2A | obsessive compulsive disorder |
| HTR2C | schizophrenia |
| HTR6 | Alzheimer's Disease |
| HTR6 | schizophrenia |
| HTRA1 | wet age-related macular degeneration |
| IAPP | Type 2 Diabetes |
| IDE | Alzheimer's Disease |
| IFNG | tuberculosis |
| IFNG | Type 1 diabetes |

(continued)

| Gene | Phenotype |
|---|---|
| IFNG | graft-versus-host disease |
| IFNG | hepatitis B |
| IFNG | multiple sclerosis |
| IFNG | asthma |
| IFNG | breast cancer |
| IFNG | kidney transplant |
| IFNG | kidney transplant complications |
| IFNG | longevity |
| IFNG | pregnancy loss, recurrent |
| IGFBP3 | breast cancer |
| IGFBP3 | prostate cancer |
| IL10 | systemic lupus erythematosus |
| IL10 | asthma |
| IL10 | graft-versus-host disease |
| IL10 | HIV |
| IL10 | kidney transplant |
| IL10 | kidney transplant complications |
| IL10 | hepatitis B |
| IL10 | juvenile arthritis |
| IL10 | longevity |
| IL10 | multiple sclerosis |
| IL10 | pregnancy loss, recurrent |
| IL10 | rheumatoid arthritis |
| IL10 | tuberculosis |
| IL12B | Type 1 diabetes |
| IL12B | asthma |
| IL13 | asthma |
| IL13 | atopy |
| IL13 | chronic obstructive pulmonary disease/COPD |
| IL13 | Graves' disease |
| IL1A | periodontitis |
| IL1A | Alzheimer's Disease |
| IL1B | periodontitis |
| IL1B | Alzheimer's Disease |
| IL1B | stomach cancer |
| IL1R1 | Type 1 diabetes |
| IL1RN | stomach cancer |
| IL2 | asthma; eczema; allergic disease |

(continued)

| Gene | Phenotype |
|---|---|
| IL4 | Asthma |
| IL4 | atopy |
| IL4 | HIV |
| IL4R | asthma |
| IL4R | atopy |
| IL4R | Total serum IgE |
| IL6 | Bone Mineralization |
| IL6 | kidney transplant |
| IL6 | kidney transplant complications |
| IL6 | Longevity |
| IL6 | multiple sclerosis |
| IL6 | bone density |
| IL6 | bone mineral density |
| IL6 | Colorectal Cancer |
| IL6 | juvenile arthritis |
| IL6 | rheumatoid arthritis |
| IL9 | asthma |
| INHA | premature ovarian failure |
| INS | Type 1 diabetes |
| INS | Type 2 diabetes |
| INS | diabetes, type 1 |
| INS | obesity |
| INS | prostate cancer |
| INSIG2 | obesity |
| INSR | Type 2 diabetes |
| INSR | hypertension |
| INSR | polycystic ovary syndrome |
| IPF1 | diabetes, type 2 |
| IRS 1 | Type 2 diabetes |
| IRS 1 | diabetes, type 2 |
| IRS2 | diabetes, type 2 |
| ITGB3 | myocardial infarction |
| ITGB3 | atherosclerosis, coronary |
| ITGB3 | coronary heart disease |
| ITGB3 | myocardial infarct |
| KCNE1 | EKG, abnormal |
| KCNE2 | EKG, abnormal |
| KCNH2 | EKG, abnormal |

(continued)

| Gene | Phenotype |
|---|---|
| KCNH2 | long QT syndrome |
| KCNJ11 | diabetes, type 2 |
| KCNJ11 | Type 2 Diabetes |
| KCNN3 | schizophrenia |
| KCNQ1 | EKG, abnormal |
| KCNQ1 | long QT syndrome |
| KIBRA | episodic memory |
| KLK1 | hypertension |
| KLK3 | prostate cancer |
| KRAS | colorectal cancer |
| LDLR | hypercholesterolemia |
| LDLR | hypertension |
| LEP | obesity |
| LEPR | obesity |
| LIG4 | breast cancer |
| LIPC | atherosclerosis, coronary |
| LPL | Coronary Artery Disease |
| LPL | hyperlipidemia |
| LPL | triglycerides |
| LRP1 | Alzheimer's Disease |
| LRP5 | bone density |
| LRRK2 | Parkinson's disease |
| LRRK2 | Parkinsons disease |
| LTA | type 1 diabetes |
| LTA | Asthma |
| LTA | systemic lupus erythematosus |
| LTA | sepsis |
| LTC4S | Asthma |
| MAOA | alcoholism |
| MAOA | schizophrenia |
| MAOA | bipolar disorder |
| MAOA | smoking behavior |
| MAOA | personality disorders |
| MAOB | Parkinson's disease |
| MAOB | smoking behavior |
| MAPT | Parkinson's disease |
| MAPT | Alzheimer's Disease |
| MAPT | dementia |

(continued)

| Gene | Phenotype |
|---|---|
| MAPT | Frontotemporal dementia |
| MAPT | progressive supranuclear palsy |
| MC1R | melanoma |
| MC3R | obesity |
| MC4R | obesity |
| MECP2 | Rett syndrome |
| MEFV | Familial Mediterranean Fever |
| MEFV | amyloidosis |
| MICA | Type 1 diabetes |
| MICA | Behcet's Disease |
| MICA | celiac disease |
| MICA | rheumatoid arthritis |
| MICA | systemic lupus erythematosus |
| MLH1 | colorectal cancer |
| MME | Alzheimer's Disease |
| MMP1 | Lung Cancer |
| MMP1 | ovarian cancer |
| MMP1 | periodontitis |
| MMP3 | myocardial infarct |
| MMP3 | ovarian cancer |
| MMP3 | rheumatoid arthritis |
| MPO | lung cancer |
| MPO | Alzheimer's Disease |
| MPO | breast cancer |
| MPZ | Charcot-Marie-Tooth disease |
| MS4A2 | asthma |
| MS4A2 | atopy |
| MSH2 | colorectal cancer |
| MSH6 | colorectal cancer |
| MSR1 | prostate cancer |
| MTHFR | colorectal cancer |
| MTHFR | Type 2 diabetes |
| MTHFR | neural tube defects |
| MTHFR | homocysteine |
| MTHFR | thromboembolism, venous |
| MTHFR | atherosclerosis, coronary |
| MTHFR | Alzheimer's Disease |
| MTHFR | esophageal cancer |

(continued)

| Gene | Phenotype |
|---|---|
| MTHFR | preeclampsia |
| MTHFR | pregnancy loss, recurrent |
| MTHFR | stroke |
| MTHFR | thrombosis, deep vein |
| MT-ND1 | diabetes, type 2 |
| MTR | colorectal cancer |
| MT-RNR1 | hearing loss, sensorineural nonsyndromic |
| MTRR | neural tube defects |
| MTRR | homocysteine |
| MT-TL1 | diabetes, type 2 |
| MUTYH | colorectal cancer |
| MYBPC3 | cardiomyopathy |
| MYH7 | cardiomyopathy |
| MYOC | glaucoma, primary open-angle |
| MYOC | glaucoma |
| NAT1 | colorectal cancer |
| NAT1 | Breast Cancer |
| NAT1 | bladder cancer |
| NAT2 | colorectal cancer |
| NAT2 | bladder cancer |
| NAT2 | breast cancer |
| NAT2 | Lung Cancer |
| NBN | breast cancer |
| NCOA3 | breast cancer |
| NCSTN | Alzheimer's Disease |
| NEUROD1 | Type 1 diabetes |
| NF1 | neurofibromatosis1 |
| NOS1 | Asthma |
| NOS2A | multiple sclerosis |
| NOS3 | hypertension |
| NOS3 | coronary heart disease |
| NOS3 | atherosclerosis, coronary |
| NOS3 | coronary artery disease |
| NOS3 | myocardial infarction |
| NOS3 | acute coronary syndrome |
| NOS3 | blood pressure, arterial |
| NOS3 | preeclampsia |
| NOS3 | nitric oxide |

(continued)

| Gene | Phenotype |
|---|---|
| NOS3 | Alzheimer’s Disease |
| NOS3 | asthma |
| NOS3 | Type 2 diabetes |
| NOS3 | cardiovascular disease |
| NOS3 | Behcet’s Disease |
| NOS3 | erectile dysfunction |
| NOS3 | kidney failure, chronic |
| NOS3 | lead toxicity |
| NOS3 | left ventricular hypertrophy |
| NOS3 | pregnancy loss, recurrent |
| NOS3 | retinopathy, diabetic |
| NOS3 | stroke |
| NOTCH4 | schizophrenia |
| NPY | alcohol abuse |
| NQO1 | lung cancer |
| NQO1 | colorectal cancer |
| NQO1 | benzene toxicity |
| NQO1 | bladder cancer |
| NQO1 | Parkinson’s Disease |
| NR3C2 | hypertension |
| NR4A2 | Parkinson’s disease |
| NRG1 | schizophrenia |
| NTF3 | schizophrenia |
| OGG1 | lung cancer |
| OGG1 | colorectal cancer |
| OLR1 | Alzheimer’s Disease |
| OPA1 | glaucoma |
| OPRM1 | alcohol abuse |
| OPRM1 | substance dependence |
| OPTN | glaucoma, primary open-angle |
| P450 | drug metabolism |
| PADI4 | rheumatoid arthritis |
| PAH | phenylketonuria/PKU |
| PAI1 | coronary heart disease |
| PAI1 | asthma |
| PALB2 | breast cancer |
| PARK2 | Parkinson’s disease |
| PARK7 | Parkinson’s disease |

(continued)

| Gene | Phenotype |
|---|---|
| PDCD1 | lupus erythematosus |
| PINK1 | Parkinson's disease |
| PKA | memory |
| PKC | memory |
| PLA2G4A | schizophrenia |
| PNOC | schizophrenia |
| POMC | obesity |
| PON1 | atherosclerosis, coronary |
| PON1 | Parkinson's disease |
| PON1 | Type 2 Diabetes |
| PON1 | atherosclerosis |
| PON1 | coronary artery disease |
| PON1 | coronary heart disease |
| PON1 | Alzheimer's Disease |
| PON1 | longevity |
| PON2 | atherosclerosis, coronary |
| PON2 | preterm delivery |
| PPARG | Type 2 Diabetes |
| PPARG | obesity |
| PPARG | diabetes, type 2 |
| PPARG | Colorectal Cancer |
| PPARG | hypertension |
| PPARGC1A | diabetes, type 2 |
| PRKCZ | Type 2 diabetes |
| PRL | systemic lupus erythematosus |
| PRNP | Alzheimer's Disease |
| PRNP | Creutzfeldt-Jakob disease |
| PRNP | Jakob-Creutzfeldt disease |
| PRODH | schizophrenia |
| PRSS1 | pancreatitis |
| PSEN1 | Alzheimer's Disease |
| PSEN2 | Alzheimer's Disease |
| PSMB8 | Type 1 diabetes |
| PSMB9 | Type 1 diabetes |
| PTCH | skin cancer, non-melanoma |
| PTGIS | hypertension |
| PTGS2 | colorectal cancer |
| PTH | bone density |

(continued)

| Gene | Phenotype |
|---|---|
| PTPN11 | Noonan syndrome |
| PTPN22 | rheumatoid arthritis |
| PTPRC | multiple sclerosis |
| PVT1 | end stage renal disease |
| RAD51 | breast cancer |
| RAGE | retinopathy, diabetic |
| RB1 | retinoblastoma |
| RELN | schizophrenia |
| REN | hypertension |
| RET | thyroid cancer |
| RET | Hirschsprung's disease |
| RFC1 | neural tube defects |
| RGS4 | schizophrenia |
| RHO | retinitis pigmentosa |
| RNASEL | prostate cancer |
| RYR1 | malignant hyperthermia |
| SAA1 | amyloidosis |
| SCG2 | hypertension |
| SCG3 | obesity |
| SCGB1A1 | asthma |
| SCN5A | Brugada syndrome |
| SCN5A | EKG, abnormal |
| SCN5A | long QT syndrome |
| SCNN1B | hypertension |
| SCNN1G | hypertension |
| SERPINA1 | COPD |
| SERPINA3 | Alzheimer's Disease |
| SERPINA3 | COPD |
| SERPINA3 | Parkinson's disease |
| SERPINE1 | myocardial infarct |
| SERPINE1 | Type 2 Diabetes |
| SERPINE1 | atherosclerosis, coronary |
| SERPINE1 | obesity |
| SERPINE1 | preeclampsia |
| SERPINE1 | stroke |
| SERPINE1 | hypertension |
| SERPINE1 | pregnancy loss, recurrent |
| SERPINE1 | thromboembolism, venous |

(continued)

| Gene | Phenotype |
|---|---|
| SLC11A1 | tuberculosis |
| SLC22A4 | Crohn's disease; ulcerative colitis |
| SLC22A5 | Crohn's disease; ulcerative colitis |
| SLC2A1 | Type 2 diabetes |
| SLC2A2 | Type 2 diabetes |
| SLC2A4 | Type 2 diabetes |
| SLC3A1 | cystinuria |
| SLC6A3 | attention deficit hyperactivity disorder |
| SLC6A3 | Parkinson's disease |
| SLC6A3 | smoking behavior |
| SLC6A3 | alcoholism |
| SLC6A3 | schizophrenia |
| SLC6A4 | depression |
| SLC6A4 | depressive disorder, major |
| SLC6A4 | schizophrenia |
| SLC6A4 | suicide |
| SLC6A4 | alcoholism |
| SLC6A4 | bipolar disorder |
| SLC6A4 | personality traits |
| SLC6A4 | attention deficit hyperactivity disorder |
| SLC6A4 | Alzheimer's Disease |
| SLC6A4 | personality disorders |
| SLC6A4 | panic disorder |
| SLC6A4 | alcohol abuse |
| SLC6A4 | affective disorder |
| SLC6A4 | anxiety disorder |
| SLC6A4 | smoking behavior |
| SLC6A4 | depressive disorder, major; bipolar disorder |
| SLC6A4 | heroin abuse |
| SLC6A4 | irritable bowel syndrome |
| SLC6A4 | migraine |
| SLC6A4 | obsessive compulsive disorder |
| SLC6A4 | suicidal behavior |
| SLC7A9 | cystinuria |
| SNAP25 | ADHD |
| SNCA | Parkinson's disease |
| SOD1 | ALS/amyotrophic lateral sclerosis |
| SOD2 | breast cancer |

(continued)

| Gene | Phenotype |
|---|---|
| SOD2 | lung cancer |
| SOD2 | prostate cancer |
| SPINK1 | pancreatitis |
| SPP1 | multiple sclerosis |
| SRD5A2 | prostate cancer |
| STAT6 | asthma |
| STAT6 | Total IgE |
| SULT1A1 | breast cancer |
| SULT1A1 | colorectal cancer |
| TAP1 | Type 1 diabetes |
| TAP1 | lupus erythematosus |
| TAP2 | Type 1 diabetes |
| TAP2 | diabetes, type 1 |
| TBX21 | asthma |
| TBXA2R | asthma |
| TCF1 | diabetes, type 2 |
| TCF1 | Type 2 diabetes |
| TF | Alzheimer’s Disease |
| TGFB1 | breast cancer |
| TGFB1 | kidney transplant |
| TGFB1 | kidney transplant complications |
| TH | schizophrenia |
| THBD | myocardial infarction |
| TLR4 | asthma |
| TLR4 | Crohn’s disease; ulcerative colitis |
| TLR4 | sepsis |
| TNF | asthma |
| TNFA | cerebrovascular disease |
| TNF | Type 1 diabetes |
| TNF | rheumatoid arthritis |
| TNF | systemic lupus erythematosus |
| TNF | kidney transplant |
| TNF | psoriasis |
| TNF | sepsis |
| TNF | Type 2 Diabetes |
| TNF | Alzheimer’s Disease |
| TNF | Crohn’s disease |
| TNF | diabetes, type 1 |

(continued)

| Gene | Phenotype |
| --- | --- |
| TNF | hepatitis B |
| TNF | kidney transplant complications |
| TNF | multiple sclerosis |
| TNF | schizophrenia |
| TNF | celiac disease |
| TNF | obesity |
| TNF | pregnancy loss, recurrent |
| TNFRSF11B | bone density |
| TNFRSF1A | rheumatoid arthritis |
| TNFRSF1B | Rheumatoid Arthritis |
| TNFRSF1B | systemic lupus erythematosus |
| TNFRSF1B | arthritis |
| TNNT2 | cardiomyopathy |
| TP53 | lung cancer |
| TP53 | breast cancer |
| TP53 | Colorectal Cancer |
| TP53 | prostate cancer |
| TP53 | cervical cancer |
| TP53 | ovarian cancer |
| TP53 | smoking |
| TP53 | esophagealcancer |
| TP73 | lung cancer |
| TPH1 | suicide |
| TPH1 | depressive disorder, major |
| TPH1 | suicidal behavior |
| TPH1 | schizophrenia |
| TPMT | thiopurine methyltransferase activity |
| TPMT | leukemia |
| TPMT | inflammatory bowel disease |
| TPMT | thiopurine S-methyltransferase phenotype |
| TSC1 | tuberous sclerosis |
| TSC2 | tuberous sclerosis |
| TSHR | Graves' disease |
| TYMS | colorectal cancer |
| TYMS | stomach cancer |
| TYMS | esophageal cancer |
| UCHL1 | Parkinson's disease |
| UCP1 | obesity |

(continued)

| Gene | Phenotype |
|---|---|
| UCP2 | obesity |
| UCP3 | obesity |
| UGT1A1 | hyperbilirubinemia |
| UGT1A1 | Gilbert syndrome |
| UGT1A6 | colorectal cancer |
| UGT1A7 | colorectal cancer |
| UTS2 | diabetes, type 2 |
| VDR | bone density |
| VDR | prostate cancer |
| VDR | bone mineral density |
| VDR | Type 1 diabetes |
| VDR | osteoporosis |
| VDR | bone mass |
| VDR | breast cancer |
| VDR | lead toxicity |
| VDR | tuberculosis |
| VDR | Type 2 diabetes |
| VEGF | breast cancer |
| vit D rec | idiopathic short stature |
| VKORC1 | warfarin therapy, response to |
| WNK4 | hypertension |
| XPA | lung cancer |
| XPC | lung cancer |
| XPC | cytogenetic studies |
| XRCC1 | lung cancer |
| XRCC1 | cytogenetic studies |
| XRCC1 | breast cancer |
| XRCC1 | bladder cancer |
| XRCC2 | breast cancer |
| XRCC3 | breast cancer |
| XRCC3 | cytogenetic studies |
| XRCC3 | lung cancer |
| XRCC3 | bladder cancer |
| ZDHHC8 | schizophrenia |

Incorporating Ancestral Data

The present disclosure also provides methods and systems, such as described herein, that correlated phenotypes using genomic profiles by incorporating ancestral data. Thus, assessing an individual's genotype correlation may be expressed or reported as a GCI score, and incorporate ancestral data in generating the GCI score. For example, OR used in determining GCI scores may be modified based on an individual's ancestry or ethnicity.

The risk of an individual to develop a certain condition is typically based on the individual's genetics and environment. When trying to estimate the risk based on genetics, current studies can be limited by the fact that only a subset of all genetics markers or variations, such as SNPs, can be measured. Particularly, for complex diseases, the complex interaction of many genetic and environmental factors can lead to the development of a condition, and therefore there can be many genetic variations, such as SNPs, that marginally contribute to the risk. Current Whole-Genome-Association (WGA) studies normally consider each region in the genome in isolation, and try to answer the question as to what is the effect of a mutation in a specific SNP in that region on the risk for the condition, when keeping all other genetic factors and environmental factors as unknown. Mathematically, these studies essentially estimate the marginal distribution of the risk probability as a function of a SNP (these distributions as referred herein as the effect of a SNP).

The risk for developing the condition can be affected not by one genetic variation or SNP, but by many SNPs or other genetic variations, and environmental factors. Therefore, if two populations differ in their allelic distribution across the genome, and in the environmental factors affecting them, there may be a potential difference in the effect of a specific genetic variation, such as a SNP, in each of the populations. This is particularly the case when there is a gene-gene or gene-environment interaction between this SNP and another SNP, other genetic variants, or environmental factor. However, even in cases where there is no interaction, a different 'background distribution' of the other genetic and environmental factors can affect the effect of a genetic variation, such as a SNP. Thus, without being bound by theory, different populations can have different effect sizes for the same genetic variant, such as a SNP. In practice, however, almost all known conditions in which there is a SNP whose effect size was measured in more than one population, the effects measured were either very close to each other, or at least within the 95% CI of each other. As a result, in some embodiments, a simplifying assumption that can be used herein, is the effect size of a genetic variation, such as a causal SNP, is in fact the same across all populations.

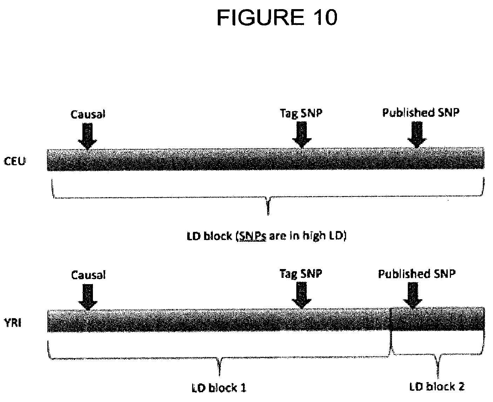

Unfortunately, even with the assumption that the effect size is the same across populations, a limitation is the fact that the causal genetic variation may be unknown, for example, the causal SNP can not or has not been genotyped. Fortunately, SNPs or other genetic variations in close proximity on the genome can be correlated, such as in LD, and therefore even if the causal SNP is not measured, a tag SNP can be used as a proxy to the causal (see **FIG. 10** for example). However, different populations can have different linkage disequilibrium patterns due to various possible reasons such as variation in recombination rates, selection pressure, or population bottleneck. Thus, in some embodiments, if a study has been done on population A, yielding a specific odds ratio in that population, the same odds ratio cannot be assumed in population B. This can be illustrated by the following example (see **FIG. 10**). For example, study has been performed on a Caucasian (CEU) population, and a large effect size has been reported for one of the SNP (the 'published SNP'). In the example, the published SNP belongs to an LD block which is shared with the causal SNP, so the r^2 (the square of the correlation coefficient) between the causal and the published SNP is 1; put differently, the published SNP and the causal SNPs are perfectly correlated in the CEU population. However, it may be the case that in another population (in this case, YRI, Yoruban), the published SNP and the causal SNP are in different LD blocks. In the extreme case, they can have r^2 = 0, in which case they are independent of each other in that population. Under such a scenario, if the same study had been done on the YRI population, no effect would have been detected for the published SNP. It would therefore be wrong to estimate the risk of an YRI individual by ignoring the LD patterns in the underlying population of the customer and of the originally studied population. The example in **FIG. 10** is an extreme case, but in reality similar patterns can happen with less extreme consequences.

Thus, in some embodiments, the present disclosure provides a method of assessing genotype correlations of an individual comprising comparing loci between populations of different ancestry. For example, odds ratios taken for a first population may be applied, or varied, to a second population, depending on such factors as LD patters. For example, for AS (Asians), the odds ratios used may be that of studies of AS, YRI (Yoruban), CEU (Caucasian/European) ancestry/ethnicity, in this order, since YRI has a lower LD than CEU. In some embodiments, locus-specific ancestry may be used for admixed populations.

In some embodiments, the populations of the first and second populations could comprise, but not be limited to any other population such as African American, Caucasian, Ashkenazi Jewish, Sepharadic Jewish, Indian, Pacific islanders, middle eastern, Druze, Bedouins, south Europeans, Scandinavians, eastern Europeans, North Africans, Basques, West Africans, East Africans. Otherwise stated, the populations of the first and second populations could comprise, but not limited to any of the HapMap populations (YRI,CEU,CHB,JPT, ASW, CHD, GIH, LWK, MEX, MKK,TSI). The description of the HapMap populations can be found in http://hapmap.org/hapmappopulations.html.en and in enclosed document.

| Label | Population Sample | Number of Samples |
|---|---|---|
| ASW | African ancestry in Southwest USA | 90 |

(continued)

| Label | Population Sample | Number of Samples |
|---|---|---|
| CEU | Utah residents with Northern and Western European ancestry from the CEPH collection | 180 |
| CHB | Han Chinese in Beijing, China | 90 |
| CHD | Chinese in Metropolitan Denver, Colorado | 100 |
| GIH | Gujarati Indians in Houston, Texas | 100 |
| JPT | Japanese in Tokyo, Japan | 91 |
| LWK | Luhya in Webuye, Kenya | 100 |
| MEX | Mexican ancestry in Los Angeles, California | 90 |
| MKK | Maasai in Kinyawa, Kenya | 180 |
| TSI | Toscans in Italy | 100 |
| YRI | Yoruba in Ibadan, Nigeria | 180 |

Methods for assessing an individual's genotype correlations to a phenotype comprise comparing a first linkage disequilibrium (LD) pattern comprising a genetic variation, such as a SNP, correlated with a phenotype, wherein the first LD pattern is of a first population of individuals; and, a second LD pattern comprising the genetic variation (such as the SNP), wherein the second LD pattern is of a second population of individuals; determining a probability of the genetic variation being correlated with the phenotype in the second population from the comparing; and assessing a genotype correlation of the phenotype from a genomic profile of the individual comprising using the probability; and, reporting results comprising said genotype correlation from to said individual or a health care manager of said individual.

For example, assuming that a published SNP P has been reported for a first population, A, with odds ratios OR[P,A], and that the causal SNP C is unknown. Also in this example, is the assumption that for a second population, B, the odds ratios of C in A and B are the same, that is OR[C,A] = OR[C,B], if the sample size is large enough. Thus, if the location of C is known, and OR[C,A], the LD patterns in B can be used to estimate the best tag SNP to capture C in the population. However, in some embodiments, the location of C is unknown as is the odds ratio of C. However, for every SNP S and value X, the probability can be computed: Prob[S=C, OR[C,A]=X] | r^2(P,S) in A, P, OR[P,A]], i.e., the probability that S is the causal SNP, with odds ratio of X (assuming an infinite sample size), given the correlation coefficient between S and P in population A, and given that P is the published SNP with odds ratio OR[P,A]. In order to calculate this probability, the fact that in the actual study the odds ratios of S is lower than the odds ratio of C is used and the question of what is the probability for this to happen given that OR[S,A] should approach X for a large enough sample size can be answered. Given the distribution of causal SNPs and their effect sizes, the expected effect size of a tag SNP can be determined by computing the expectation (the weighted average) of the effect sizes resulting from the different SNPs being causal.

In the example given in **FIG. 10,** since the LD block is of a perfect LD, all SNPs in the CEU block have the same probability of being causal with the same distribution of effect size (i.e., the log odds ratio is Normally distributed, where the confidence intervals determine its standard deviation). However, when the published SNP in YRI that aims at tagging the causal, the expected odds ratio of this SNP will be the weighted average between the published odds ratio and 1, where the weight corresponds to the length of the LD blocks involved.

Thus, modifications of ORs are determined by methods including, but not limited to, determining a causal genetic variation probability, such as an OR, for each of a plurality of genetic variations in a first population of individuals, or reference population, such as CEU as described in the above example. The OR may be then be used in assessing a genotype correlation from a genomic profile of an individual of a another population of individuals or reference group, such as YRI, reporting results comprising said genotype correlation from step (c) to said individual or a health care manager of said individual. Thus, the each of the genetic variations used in calculating their probability of being the causal genetic variant (such as a causal SNP), is typically proximal to a known genetic variation correlated to a phenotype in the first population, such as the published genetic variation, such as a published SNP. In some embodiments, each of each of the genetic variations used in calculating their probability of being the causal genetic variant (such as a causal SNP), is in linkage disequilibrium to the known or published genetic variation.

For example, again assuming that a published SNP P has been reported for a first population, A, with odds ratios OR[P,A], and that the causal SNP C is unknown, and that for a second population, B, the odds ratios of C in A and B are the same, that is OR[C,A] = OR[C,B], if the sample size is large enough. Another assumption in the example, the LD patterns are known for the studied population and for an individual's population. For example, it is assumed that the study has been done on the CEU population (first population), and that the individual is of the YRI population (second population), although the example can be extended to other populations. In every position, it is assumed that there is a risk allele R, and a non-risk allele N. The three possible genotypes in a given SNP are RR, RN, and NN. For a given SNP S, a genotype G (which is either RR, RN, or NN), and a group of individuals I, is denoted by F(S,I,G) the number of individuals in I with genotype G at SNP S. Thus, the odds ratios measured on the CEU population in the published SNP P is given by $OR(P,CEU,G)=\frac{F(P,CA,G)F(P,CT,NN)}{F(P,CA,NN)F(P,CT,G)}$, where CA and CT represent the case and control populations. Similarly, it is denoted by f(S,I,G) the frequency of the genotype G in population I. For a pair of SNPs $S_1$ and $S_2$, it is denoted by $P_{CEU}(S_1,G_1 \mid S_2, G_2)$ the probability that an individual has genotype $G_1$ at SNP $S_1$, given that the individual has $G_2$ at $S_2$ (in CEU). A similar notation is used for YRI, the second population.

An algorithm is used to determine an OR for the second population, and thus use in assessing an individual's genotype correlation to a phenotype, such as through the use of a GCI score. For example, the input and output of the algorithm disclosed herein may have the following information provided:

1) A list of SNPs (such as those disclosed in the HapMap), and another special SNP P, which is the published SNP.
2) The list of SNPs from the above SNPs that are measured in the study.
3) For the published SNP P, one of the following is assumed to be known:

   (a) The genotype counts from the study for the cases and the controls, that is, the values of F(P,CA,G) and F(P,CT,G) are known for every genotype G.
   (b) Alternatively, it is assumed that the genotypic odds ratios are known at SNP P, their confidence intervals, and the total number of cases and controls.

4) For every pair of SNPs $S_1$,$S_2$, and every pair of genotypes $G_1$,$G_2$, the algorithm will be provided with $P_{CEU}(S_1,G_1 \mid S_2, G_2)$ and with $P_{YRI}(S_1,G_1 \mid S_2, G_2)$ - this information can be found from the HapMap or other reference dataset.

The algorithm can then output for every SNP S in the proximity of P, the expected odds ratio of the SNP under the assumption that the number of individuals in the study is very large (approaching infinity). The algorithm will make the assumption that the odds ratios of the causal C for CEU (i.e. first population) and YRI (i.e. second population) approach the same number when the sample size approaches infinity.

Thus, the algorithm disclosed herein can include the following major steps (see also Example 5):

1) Find the LD probabilities based on the reference dataset.
2) Find the counts F(P,CA,G), F(P,CT,G) if they are not given as an input (alternative 1-b).
3) Sample n (n very large, e.g. >> 1,000,000) instances for the genotype frequencies of the cases and controls at P, in CEU. The sampling is based on the posterior distribution of f(P,CA,G), f(P,CT,G), given the counts.
4) For each instance of the frequencies, and for each SNP S:

   (a) Calculate f(S,CA,G) and f(S,CT,G) based on the frequencies in P and on $P_{CEU}$.
   (b) Generate an instance of F(S,CA,G), F(S,CT,G) based on the sampled allele frequencies in S.
   (c) Calculate the p-value for S based on F(S), and based on f(S) (the latter is the p-value in the asymptotic sense).
   (d) Find the min p-value based on F(S) across all measured SNPs S. If this is not P, then this instance is rejected.
   (e) If the instance is not rejected, the instance is kept, together with the min p-value based on f(S); this will be the causal SNP of that instance.

5) The previous phase results in a set of causal SNPs$C_1$,...,$C_n$, and their corresponding odds ratios. For each such causal, it is assumed that the same odds ratio holds for the YRI population.

   (a) This information, together with the genotype frequencies in YRI at $C_i$ is used to estimate the frequencies $f_{YRI}(C_i,CA,G)$ for every genotype G.
   (b) The LD information is used to estimate $f_{YRI}(S,CA,G)$,$f_{YRI}(S,CT,G)$ for every SNP S, and calculate the asymptotic odds ratios based on these frequencies.
   (c) For each SNP S, the asymptotic odds ratios are averaged across all instances, resulting in the expected asymptotic odds ratios.

In some embodiments, ancestral data may be used to assess an individual for their sub-group, for example, the present disclosure provides a method of assessing a reference sub-group of an individual comprising: obtaining a genetic sample of the individual; generating a genomic profile for the individual; determining the individual's one or more reference sub-groups by comparing the individual's genomic profile to a current database of human genotype correlations with ethnicity, geographic origin, or ancestry; and, reporting the results from step c) to the individual or a health care manager of the individual.

In one aspect a reference data set comprising multiple sets of genotyping data from individuals, wherein substantially the entire genome is used in the present disclosure. In one embodiment, the reference data contains genotyping data from substantially the entire genome of multiple individuals. Wherein in one embodiment, substantially the entire genome means that genetic markers are detected that cover at least 80% of an individuals genome, including but not limited to at least 81%, 82%, 83%, 84%, 85%, 86%, 87%, 88%, 89%, 90%, 91 %, 92%, 93%, 94%, 95%, 96%, 97%, 98%, 99% or 100% genomic coverage. In another embodiment at least 75% of the sets of genotyping data from the individuals included in the reference data include information from genetic markers that cover at least 80% of each individual's genome. In a further embodiment greater than 75% (including but not limited to greater than 76%, 77%, 78%, 79%, 80%, 81%, 82% 83%, 84%, 85%, 86%, 87%, 88%, 89%, 90%, 91%, 92%, 93%, 94%, 95%, 96%, 97%, 98%, 99% or 100%) of the sets of genotyping data from the individuals included in the reference data include information from genetic markers that cover at least 80% of each individual's genome.

In one embodiment, the reference data set includes information on multiple genetic markers including but not limited to nucleotide repeats, nucleotide insertions, nucleotide deletions, chromosomal translocations, chromosomal duplications, copy number variations, microsatellite repeats, nucleotide repeats, centromeric repeats, or telomeric repeats or SNPs. In another embodiment the reference data set includes information which is substantially limited to a single genetic marker, such as SNPS or microsatellites. Wherein at least 80% of the genetic markers included in a reference set are of the same type, including but not limited to at least 81%, 82%, 83%, 84%, 85%, 86%, 87%, 88%, 89%, 90%, 91%, 92%, 93%, 94%, 95%, 96%, 97%, 98%, 99% or 100% of the genetic markers.

In another embodiment, the reference data set consists essentially of whole genome SNP genotyping data. In some embodiments the SNP data is derived from analyses of indviduals' genomes using a high density DNA array for SNP identification and profile generation. Such arrays include but are not limited to those commercially available from Affymetrix and Illumina (see Affymetrix GeneChip® 500K Assay Manual, Affymetrix, Santa Clara, CA; Sentrix® humanHap650Y genotyping beadchip, Illumina, San Diego, CA). In some embodiments a reference set consists essentially of SNP data generated by genotyping more than 900,000 SNPs using the Affymetrix Genome Wide Human SNP Array 6.0. In alternative embodiment, more than 500,000 SNPs through whole-genome sampling analysis may be determined by using the Affymetrix GeneChip Human Mapping 500K Array Set.

In another embodiment, the reference data set contains information about the ethnicity, geographic origin and/or ancestry of each individual whose genotype data is included. In one embodiment said information is present in a reference data set, such as the HapMap or the Genographic Project (https://www3.nationalgeographic.com/genographic/). In another embodiment said information is self-reported, such as by subscribers or non-subscribers. In another embodiment subscribers may receive an incentive to self-report information about their ethnicity, geographic origin and/or ancestry. In another embodiment, subscribers may receive an incentive to self-report information about their disease status (such as information about any diseases or conditions they may display symptoms of or have a hereditary pre-disposition for). In another embodiment the individual receives an incentive to allow the use of this information and the individual's genotype in at least one reference data set. In some embodiments the incentive may be a financial incentive a discount on services offered, an offer of free services, an offer of a service upgrade (such as an increase in subscriber status, from basic to a premium membership category), an offer of free or discounted services for a relative, or an offer of discounted, free or credited services with a 3rd party vendor (such as Amazon, Starbucks, WebMD). In a related embodiment subscribers or non-subscribers who disclose information related to their ethnicity, geographic origin and/or ancestry, or disease status may be advised about the possible uses of such disclosed information and given the opportunity to supply or to withhold their informed consent.

In one embodiment, the reference data set contains information from multiple individuals with different ethnicities, geographic origins and/or ancestries. In another embodiment, the reference data set contains more than one individual from each class of ethnicity, geographic origin and/or ancestry represented in said reference data set. In another embodiment the reference data set contains more than five individual from each class of ethnicity, geographic origin and/or ancestry represented in said reference data set. In another embodiment the reference data set contains more than ten individuals from each class of ethnicity, geographic origin and/or ancestry represented in said reference data set. In another embodiment the reference data set contains more than twenty individuals from each class of ethnicity, geographic origin and/or ancestry represented in said reference data set.

In another embodiment, the assembled data in a reference set is analyzed to correlate ethnicity, geographic origin and/or ancestry, with at least one disease or condition and genetic marker associations. In another embodiment self-reported ethnicity, geographic origin and/or ancestry may be used to flag specific diseases or conditions for risk analysis. In another embodiment, an individual's ethnicity, geographic origin and/or ancestry is correlated with their genotype for further analysis (such as *in silico* population genetics studies) of associations between genetic markers and a disease or condition within a sub-grouping of individuals with a similar or shared ethnicity, geographic origin and/or ancestry. For example, it is known certain groups of individuals with a shared ethnicity, geographic origin and/or ancestry, such as the Ashkenazi Jews have a much higher likelihood of having children with diseases such as Tay Sachs. The analysis of an individual who self identifies as an Ashkenazi Jew could be modified to take this information into account when analyzing the individual's genetic markers.

In another embodiment, the data in reference data set can stratified into reference data sub-groups. A population, when considered as a whole, may contain multiple sub-groups, which may have different allele frequencies. The presence of multiple subgroups with different allele frequencies within a population can make association studies less informative. The different underlying allele frequencies in sampled subgroups may be independent of a disease or condition within each group, and they can lead to erroneous conclusions of linkage disequilibrium or disease relevance. Comparison of an individual's genotype to a reference data sub-group rather than to the entire reference data set can reduce the likelihood of errors created by spurious allelic associations. The data in each reference data sub-group may be organized by at least one shared feature, such as shared ethnicity, geographic origin and/or ancestry. The genotypes of individuals whose data is comprised within each sub-group can be further analyzed to identify common genetic markers that are of indicative of a specific ethnicity, geographic origin and/or ancestry. In an alternative embodiment assembled data in a reference set can be used to genetic markers which are associated with at least one disease or condition, and that are also associated with at least one ethnicity, geographic origin and/or ancestry.

In one embodiment, an individual's at least one self reported ethnic, geographic origin and/or ancestral trait is used to modify the analysis of the individual's genotype. A modified analysis may focus on genetic markers that are associated with a disease or condition, which are also common to at least one self identified ethnic, geographic origin and/or ancestral sub-group. In an alternative embodiment information related to an individual's ethnicity, geographic origin and/or ancestry is determined based on the individual's genotype. For example, an individual's genotype is compared to at least one reference data set and used to determine information about the individual's ethnicity, geographic origin and/or ancestry. This information is then incorporated into the analysis of the individual's genotype for association with at least one disease or condition. The analysis may focus on genetic markers associated with at least one disease or condition, which may also be common to at least one ethnicity, geographic origin and/or ancestry.

In another embodiment both information about an individual's ethnicity, geographic origin and/or ancestry, and information derived from analysis of the individual's genetic markers is used to determine the likelihood that the individual shares a specific ethnicity, geographic origin and/or ancestry. By combining both types of information the information obtained from the genotype analysis can be used to verify the individual's self-reported ethnicity, geographic origin and/or ancestry and to correct for any inaccuracies. In one embodiment information about an individual's ethnicity, geographic origin and/or ancestry is self-reported. In an alternative embodiment information about an individual's ethnicity, geographic origin and/or ancestry is estimated. Estimating an individual's ethnicity, geographic origin and/or ancestry can provide a continuous measure to assess population structure in the study of complex diseases or conditions. There can be a fair amount of heterogeneity in ethnic, geographic origin and/or ancestral groupings based on individuals' self reported information. For example, individual ethnic, geographic origin and/or ancestral proportions (such as European, North African, Aboriginal, etc.) can be estimated based on published allele frequencies. The estimated example individual ethnic, geographic origin and/or ancestral proportion can be used as a surrogate for self-reported information to investigate an association between at least one genetic marker and at least one disease or condition. Genetic risk models can then be used to determine if adjusting for an estimated individual ethnic, geographic origin and/or ancestral proportion provides a better fit to the data compared to a model with no adjustment for ethnicity, geographic origin and/or ancestry or one based on self-reported information. The model that provides the best fit can then be used to determine an individual's risk of acquiring at least one disease or condition.

In another embodiment, ethnicity, geographic origin and/or ancestry information from an individual that is based on genotype and/or self-reported data may be used to mathematically determine the closest reference sub-group or sub-groups to the individual, in terms of contribution to the individual's global genome. For example, if it may be determined that an individual's genotype suggests that he/she shares genetic markers indicative of more than one ethnicity, geographic origin and/or ancestry. This determination may include likelihoods, and optionally confidence intervals (such as there is an X%±Y), that at least one of an individual's relatives was from a specific ethnic, geographic and/or ancestral origin. This determination than can be used to inform an individual of the genetic markers typically associated with at least one disease or condition in individual's who share a similar ethnic, geographic and/or ancestral origin and their risk of acquiring said at least one disease or condition. In another embodiment a report may be generated which includes information on the contribution to an individual's entire genome from various ethnic sources, geographic origins and/or ancestral sources. For example a report may describe aggregate ancestral origins over an individual's entire genome in percentages, such as 20% from Africa, 30% from Asia, 50% from Europe. In a further embodiment such a report may optionally include confidence intervals (such as 20%±3 from Africa, 30%±5 from Asia, 50%±2 from Europe).

In another embodiment, an individual's determined ethnicity, geographic origin and/or ancestry may be used to determine an individual's risk of acquiring at least one disease or condition based on analysis of specific loci. In a related embodiment, a report may generated for at least one locus that characterizes the likelihood that an individual inherited said locus from a relative with a specific ethnicity, geographic origin and/or ancestry and the association of an allele at said locus with at least one disease or condition. In another embodiment at least two locus specific association results may be aggregated to determine an individual's combined risk of acquiring at least one disease or condition.

The risk of acquiring at least one disease or condition may be determined for an individual who has an ethnicity, geographic origin and/or ancestry that differs from those of individuals previously reported in association studies. In another embodiment the risk of acquiring at least one disease or condition may be determined for an individual who has a unique or rare ethnicity, geographic origin and/or ancestry that makes it difficult or impossible to find a reference data sub-group to compare the individual's genotype to. For example an individual may want to know his/her risk of acquiring an inherited disease which may directly related to his ethnicity, geographic origin and/or ancestry. Some exceedingly rare diseases, such as oculopharyngeal muscular dystrophy, are found only within small localized groups in a population. Often diseases of this nature can be traced back to a single founder or to a limited number of past disease carriers. For diseases of this nature it is often possible to exclude an individual from an at-risk group if it can be determined that the individual is not related to the original founder or disease carriers. It may beneficial to conduct one or more association studies of other individuals with a shared genetic background or shared ethnicity, geographic origin and/or ancestry. Wherein the individuals' ethnicity, geographic origin and/or ancestry is determined by estimation or by self-reported information. These studies can combine information on individual's genotype, ethnicity, geographic origin and/or ancestry, and status of at least one disease or condition. Results obtained from at least two studies can be compared to determine if a similar association between an allele of a genetic marker and at least one disease or condition is observed. Results may depend on the correlation structure and allele frequencies in each of the populations studied and the relationship between them. Further, said studies can be used to identify genetic markers that are associated with susceptibility to said at least one disease or condition. In one embodiment the absence of at least one allele for at least one genetic marker is used to exclude an individual from being at risk for at least one disease or condition. In an alternative embodiment the presence of at least one allele for at least one genetic marker is used to categorize an individual as being at risk for at least one disease or condition.

The following examples illustrate and explain the disclosure. The scope of the disclosure is not limited by these examples.

**EXAMPLES**

Example 1: Generation and Analysis of SNP Profile

The individual is provided a sample tube in the kit, such as that available from DNA Genotek, into which the individual deposits a sample of saliva (approximately 4 mls) from which genomic DNA will be extracted. The saliva sample is sent to a CLIA certified laboratory for processing and analysis. The sample is typically sent to the facility by overnight mail in a shipping container that is conveniently provided to the individual in the collection kit.

In a preferred embodiment, genomic DNA is isolated from saliva. For example, using DNA self collection kit technology available from DNA Genotek, an individual collects a specimen of about 4 ml saliva for clinical processing. After delivery of the sample to an appropriate laboratory for processing, DNA is isolated by heat denaturing and protease digesting the sample, typically using reagents supplied by the collection kit supplier at 50°C for at least one hour. The sample is next centrifuged, and the supernatant is ethanol precipitated. The DNA pellet is suspended in a buffer appropriate for subsequent analysis.

The individual's genomic DNA is isolated from the saliva sample, according to well known procedures and/or those provided by the manufacturer of a collection kit. Generally, the sample is first heat denatured and protease digested. Next, the sample is centrifuged, and the supernatant is retained. The supernatant is then ethanol precipitated to yield a pellet containing approximately 5-16 ug of genomic DNA. The DNA pellet is suspended in 10 mM Tris pH 7.6, 1 mM EDTA (TE). A SNP profile is generated by hybridizing the genomic DNA to a commercially available high density SNP array, such as those available from Affymetrix or Illumina, using instrumentation and instructions provided by the array manufacturer. The individual's SNP profile is deposited into a secure database or vault.

The patient's data structure is queried for risk-imparting SNPs by comparison to a clinically-derived database of established, medically relevant SNPs whose presence in a genome correlates to a given disease or condition. The database contains information of the statistical correlation of particular SNPs and SNP haplotypes to particular diseases or conditions. For example, as shown in Example III, polymorphisms in the apolipoprotein E gene give rise to differing isoforms of the protein, which in turn correlate with a statistical likelihood of developing Alzheimer's Disease. As another example, individuals possessing a variant of the blood clotting protein Factor V known as Factor V Leiden have an increased tendency to clot. A number of genes in which SNPs have been associated to a disease or condition phenotype are shown in Table 1. The information in the database is approved by a research/clinical advisory board for its scientific accuracy and importance, and may be reviewed with governmental agency oversight. The database is continually updated as more SNP-disease correlations emerge from the scientific community.

The results of the analysis of an individual's SNP profile is securely provided to patient by an on-line portal or mailings. The patient is provided interpretation and supportive information, such as the information shown for Factor V Leiden in Example IV. Secure access to the individual's SNP profile information, such as through an on-line portal, will facilitate discussions with the patient's physician and empower individual choices for personalized medicine.

Example 2: Update of Genotype Correlations

In response to a request for an initial determination of an individual's genotype correlations, a genomic profile is generated, genotype correlations are made, and the results are provided to the individual as described in Example I. Following an initial determination of an individual's genotype correlations, subsequent, updated correlations are or can be determined as additional genotype correlations become known. The subscriber has a premium level subscription and their genotype profile and is maintained in a secure database. The updated correlations are performed on the stored genotype profile.

For example, an initial genotype correlation, such as described above in Example I, could have determined that a particular individual does not have ApoE4 and thus is not predisposed to early-onset Alzheimer's Disease, and that this individual does not have Factor V Leiden. Subsequent to this initial determination, a new correlation could become known and validated, such that polymorphisms in a given gene, hypothetically gene XYZ, are correlated to a given condition, hypothetically condition 321. This new genotype correlation is added to the master database of human genotype correlations. An update is then provided to the particular individual by first retrieving the relevant gene XYZ data from the particular individual's genomic profile stored in a secure database. The particular individual's relevant gene XYZ data is compared to the updated master database information for gene XYZ. The particular individual's susceptibility or genetic predisposition to condition 321 is determined from this comparison. The results of this determination are added to the particular individual's genotype correlations. The updated results of whether or not the particular individual is susceptible or genetically predisposed to condition 321 is provided to the particular individual, along with interpretative and supportive information.

Example 3: Correlation of ApoE4 Locus and Alzheimer's Disease

The risk of Alzheimer's disease (AD) has been shown to correlate with polymorphisms in the apolipoprotein E (APOE) gene, which gives rise to three isoforms of APOE referred to as ApoE2, ApoE3, and ApoE4. The isoforms vary from one another by one or two amino acids at residues 112 and 158 in the APOE protein. ApoE2 contains 112/158 cys/cys; ApoE3 contains 112/158 cys/arg; and ApoE4 contains 112/158 arg/arg. As shown in Table 2, the risk of Alzeimer's disease onset at an earlier age increases with the number of APOE ε4 gene copies. Likewise, as shown in Table 3, the relative risk of AD increases with number of APOE ε4 gene copies.

Table 2: Prevalence of AD Risk Alleles (Corder et al., *Science:* 261:921-3, 1993)

| APOE ε4 Copies | Prevalence | Alzheimer's Risk | Onset Age |
|---|---|---|---|
| 0 | 73% | 20% | 84 |
| 1 | 24% | 47% | 75 |
| 2 | 3% | 91% | 68 |

Table 3: Relative Risk of AD with ApoE4 (Farrer et al., *JAMA:* 278:1349-56, 1997)

| APOE Genotype | Odds Ratio |
|---|---|
| ε2ε2 | 0.6 |
| ε2ε3 | 0.6 |
| ε3ε3 | 1.0 |
| ε2ε4 | 2.6 |
| ε3ε4 | 3.2 |

(continued)

| APOE Genotype | Odds Ratio |
| --- | --- |
| ε4ε4 | 14.9 |

Example 4: Information for Factor V Leiden Positive Patient

The following information is exemplary of information that could be supplied to an individual having a genomic SNP profile that shows the presence of the gene for Factor V Leiden. The individual may have a basic subscription in which the information may be supplied in an initial report.

What is Factor V Leiden?

Factor V Leiden is not a disease, it is the presence of a particular gene that is passed on from one's parents. Factor V Leiden is a variant of the protein Factor V (5) which is needed for blood clotting. People who have a Factor V deficiency are more likely to bleed badly while people with Factor V Leiden have blood that has an increased tendency to clot.

People carrying the Factor V Leiden gene have a five times greater risk of developing a blood clot (thrombosis) than the rest of the population. However, many people with the gene will never suffer from blood clots. In Britain and the United States, 5 per cent of the population carry one or more genes for Factor V Leiden, which is far more than the number of people who will actually suffer from thrombosis.

How do you get Factor V Leiden?

The genes for the Factor V are passed on from one's parents. As with all inherited characteristics, one gene is inherited from the mother and one from the father. So, it is possible to inherit: -two normal genes or one Factor V Leiden gene and one normal gene -or two Factor V Leiden genes. Having one Factor V Leiden gene will result in a slightly higher risk of developing a thrombosis, but having two genes makes the risk much greater.

What are the symptoms of Factor V Leiden?

There are no signs, unless you have a blood clot (thrombosis).

What are the danger signals?

The most common problem is a blood clot in the leg. This problem is indicated by the leg becoming swollen, painful and red. In rarer cases a blood clot in the lungs (pulmonary thrombosis) may develop, making it hard to breathe. Depending on the size of the blood clot this can range from being barely noticeable to the patient experiencing severe respiratory difficulty. In even rarer cases the clot might occur in an arm or another part of the body. Since these clots formed in the veins that take blood to the heart and not in the arteries (which take blood from the heart), Factor V Leiden does not increase the risk of coronary thrombosis.

What can be done to avoid blood clots?

Factor V Leiden only slightly increases the risk of getting a blood clot and many people with this condition will never experience thrombosis. There are many things one can do to avoid getting blood clots. Avoid standing or sitting in the same position for long periods of time. When traveling long distances, it is important to exercise regularly - the blood must not 'stand still'. Being overweight or smoking will greatly increase the risk of blood clots. Women carrying the Factor V Leiden gene should not take the contraceptive pill as this will significantly increase the chance of getting thrombosis. Women carrying the Factor V Leiden gene should also consult their doctor before becoming pregnant as this can also increase the risk of thrombosis.

How does a doctor find out if you have Factor V Leiden?

The gene for Factor V Leiden can be found in a blood sample.

A blood clot in the leg or the arm can usually be detected by an ultrasound examination.

Clots can also be detected by X-ray after injecting a substance into the blood to make the clot stand out. A blood clot in the lung is harder to find, but normally a doctor will use a radioactive substance to test the distribution of blood flow in the lung, and the distribution of air to the lungs. The two patterns should match - a mismatch indicates the presence of a clot.

How is Factor V Leiden treated?

People with Factor V Leiden do not need treatment unless their blood starts to clot, in which case a doctor will prescribe blood-thinning (anticoagulant) medicines such as warfarin (e.g. Marevan) or heparin to prevent further clots. Treatment will usually last for three to six months, but if there are several clots it could take longer. In severe cases the course of drug treatment may be continued indefinitely; in very rare cases the blood clots may need to be surgically removed.

How is Factor V Leiden treated during pregnancy?

Women carrying two genes for Factor V Leiden will need to receive treatment with a heparin coagulant medicine during pregnancy. The same applies to women carrying just one gene for Factor V Leiden who have previously had a blood clot themselves or who have a family history of blood clots.

All women carrying a gene for Factor V Leiden may need to wear special stockings to prevent clots during the last half of pregnancy. After the birth of the child they may be prescribed the anticoagulant drug heparin.

Prognosis

The risk of developing a clot increases with age, but in a survey of people over the age of 100 who carry the gene, it was found that only a few had ever suffered from thrombosis. The National Society for Genetic Counselors (NSGC) can provide a list of genetic counselors in your area, as well as information about creating a family history. Search their on-line database at www.nsgc-org/consumer.

Example 5: Generating Odds Ratio for an Individual of a Different Ancestry

**1.** *Find the LD probabilities based on the reference dataset.*

The number of HapMap individuals with genotype pair ($G_1$,$G_2$) at SNPs $S_1$,$S_2$ is counted to generate the joint distribution of the two SNPs. The marginal distributions of each of the SNPs is combined, using Bayes law, to estimate $P_{CEU}(S_1,G_1 \mid S_2, G_2)$ (CEU is the published, or first population) and with $P_{YRI}(S_1,G_1 \mid S_2, G_2)$ (YRI is the second population, ancestry of the individual)

**2.** *Find the counts F(P,CA,G), F(P,CT,G) if they are not given as an input (alternative 1-b).*

If the counts are not given as an input, the following set of equations is used to find them:

$$F(P,CA,NN)+F(P,CA,NR)+F(P,CA,RR)=N$$

$$F(P,CT,NN)+F(P,CT,NR)+F(P,CT,RR)=M$$

$$OR(P,CEU,RR)=\frac{F(P,CA,RR)F(P,CT,NN)}{F(P,CA,NN)F(P,CT,RR)}$$

$$OR(P,CEU,RN)=\frac{F(P,CA,RN)F(P,CT,NN)}{F(P,CA,NN)F(P,CT,RN)}$$

$$\frac{1}{F(P,CA,NN)}+\frac{1}{F(P,CA,RR)}+\frac{1}{F(P,CT,NN)}+\frac{1}{F(P,CT,RR)}=\left(\frac{\log\left(\frac{UB(P,CEU,RR)}{OR(P,CEU.RR)}\right)}{1.96}\right)^2$$

$$\frac{1}{F(P,CA,NN)}+\frac{1}{F(P,CA,RN)}+\frac{1}{F(P,CT,NN)}+\frac{1}{F(P,CT,RN)}=\left(\frac{\log\left(\frac{UB(P,CEU,RN)}{OR(P,CEU.RN)}\right)}{1.96}\right)^2$$

In the above equations, UB(P,CEU,G) is the upper bound on the confidence interval of the odds ratios for genotype G at the published SNP P. M and N are the number of controls and cases in the study respectively.

These are six equations with six variables. Enumeration over all values of F(P,CA,NN) and F(P,CA,RN) is performed. For each such pair of values, 2-4 equations determining the rest of the variables is present by solving a set of linear equations, and the last two equations are used for validation. The running time is bounded by $N^2$.

**3.** *Sample n (n very large, e.g. >> 1,000,000) instances for the genotype frequencies of the cases and controls at P, in CEU. The sampling is based on the posterior distribution of f(P,CA,G), f(P,CT,G), given the counts.*

Given f(P), the likelihood of seeing F(P) can be calculated under the assumption of a Multinomial distribution. By assuming a uniform prior on the possible values of f(P), it is known that the probability Prob(f(P)|F(P)) $\alpha$Prob(F(P) | f(P)). An MCMC approach is used to sample from this distribution using a Gibbs Sampler.

**4.**For each instance of the frequencies, and for each SNP S:

a) *Calculate f(S,CA,G) and f(S,CT,G) based on the frequencies in P and on $P_{CEU}$.*

The formula $f(S,CA,G)=\sum_{G'} f(P,CA,G')P_{CEU}(S,G\mid P,G')$ is used to estimate the frequencies at S in the cases. A similar formula can be used for the controls.

b) Generate an instance of F(S,CA,G), F(S,CT,G) based on the sampled allele frequencies in S. This is done by assuming a multinomial random variable that represents the genotype at S.

c) Calculate the p-value for S based on F(S), and based on f(S) (the latter is the p-value in the asymptotic sense). The Armitage-Trend test is used to calculate the p-value based on F(S). In order to calculate the asymptotic p-value, it is assumed a sample size of N cases and N controls, with counts that match the expectation, e.g., F(S,CA,G) will be assumed to be Nf(S,CA,G).

d) Find the min p-value based on F(S) across all measured SNPs S. If this is not P, then this instance is rejected.

e) If the instance is not rejected, the instance is kept, together with the min p-value based on f(S); this is the causal SNP of that instance.

**5.** The previous phase results in a set of causal SNPs$C_1,\ldots,C_n$, and their corresponding odds ratios. For each such causal, it is assumed that the same odds ratio holds for the YRI population.

a) *This information is used, together with the genotype frequencies in YRI at $C_i$ to estimate the frequencies $f_{YRI}(C_i,CA,G)$ for every genotype G.*

To do so, the following equation is solved:

$$\begin{aligned}
&f_{YRI}(S,CA,NN)+f_{YRI}(S,CA,RN)+f_{YRI}(S,CA,RR)=1\\
&OR(RR)=\frac{f_{YRI}(S,CA,RR)f_{YRI}(S,CT,NN)}{f_{YRI}(S,CA,NN)f_{YRI}(S,CT,RR)}\\
&OR(RN)=\frac{f_{YRI}(S,CA,RN)f_{YRI}(S,CT,NN)}{f_{YRI}(S,CA,NN)f_{YRI}(S,CT,RN)}
\end{aligned}$$

There are three missing variables in this equation, since $f_{YRI}$(S,CT,G) are assumed to be known from the reference population (HapMap). The above set of equations is therefore a set of linear equations and can be solved efficiently.

b) The LD information is used to estimate $f_{YRI}$(S,CA,G), $f_{YRI}$(S,CT,G) for every SNP S, and calculate the asymptotic odds ratios based on these frequencies. This is done in a similar manner to step **4**(a).

c) For each SNP S, the asymptotic odds ratios is averaged across all instances, resulting in the expected asymptotic odds ratios.

The odds ratios can then be used in determining an individual's genotype correlation.

## Claims

**1.** A method of correlating a genotype of an individual to a phenotype comprising:

(a) using a computer system for the following steps:

1) determining a first linkage disequilibrium (LD) pattern associated with a genetic variation correlated with a phenotype, wherein said first LD pattern is of a first population of individuals (CA) and determining a second LD pattern associated with said genetic variation, wherein said second LD pattern is of a second population of individuals (CT) and wherein said second population is of an ancestry different from said first population (CA);
2) determining the number of individuals of said first population CA and of said second population CT with a certain genotype G at a genetic variation P;
3) for each instance of the frequencies and for each genetic variation S in the proximity of P the computer system is used for:

(i) determining the allele frequencies of genotype G at a genetic variation S which is in the proximity of P in said first population CA and in said second population CT, based on the frequencies in the genetic variation P of the first population CA;
(ii) determining the number of individuals with a certain genotype G at a genetic variation S which is in the proximity of P, based on the allele frequencies in S;
(iii) determining the p-value for S based on the number of individuals with a certain genotype G at a genetic variation S which is in the proximity of P and on the allele frequencies of genotype G at a genetic variation S which is in the proximity of P;
(iv) determining the minimum p-value based on the number of individuals with a certain genotype G at a genetic variation S which is in the proximity of P across all measured genetic variations S;
(v) if the minimum p-value does not correspond to the proximity genetic variation P, the instance is rejected;
(vi) if the minimum p-value corresponds to the proximity genetic variation P, the genetic variation corresponding thereto will be the causal genetic variation (C) of that instance;

4) using the genotype frequencies of the second population CT at the causal genetic variation C for estimating the allelic frequencies for the second population CT for every genotype G, thereby determining a probability of said genetic variation being correlated with said phenotype in said second population), wherein said probability is a genotypic odds ratio (OR) and wherein said genotypic OR is derived from a known OR, wherein said known OR is for said genetic variation correlated with said phenotype for said first population;

(b) analyzing a genomic profile of said individual, wherein said individual is of an ancestry of said second population, wherein said genomic profile is generated using a high density DNA microarray, DNA sequencing, or RT-PCR;
(c) using a computer system to determine a correlation of said phenotype to the genomic profile of said individual comprising using said probability of step (a)4),
thereby correlating the individual's genotype to the individual's likelihood of being predisposed to said phenotype; and
(d) reporting results comprising said genotype correlation from step (c) to said individual or a health care manager of said individual by transmission of the results over a computer network.

**2.** The method of claim 1, wherein said genotype correlation to a phenotype is reported as a GCI score.

**3.** The method of claim 1, wherein said genetic variation is unknown.

**4.** The method of claim 1, wherein said genetic variation is a single nucleotide polymorphism (SNP), truncation, insertion, deletion, or repeat.

**5.** The method of claim 1 wherein said first or second population is a HapMap population (YRI,CEU,CHB,JPT,ASW,CHD,GIH,LWK,MEX,MKK,TSI) selected from the group of: African American, Caucasian, Ashkenazi Jewish, Sepharadic Jewish, Indian, Pacific Islander, Middle Eastern, Druze, Bedouin, South European, Scandinavian, Eastern European, North African, Basque, West African, and East African.

**6.** A computer system for correlating a genotype of an individual to a phenotype, the computer system comprising software code adapted to perform the following steps:

(a) determining a first linkage disequilibrium (LD) pattern associated with a genetic variation correlated with a phenotype, wherein said first LD pattern is of a first population of individuals (CA) and determining a second LD pattern associated with said genetic variation, wherein said second LD pattern is of a second population of individuals (CT) and wherein said second population is of an ancestry different from said first population (CA);
(b) determining the number of individuals of said first population CA and of said second population CT with a certain genotype G at a genetic variation P;
(c) for each instance of the frequencies and for each genetic variation S in the proximity of P the computer system is used for:

1) determining the allele frequencies of genotype G at a genetic variation S which is in the proximity of P in said first population CA and in said second population CT, based on the frequencies in the genetic variation P of the first population CA;
2) determining the number of individuals with a certain genotype G at a genetic variation S which is in the proximity of P, based on the allele frequencies in S;
3) determining the p-value for S based on the number of individuals with a certain genotype G at a genetic variation S which is in the proximity of P and on the allele frequencies of genotype G at a genetic variation S which is in the proximity of P;
4) determining the minimum p-value based on the number of individuals with a certain genotype G at a genetic variation S which is in the proximity of P across all measured genetic variations S;
5) if the minimum p-value does not correspond to the proximity genetic variation P, the instance is rejected;
6) if the minimum p-value corresponds to the proximity genetic variation P, the genetic variation corresponding thereto will be the causal genetic variation (C) of that instance;

(d) using the genotype frequencies of the second population CT at the causal genetic variation C for estimating the allelic frequencies for the second population CT for every genotype G, thereby determining a probability of said genetic variation being correlated with said phenotype in said second population , wherein said probability is a genotypic odds ratio (OR) and wherein said genotypic OR is derived from a known OR, wherein said known OR is for said genetic variation correlated with said phenotype for said first population;
(e) determining a correlation of the phenotype of said individual to the genomic profile of said individual comprising using said probability of step (d), thereby correlating the individual's genotype to the individual's likelihood of being predisposed to said phenotype, wherein said individual is of an ancestry of said second population and wherein said genomic profile has been generated using a high density DNA microarray, DNA sequencing, or RT-PCR; and,
(f) reporting results comprising said genotype correlation from step (c) to said individual or a health care manager of said individual by transmission of the results over a computer network.

**Patentansprüche**

**1.** Verfahren zum Korrelieren eines Genotyps eines Individuums mit einem Phänotyp, umfassend:

(a) Verwenden eines Computersystems für die folgenden Schritte:

(1) Bestimmen eines ersten Musters eines Kopplungsungleichgewichts (linkage disequilibrium; LD), das mit einer genetischen Variation, die mit einem Phänotyp korreliert, in Verbindung steht, wobei das erste LD-Muster von einer ersten Population von Individuen (CA) stammt, und Bestimmen eines zweiten LD-Musters, das mit der genetischen Variation in Verbindung steht, wobei das zweite LD-Muster von einer zweiten Population von Individuen (CT) stammt und wobei die zweite Population von einer Abstammung ist, die sich von der der ersten Population (CA) unterscheidet;
2) Bestimmen der Anzahl der Individuen der ersten Population CA und der zweiten Population CT mit einem bestimmten Genotyp G an einer genetischen Variation P;
3) für jedes Vorkommen der Häufigkeiten und für jede genetische Variation S in der Nähe von P wird das Computersystem verwendet zum:

(i) Bestimmen der Allel-Häufigkeiten des Genotyps G an einer genetischen Variation S, die in der ersten Population CA und in der zweiten Population CT in der Nähe von P ist, basierend auf den Häufigkeiten in der genetischen Variation P der ersten Population CA;
(ii) Bestimmen der Anzahl der Individuen mit einem bestimmten Genotyp G an einer genetischen Variation S, die in der Nähe von P ist, basierend auf den Allel-Häufigkeiten in S;
(iii) Bestimmen des p-Wertes für S basierend auf der Anzahl der Individuen mit einem bestimmten Genotyp G an einer genetischen Variation S, die in der Nähe von P ist, und basierend auf den Allel-Häufigkeiten des Genotyps G an einer genetischen Variation S, die in der Nähe von P ist;
(iv) Bestimmen des minimalen p-Wertes basierend auf der Anzahl der Individuen mit einem bestimmten Genotyp G an einer genetischen Variation S, die in der Nähe von P ist, über alle gemessenen genetischen Variationen S;
(v) wenn der minimale p-Wert nicht mit der in der Nähe befindlichen genetischen Variation P übereinstimmt, wird das Vorkommen verworfen;
(vi) wenn der minimale p-Wert mit der in der Nähe befindlichen genetischen Variation P übereinstimmt, wird die damit übereinstimmende genetische Variation die kausale genetische Variation (C) dieses Vorkommens sein;

4) Verwenden der Genotyp-Häufigkeiten der zweiten Population CT an der kausalen genetischen Variation C zum Abschätzen der Allel-Häufigkeiten für die zweite Population CT für jeden Genotyp G und dadurch Bestimmen einer Wahrscheinlichkeit, dass die genetische Variation mit dem Phänotyp in der zweiten Population korreliert, wobei die Wahrscheinlichkeit ein genotypisches Quotenverhältnis (odds ratio; OR) ist und wobei das genotypische OR von einem bekannten OR abgeleitet ist, wobei das bekannte OR für die genetische Variation ist, die mit dem Phänotyp der ersten Population korreliert;

(b) Analysieren eines genomischen Profils des Individuums, wobei das Individuum von einer Abstammung der zweiten Population ist, wobei das genomische Profil unter Verwendung eines DNA-Microarrays hoher Dichte, einer DNA-Sequenzierung oder RT-PCR erzeugt wurde;
(c) Verwendung eines Computersystems zur Bestimmung einer Korrelation des Phänotyps mit dem genomischen Profil des Individuums, umfassend das Verwenden der Wahrscheinlichkeit aus Schritt (a) 4), dadurch Korrelieren des Genotyps des Individuums mit der Wahrscheinlichkeit des Individuums, für diesen Phänotyp prädisponiert zu sein; und
(d) Berichten der Ergebnisse, umfassend die Genotyp-Korrelation aus Schritt (c), an das Individuum oder an einen Gesundheitsexperten des Individuums durch Übersenden der Ergebnisse über ein Computernetzwerk.

**2.** Verfahren nach Anspruch 1, wobei die Genotyp-Korrelation mit einem Phänotyp als GCI-Wert angegeben wird.

**3.** Verfahren nach Anspruch 1, wobei die genetische Variation unbekannt ist.

**4.** Verfahren nach Anspruch 1, wobei die genetische Variation ein Einzelnucleotid-Polymorphismus (single nucleotide polymorphism; SNP), eine Verkürzung, Insertion, Deletion oder Wiederholung ist.

**5.** Verfahren nach Anspruch 1, wobei die erste oder die zweite Population eine HapMap-Population (YRI, CEU, CHB, JPT, ASW, CHD, GIH, LWK, MEX, MKK, TSI) ist, ausgewählt aus der Gruppe aus: afroamerikanisch, kaukasisch, aschkenasisch jüdisch, sephardisch jüdisch, indisch, pazifisch insulanisch, mittelöstlich, drusisch, beduinisch, südeuropäisch, skandinavisch, osteuropäisch, nordafrikanisch, baskisch, westafrikanisch und ostafrikanisch.

**6.** Computersystem zum Korrelieren eines Genotyps eines Individuums mit einem Phänotyp, wobei das Computersystem ein Softwareprogramm umfasst, das angepasst ist, um die folgenden Schritte auszuführen:

(a) Bestimmen eines ersten Musters eines Kopplungsungleichgewichts (LD), das mit einer genetischen Variation in Verbindung steht, die mit einem Phänotyp korreliert, wobei das erste LD-Muster von einer ersten Population von Individuen (CA) stammt, und Bestimmen eines zweiten LD-Musters, das mit der genetischen Variation in Verbindung steht, wobei das zweite LD-Muster von einer zweiten Population von Individuen (CT) stammt und wobei die zweite Population von einer Abstammung ist, die sich von der der ersten Population (CA) unterscheidet;
(b) Bestimmen der Anzahl an Individuen der ersten Population CA und der zweiten Population CT mit einem bestimmten Genotyp G an einer genetischen Variation P;
(c) Für jedes Vorkommen der Häufigkeiten und für jede genetische Variation S in der Nähe von P wird das Computersystem verwendet zum:

1) Bestimmen der Allel-Häufigkeiten des Genotyps G an einer genetischen Variation S, die in der ersten Population CA und in der zweiten Population CT in der Nähe von P ist, basierend auf den Häufigkeiten in der genetischen Variation P der ersten Population CA;
2) Bestimmen der Anzahl der Individuen mit einem bestimmten Genotyp G an einer genetischen Variation S, die in der Nähe von P ist, basierend auf den Allel-Häufigkeiten in S;
3) Bestimmen des p-Werts für S basierend auf der Anzahl der Individuen mit einem bestimmten Genotyp G an einer genetischen Variation S, die in der Nähe von P ist, und basierend auf den Allel-Häufigkeiten des Genotyps G an einer genetischen Variation S, die in der Nähe von P ist;
4) Bestimmen des minimalen p-Werts basierend auf der Anzahl der Individuen mit einem bestimmten Genotyp G an einer genetischen Variation S, die in der Nähe von P ist, über alle gemessenen genetischen Variationen S;
5) wenn der minimale p-Wert nicht mit der in der Nähe befindlichen genetischen Variation P übereinstimmt, wird das Vorkommen verworfen;
6) wenn der minimale p-Wert mit der in der Nähe befindlichen genetischen Variation P übereinstimmt, wird die damit übereinstimmende genetische Variation die kausale genetische Variation (C) dieses Vorkommens sein;

(d) Verwenden der Genotyp-Häufigkeiten der zweiten Population CT an der kausalen genetischen Variation C zum Abschätzen der Allel-Häufigkeiten für die zweite Population CT für jeden Genotyp G und dadurch Bestimmen einer Wahrscheinlichkeit, dass die genetische Variation mit dem Phänotyp in der zweiten Population korreliert, wobei die Wahrscheinlichkeit ein genotypisches Quotenverhältnis (OR) ist und wobei das genotypische OR von einem bekannten OR abgeleitet ist, wobei das bekannte OR für die genetische Variation ist, die mit dem Phänotyp der ersten Population korreliert;
(e) Bestimmen einer Korrelation des Phänotyps des Individuums mit dem genomischen Profil des Individuums, umfassend die Verwendung der Wahrscheinlichkeit aus Schritt (d), dadurch Korrelieren des Genotyps des Individuums mit der Wahrscheinlichkeit des Individuums, für diesen Phänotyp prädisponiert zu sein, wobei das Individuum von einer Abstammung der zweiten Population ist und wobei das genomische Profil unter Verwendung eines DNA-Microarrays hoher Dichte, einer DNA-Sequenzierung oder RT-PCR erzeugt wurde; und
(f) Berichten der Ergebnisse, umfassend die Genotyp-Korrelation aus Schritt (c), an das Individuum oder an einen Gesundheitsexperten des Individuums durch Übersenden der Ergebnisse über ein Computernetzwerk.

## Revendications

**1.** Méthode de corrélation d'un génotype d'un individu à un phénotype comprenant :

(a) l'utilisation d'un système informatique pour les étapes suivantes :

1) la détermination d'un premier profil de déséquilibre de liaison (LD) associé à une variation génétique corrélée à un phénotype, où ledit premier profil de LD est d'une première population d'individus (CA) et la détermination d'un second profil de LD associé à ladite variation génétique, où ledit second profil de LD est d'une seconde population d'individus (CT) et où ladite seconde population est d'une origine ancestrale différente de ladite première population (CA) ;
2) la détermination du nombre d'individus de ladite première population CA et de ladite seconde population CT avec un certain génotype G au niveau d'une variation génétique P ;
3) pour chaque cas des fréquences et pour chaque variation génétique S à proximité de P, le système informatique est utilisé pour :

(i) la détermination des fréquences alléliques du génotype G au niveau d'une variation génétique S qui est à proximité de P dans ladite première population CA et dans ladite seconde population CT, en se basant sur les fréquences de la variation génétique P de la première population CA ;
(ii) la détermination du nombre d'individus avec un certain génotype G au niveau d'une variation génétique S qui est à proximité de P, en se basant sur les fréquences alléliques de S ;
(iii) la détermination de la valeur p pour S en se basant sur le nombre d'individus avec un certain génotype G au niveau d'une variation génétique S qui est à proximité de P et sur les fréquences alléliques du génotype G au niveau d'une variation génétique S qui est à proximité de P ;
(iv) la détermination de la valeur p minimale en se basant sur le nombre d'individus avec un certain génotype G au niveau d'une variation génétique S qui est à proximité de P sur toutes les variations génétiques S mesurées ;
(v) si la valeur p minimale ne correspond pas à la variation génétique P de proximité, le cas est rejeté ;
(vi) si la valeur p minimale correspond à la variation génétique P de proximité, la variation génétique y correspondant sera la variation génétique causale (C) de ce cas ;

4) l'utilisation des fréquences de génotype de la seconde population CT au niveau de la variation génétique causale C pour estimer les fréquences alléliques pour la seconde population CT pour chaque génotype G, déterminant de cette façon une probabilité de ladite variation génétique d'être corrélée audit phénotype dans ladite seconde population, où ladite probabilité est un rapport des cotes (« odds ratio », OR) génotypique et où ledit OR génotypique est dérivé d'un OR connu, où ledit OR connu est pour ladite variation génétique corrélée avec ledit phénotype pour ladite première population ;

(b) l'analyse d'un profil génomique dudit individu, où ledit individu est d'une origine ancestrale de ladite seconde population, où ledit profil génomique est généré en utilisant une micropuce d'ADN de haute densité, un séquençage d'ADN ou une RT-PCR ;
(c) l'utilisation d'un système informatique pour déterminer une corrélation dudit phénotype au profil génomique dudit individu comprenant l'utilisation de ladite probabilité de l'étape (a)4), corrélant de cette façon le génotype de l'individu à la probabilité de l'individu d'être prédisposé audit phénotype ; et
(d) le rapport des résultats comprenant ladite corrélation du génotype de l'étape (c) audit individu ou à un responsable des soins de santé dudit individu par transmission des résultats sur un réseau informatique.

**2.** Méthode selon la revendication 1, dans laquelle ladite corrélation du génotype à un phénotype est rapportée sous la forme d'un score GCI.

**3.** Méthode selon la revendication 1, dans laquelle ladite variation génétique est inconnue.

**4.** Méthode selon la revendication 1, dans laquelle ladite variation génétique est un polymorphisme d'un seul nucléotide (SNP), une troncature, une insertion, une délétion, ou une répétition.

**5.** Méthode selon la revendication 1, dans laquelle ladite première ou seconde population est une population HapMap (YRI, CEU, CHB, JPT, ASW, CHD, GIH, LWK, MEX, MKK, TSI) choisie dans le groupe des : afro-américains, caucasiens, juifs ashkénazes, juifs séfarades, indiens, habitants des îles du Pacifique, du Moyen-Orient, druzes, bédouins, européens du Sud, scandinaves, européens de l'Est, nord-africains, basques, africains de l'Ouest, et africains de l'Est.

**6.** Système informatique pour la corrélation d'un génotype d'un individu à un phénotype, le système informatique comprenant un code de logiciel adapté pour effectuer les étapes suivantes :

(a) la détermination d'un premier profil de déséquilibre de liaison (LD) associé à une variation génétique corrélée à un phénotype, où ledit premier profil de LD est d'une première population d'individus (CA) et la détermination d'un second profil de LD associé à ladite variation génétique, où ledit second profil de LD est d'une seconde population d'individus (CT) et où ladite seconde population est d'une origine ancestrale différente de ladite première population (CA) ;
(b) la détermination du nombre d'individus de ladite première population CA et de ladite seconde population CT avec un certain génotype G au niveau d'une variation génétique P ;
(c) pour chaque cas des fréquences et pour chaque variation génétique S à proximité de P, le système informatique est utilisé pour :

1) la détermination des fréquences alléliques du génotype G au niveau d'une variation génétique S qui est à proximité de P dans ladite première population CA et dans ladite seconde population CT, en se basant sur les fréquences de la variation génétique P de la première population CA ;
2) la détermination du nombre d'individus avec un certain génotype G au niveau d'une variation génétique S qui est à proximité de P, en se basant sur les fréquences alléliques de S ;
3) la détermination de la valeur p pour S en se basant sur le nombre d'individus avec un certain génotype G au niveau d'une variation génétique S qui est à proximité de P et sur les fréquences alléliques du génotype G au niveau d'une variation génétique S qui est à proximité de P ;
4) la détermination de la valeur p minimale en se basant sur le nombre d'individus avec un certain génotype G au niveau d'une variation génétique S qui est à proximité de P sur toutes les variations génétiques S mesurées ;

5) si la valeur p minimale ne correspond pas à la variation génétique P de proximité, le cas est rejeté ;

6) si la valeur p minimale correspond à la variation génétique P de proximité, la variation génétique y correspondant sera la variation génétique causale (C) de ce cas ;

(d) l'utilisation des fréquences de génotype de la seconde population CT au niveau de la variation génétique causale C pour estimer les fréquences alléliques pour la seconde population CT pour chaque génotype G, déterminant de cette façon une probabilité de ladite variation génétique d'être corrélée avec ledit phénotype dans ladite seconde population, où ladite probabilité est un rapport des cotes (OR) génotypique et où ledit OR génotypique est dérivé d'un OR connu, où ledit OR connu est pour ladite variation génétique corrélée audit phénotype pour ladite première population ;

(e) la détermination d'une corrélation du phénotype dudit individu au profil génomique dudit individu comprenant l'utilisation de ladite probabilité de l'étape (d), corrélant de cette façon le génotype de l'individu à la probabilité de l'individu d'être prédisposé audit phénotype, où ledit individu est d'une origine ancestrale de ladite seconde population et où ledit profil génomique a été généré en utilisant une micropuce d'ADN de haute densité, un séquençage d'ADN, ou une RT-PCR ; et

(f) le rapport des résultats comprenant ladite corrélation du génotype de l'étape (c) audit individu ou à un responsable des soins de santé dudit individu par transmission des résultats sur un réseau informatique.

FIG. 1

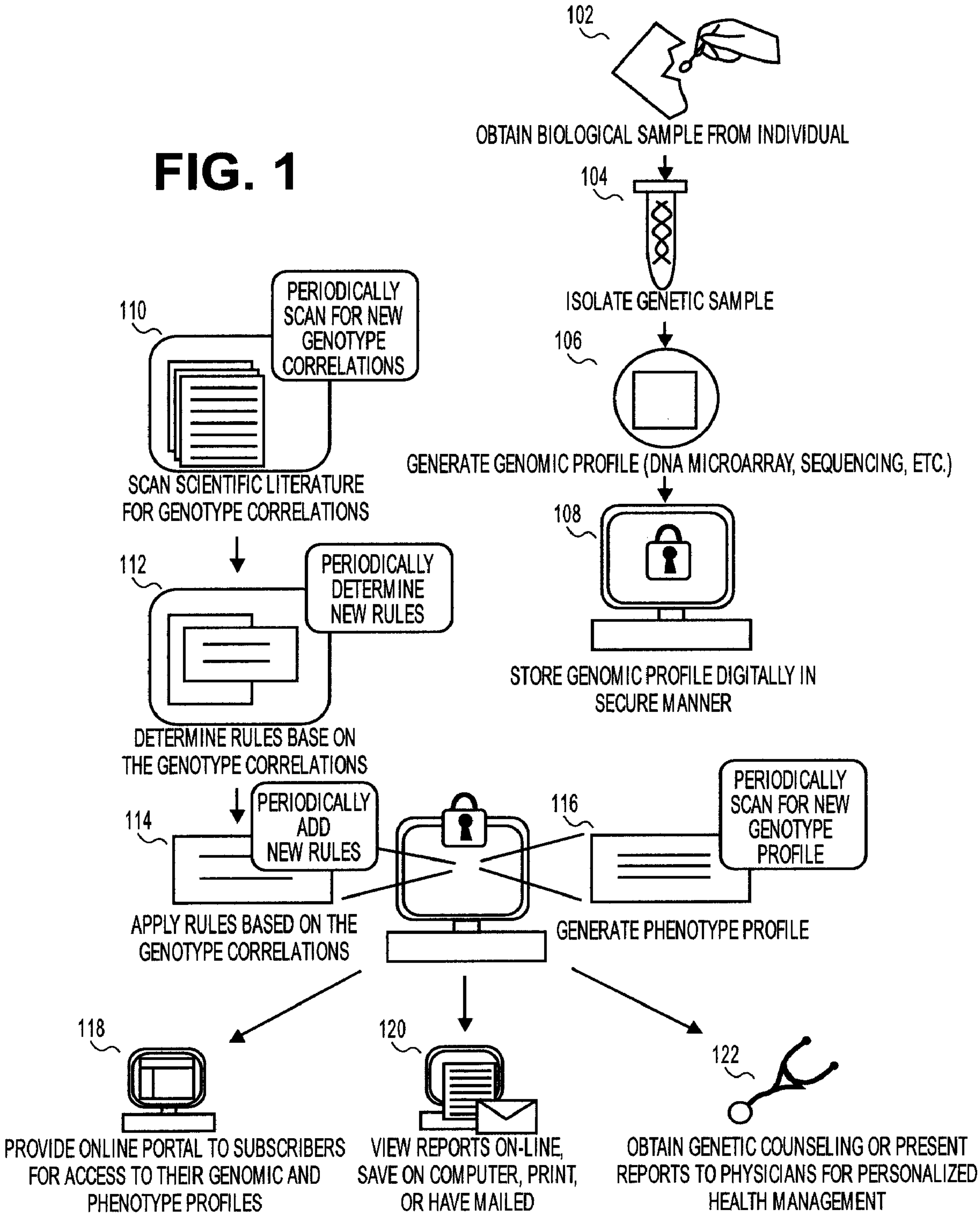
102
OBTAIN BIOLOGICAL SAMPLE FROM INDIVIDUAL
104
ISOLATE GENETIC SAMPLE
106
GENERATE GENOMIC PROFILE (DNA MICROARRAY, SEQUENCING, ETC.)
108
STORE GENOMIC PROFILE DIGITALLY IN SECURE MANNER
110
PERIODICALLY SCAN FOR NEW GENOTYPE CORRELATIONS
SCAN SCIENTIFIC LITERATURE FOR GENOTYPE CORRELATIONS
112
PERIODICALLY DETERMINE NEW RULES
DETERMINE RULES BASE ON THE GENOTYPE CORRELATIONS
114
PERIODICALLY ADD NEW RULES
APPLY RULES BASED ON THE GENOTYPE CORRELATIONS
116
PERIODICALLY SCAN FOR NEW GENOTYPE PROFILE
GENERATE PHENOTYPE PROFILE
118
PROVIDE ONLINE PORTAL TO SUBSCRIBERS FOR ACCESS TO THEIR GENOMIC AND PHENOTYPE PROFILES
120
VIEW REPORTS ON-LINE, SAVE ON COMPUTER, PRINT, OR HAVE MAILED
122
OBTAIN GENETIC COUNSELING OR PRESENT REPORTS TO PHYSICIANS FOR PERSONALIZED HEALTH MANAGEMENT

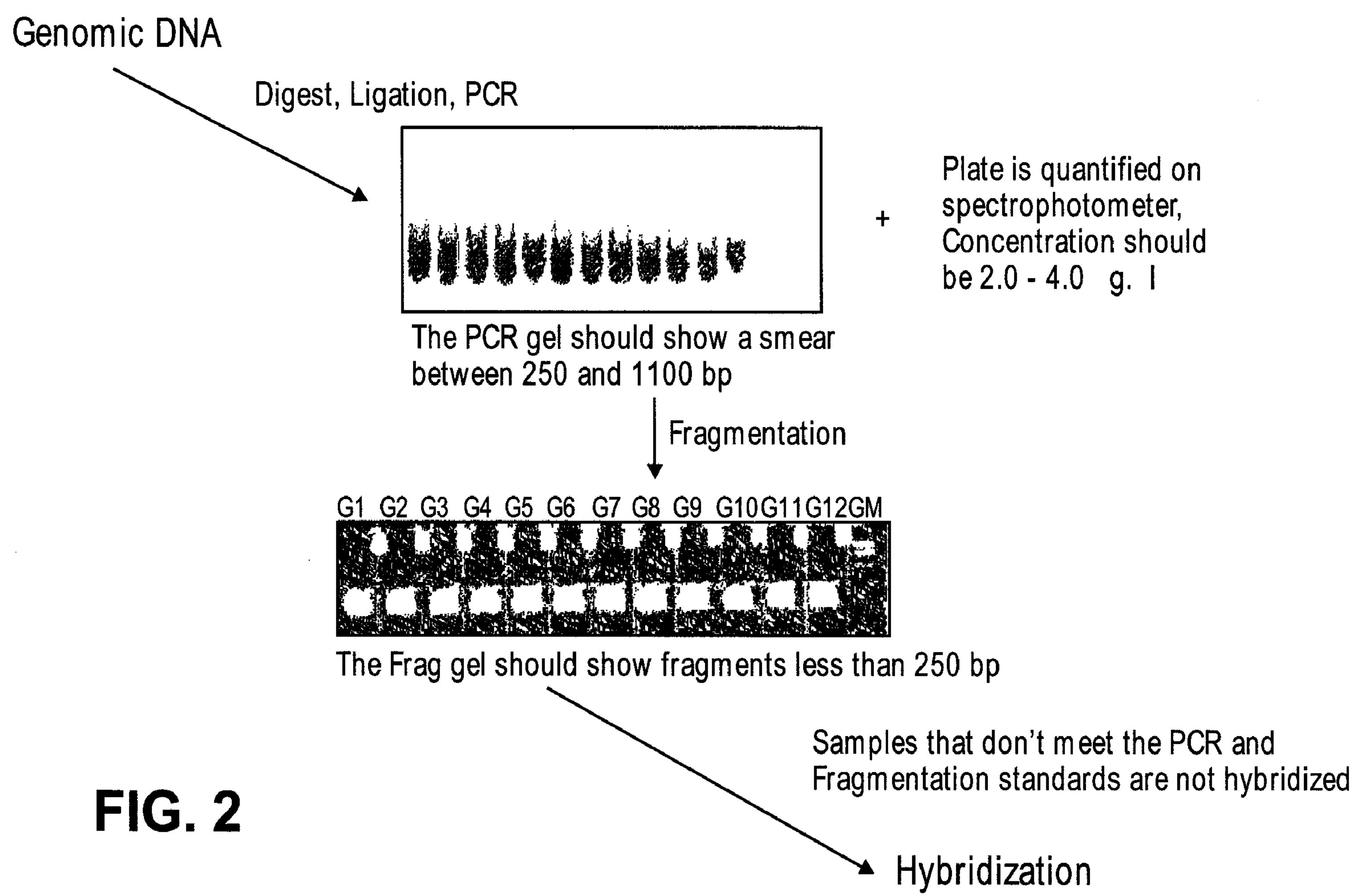
Genomic DNA
Digest, Ligation, PCR
+
Plate is quantified on spectrophotometer, Concentration should be 2.0 - 4.0 g. l
The PCR gel should show a smear between 250 and 1100 bp
Fragmentation
G1 G2 G3 G4 G5 G6 G7 G8 G9 G10 G11 G12 GM
The Frag gel should show fragments less than 250 bp
Samples that don't meet the PCR and Fragmentation standards are not hybridized
Hybridization

FIG. 2

FIG. 3

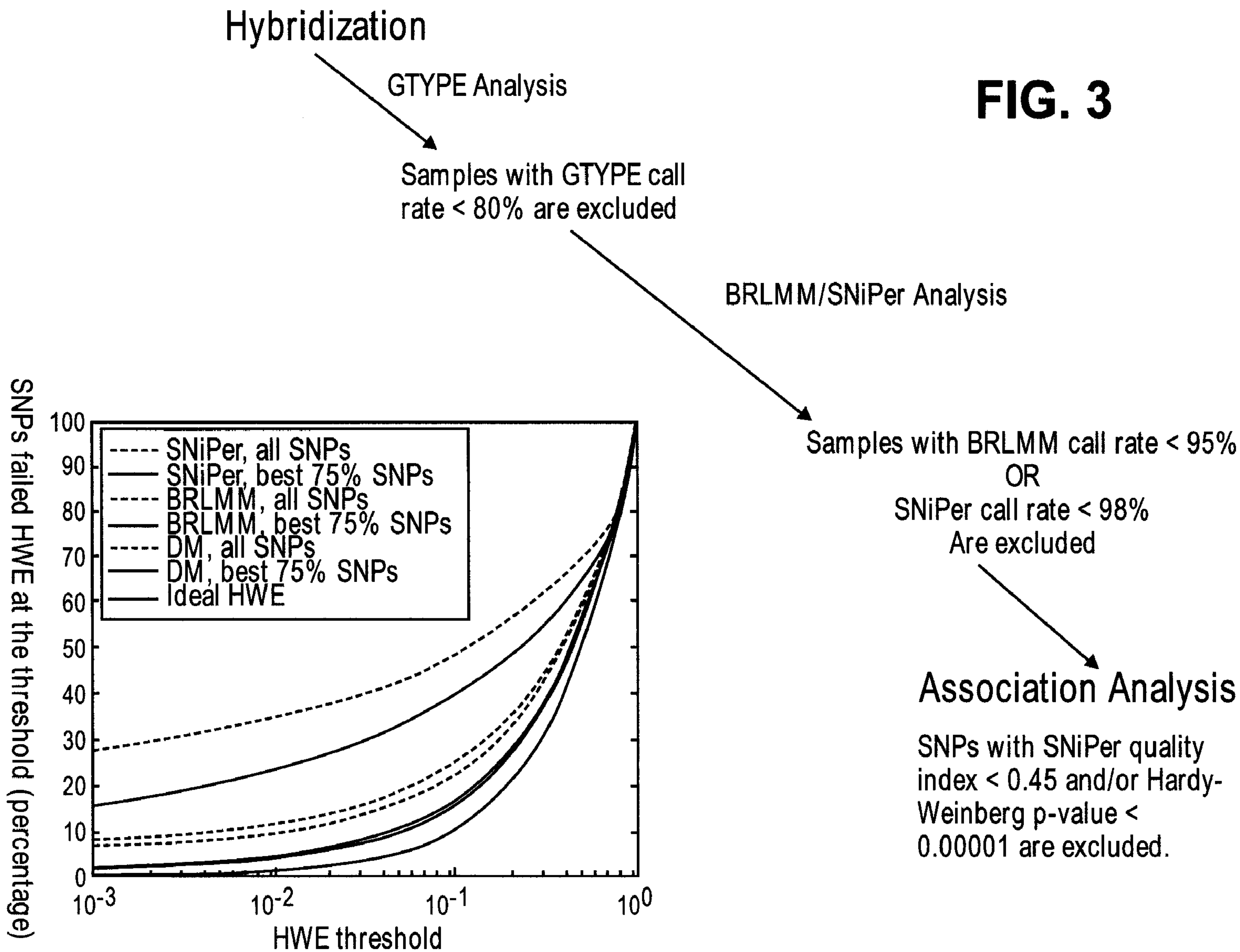
Hybridization
GTYPE Analysis
Samples with GTYPE call rate < 80% are excluded
BRLMM/SNiPer Analysis
Samples with BRLMM call rate < 95%
OR
SNiPer call rate < 98%
Are excluded
Association Analysis
SNPs with SNiPer quality index < 0.45 and/or Hardy-Weinberg p-value < 0.00001 are excluded.
SNiPer, all SNPs
SNiPer, best 75% SNPs
BRLMM, all SNPs
BRLMM, best 75% SNPs
DM, all SNPs
DM, best 75% SNPs
Ideal HWE
SNPs failed HWE at the threshold (percentage)
100
90
80
70
60
50
40
30
20
10
0
$10^{-3}$
$10^{-2}$
$10^{-1}$
$10^{0}$
HWE threshold

| Short Phenotype Name | Locus | Gene (anchaloc on B36) | Gender applicability (F, M, B) | Test SNP | CHR | B3S Location | Test Risk Allele (plus, R) | Test NonRisk Allele (plus, N) | Ethnicity Race-distr |
|---|---|---|---|---|---|---|---|---|---|
| AD | AD_1 | APOE | B | rs4420638 | chr19 | 50114785 | G | A | CEU |
| BC | BC_1 | FGFR2 | F | rs2981582 | chr10 | 123342306 | T | C | CEU/AS |
| BC | BC_2 | TNRC9 | F | rs3803662 | chr16 | 51143841 | T | C | CEU/AS |
| BC | BC_3 | MAP3K1 | F | rs4700485 | chr5 | 56067640 | G | A | CEU/AS |
| BC | BC_4 | LSP1 | F | rs3817198 | chr11 | 1865581 | C | T | CEU/AS |
| BC | BC_5 | CASP8 | F | rs17468277 | chr2 | 201857833 | C | T | CEU |
| BCERP | BCERP_1 | chr2.217614077 | F | rs6721996 | chr2 | 217614076 | G | A | CEU |
| BCERP | BCERP_2 | TNRC9 | F | rs3803662 | chr16 | 51143841 | T | C | CEU |
| BMIOB | BMIOB_1 | FTO | B | rs9939609 | chr16 | 52378027 | A | T | CEU |
| BMIOW | BMIOW_1 | FTO | B | rs9939609 | chr16 | 52378027 | A | T | CEU |
| BP | BP_1 | PALB2 | B | rs420259 | chr16 | 23541526 | T | C | CEU |
| CD | CD_1 | chr10.101277754 | B | rs1088365 | chr10 | 101277754 | G | A | CEU |
| CD | CD_2 | PTGER4 | B | rs17234657 | chr5 | 404037265 | G | T | CEU |
| CD | CD_3 | ATG16L1 | B | rs10210302 | chr2 | 233823577 | T | C | CEU |
| CD | CD_4 | BSN | B | rs9858542 | chr3 | 49676986 | A | G | CEU |
| CD | CD_5 | IL23R | B | rs11805303 | chr1 | 67448103 | T | C | CEU |
| CD | CD_6 | IRGM | B | rs1000113 | chr5 | 150220268 | T | C | CEU |
| CD | CD_7 | NOD2 (CARD15) | B | rs17221417 | chr16 | 49297082 | G | C | CEU |
| CD | CD_8 | PTPN2 | B | rs2542151 | chr18 | 12769946 | G | T | CEU |
| CD | CD_9 | ZNF365 | B | rs10761659 | chr10 | 64115569 | G | A | CEU |
| CelD | CelD_1 | IL2-IL22 Locus | B | rs6840978 | chr4 | 123774157 | C | T | CEU |
| CelD | CelD_2 | HLA-DQ2.5cis | B | rs2187668 | chr6 | 32713862 | A | G | CEU |
| CelD | CelD_3 | CTLA4 | B | rs11571315 | chr2 | 204442732 | A | G | CEU |
| EMI | EMI_1 | THBS4 | B | rs1866389 | chr5 | 79397020 | C | G | CEU |

FIG. 4A

| Short Phenotype Name | Locus | Gene (anchaloc on B36) | Gender applicability (F, M, B) | Test SNP | CHR | B3S Location | Test Risk Allele (plus, R) | Test NonRisk Allele (plus, N) | Ethnicity Race-distr |
|---|---|---|---|---|---|---|---|---|---|
| EMI | EMI_2 | 9p21 | B | rs1333049 | chr9 | 22114476 | C | G | CEU |
| MI | MI_1 | 9p21 | B | rs1333049 | chr9 | 22114476 | C | G | CEU |
| OAK | OAK_1 | GDF5 | B | rs4911178 | chr20 | 33489396 | A | G | CHB |
| PC | PC_1 | 8q24_R1 | M | rs9643226 | chr8 | 128563663 | C | G | CEU |
| PC | PC_2 | 8q24_R3 | M | rs6983267 | chr8 | 128482487 | G | T | CEU |
| RA | RA_1 | PTPN22 | B | rs6679677 | chr1 | 114105330 | A | C | CEU |
| RA | RA_2 | MHC | B | rs6457617 | chr6 | 32771828 | T | C | CEU |
| T2D | T2D_1 | CDKAL1 | B | rs7754840 | chr6 | 20769228 | C | G | CEU |
| T2D | T2D_10 | TCF7L2 | B | rs4506565 | chr10 | 114746030 | T | A | CEU |
| T2D | T2D_2 | CDKAL1 | B | rs7756992 | chr6 | 20787687 | G | A | CHB |
| T2D | T2D_2 | CDKAL1 | B | rs7756992 | chr6 | 20787687 | G | A | CEU |
| T2D | T2D_3 | CDKN2A/B | B | rs10811661 | chr9 | 22124093 | T | C | CEU |
| T2D | T2D_4 | Chr11.41871942 | B | rs12804210 | chr11 | 41871941 | T | C | CEU |
| T2D | T2D_5 | FTO | B | rs8050136 | chr16 | 52373775 | A | C | CEU |
| T2D | T2D_6 | HHEX | B | rs1111875 | chr10 | 94452861 | G | A | CEU |
| T2D | T2D_7 | IGF2BP2 | B | rs4402960 | chr3 | 186994380 | T | G | CEU |
| T2D | T2D_8 | KCNJ11 | B | rs5215 | chr11 | 17366147 | C | T | CEU |
| T2D | T2D_9 | PPARG | B | rs1801282 | chr3 | 12368124 | C | G | CEU |
| AMD | AMD_1 | GRK5 | B | rs1537576 | chr10 | 39926059 | C | G | CEU |
| AMD | AMD_2 | LOC387715 | B | rs10490924 | chr10 | 124204438 | T | G | CEU |
| AMD | AMD_3 | CFH | B | rs10737680 | chr1 | 194946078 | C | A | CEU |
| AMD | AMD_4 | CFB-C2 | B | rs541862 | chr6 | 32024930 | A | G | CEU |

FIG. 4B

| Short Phenotype Name | Gene (or chr.loc on B36) | Functional on published SNP | Published Risk allele (plus) | Published Non Risk allele (plus) | UNITS for effect estimate | Effect Estimate | Genotypic risk: risk homoz (RR vs NN) | Genotypic risk heteroz (RN vs NN) |
|---|---|---|---|---|---|---|---|---|
| AD | APOE | rs429358 & rs7412 (ApoE2/3/4 SNPs) | C | T | OR (95% CI) | genotypic | 21.59 (9.88, 47.21 | 5.02 (3.72, 6.77) |
| BC | FGFR2 | rs2981582 | T | C | OR (95% CI) | genotypic | 1.63 (1.51, 1.72) | 1.23 (1.18, 1.28) |
| BC | TNRC9 | rs3803662 | T | C | OR (95% CI) | genotypic | 1.39(1.26, 1.45) | 1.23(1.18, 1.29) |
| BC | MAP3K1 | rs889312 | C | A | OR (95% CI) | genotypic | 1.13(1.09, 1.18) | 1.13(1.10, 1.16) |
| BC | LSP1 | rs3817198 | C | T | OR (95% CI) | genotypic | 1.17(1.08, 1.25) | 1.06(1.02, 1.11) |
| BC | CASP8 | rs1045485 | G | C | OR (95% CI) | genotypic | 1.35(1.04, 1.18) | 1.12(1.15, .61) |
| BCERP | chr2.217614077 | rs13387042 | A | G | OR (95% CI) | genotypic | 1.44(1.30, 1.58) | 1.11(1.03, 1.20) |
| BCERP | TNRC9 | rs3803662 | T | C | OR (95% CI) | genotypic | 1.64(1.45, 1.85) | 1.27(1.19, 1.36) |
| BMIOB | FTO | rs9939609 | A | T | OR (95% CI) | genotypic | 1.74(1.60, 1.89) | 1.31(1.23, 1.39) |
| BMIOW | FTO | rs9939609 | A | T | OR (95% CI) | genotypic | 1.42(1.32, 1.52) | 1.17(1.11, 1.23) |
| BP | PALB2 | rs420259 | T | C | OR (95% CI) | genotypic | 2.07(1.60, 2.69) | 2.08(1.60, 2.71) |
| CD | chr10.101277754 | rs1088365 | G | A | OR (95% CI) | genotypic | 1.62(1.37, 1.92) | 1.2(1.03, 1.39) |
| CD | PTGER4 | rs17234657 | G | T | OR (95% CI) | genotypic | 2.32(1.59, 3.39) | 1.54(1.34, 1.76) |
| CD | ATG16L1 | rs10210302 | T | C | OR (95% CI) | genotypic | 1.85(1.56, 2.21) | 1.19(1.01, 1.41) |
| CD | BSN | rs9858542 | A | G | OR (95% CI) | genotypic | 1.84(1.49, 2.26) | 1.09(0.96, 1.24) |
| CD | IL23R | rs11805303 | T | C | OR (95% CI) | genotypic | 1.86(1.54, 2.24) | 1.39(1.22, 1.58) |
| CD | IRGM | rs1000113 | T | C | OR (95% CI) | genotypic | 1.92(0.92, 4.00) | 1.54(1.31, 1.82) |
| CD | NOD2 (CARD15) | rs17221417 | G | C | OR (95% CI) | genotypic | 1.92(1.58, 2.34) | 1.29(1.13, 1.46) |
| CD | PTPN2 | rs2542151 | G | T | OR (95% CI) | genotypic | 2.01(1.46, 2.76) | 1.3(1.13, 1.48) |
| CD | ZNF365 | rs10761659 | G | A | OR (95% CI) | genotypic | 1.55(1.30, 1.84) | 1.23(1.05, 1.45) |
| CelD | IL2-IL22 locus | rs6840978 | C | T | OR (95% CI) | allelic | | |
| CelD | HLA-DQ2.5cis | rs2187668 | A | G | OR (95% CI) | allelic | | |
| CelD | CTLA4 | rs231779 | T | C | OR (95% CI) | allelic | | |

FIG. 4C

| Short Phenotype Name | Gene (or chr.loc on B36) | Functional on published SNP | Published Risk allele (plus) | Published Non Risk allele (plus) | UNITS for effect estimate | Effect Estimate | Genotypic risk: risk homoz (RR vs NN) | Genotypic risk heteroz (RN vs NN) |
|---|---|---|---|---|---|---|---|---|
| EMI | THBS4 | rs1866389 | C | G | OR (95% CI) | genotypic | 1.64 (0.75, 3.57) | 1.85(1.28, 2.67) |
| EMI | 9p21 | rs10757278 | G | A | OR (95% CI) | genotypic | 2.08(1.69, 2.58) | 1.56(1.32, 1.85) |
| MI | 9p21 | rs10757278 | G | A | OR (95% CI) | genotypic | 1.72(1.45, 2.03) | 1.28(1.14, 1.45) |
| OAK | GDF5 | rs143383 | T | C | OR (95% CI) | genotypic | 2.04(1.16, 3.58) | 1.27(0.71, 2.28) |
| PC | 8q24_R1 | rs1447295 | A | C | OR (95% CI) | genotypic | 1.43(1.29, 1.59) | 2.23(1.58, 3.14) |
| PC | 8q24_R3 | rs6983267 | G | T | OR (95% CI) | genotypic | 1.26(1.13, 1.41) | 1.58(1.40, 1.78) |
| RA | PTPN22 | rs6679677 | A | C | OR (95% CI) | genotypic | 3.32(1.93, 5.59) | 1.98(1.72, 2.27) |
| RA | MHC | rs6457617 | T | C | OR (95% CI) | genotypic | 5.21(4.31, 6.30) | 2.36(1.97, 2.84) |
| T2D | CDKAL1 | rs7754840 | C | G | OR (95% CI) | genotypic | 1.34(1.23, 1.47) | 1.33(1.22, 1.45) |
| T2D | TCF7L2 | rs4506565 | T | A | OR (95% CI) | genotypic | 1.88(1.56, 2.27) | 1.36(1.20, 1.54) |
| T2D | CDKAL1 | rs7756992 | G | A | OR (95% CI) | genotypic | 1.52(1.21, 1.90) | 1.27(1.05, 1.55) |
| T2D | CDKAL1 | rs7756992 | G | A | OR (95% CI) | genotypic | 1.50(1.31, 1.72) | 1.15(1.06, 1.24) |
| T2D | CDKN2A/B | rs10811661 | T | C | OR (95% CI) | genotypic | 1.39(1.13, 1.71) | 1.16(0.94, 1.43) |
| T2D | Chr11.41871942 | rs9300039 | C | A | OR (95% CI) | genotypic | 2.61(1.33, 5.11) | 1.80(0.91, 3.57) |
| T2D | FTO | rs8050136 | A | C | OR (95% CI) | genotypic | 1.49(1.33, 1.68) | 1.15(1.06, 1.26) |
| T2D | HHEX | rs1111875 | G | A | OR (95% CI) | genotypic | 1.20(1.10, 1.31) | 1.06(0.98, 1.16) |
| T2D | IGF2BP2 | rs4402960 | T | G | OR (95% CI) | genotypic | 1.21(1.10, 1.34) | 1.16(1.09, 1.24) |
| T2D | KCNJ11 | rs5219 | T | C | OR (95% CI) | genotypic | 1.22(1.04, 1.44) | 1.12(0.98, 1.28) |
| T2D | PPARG | rs1801282 | C | G | OR (95% CI) | genotypic | 1.53(1.08, 2.16) | 1.30(0.91, 1.86) |
| AMD | GRK5 | rs1537576 | C | G | OR (n/a CI) | genotypic | | |
| AMD | LOC387715 | rs10490924 | T | G | OR (n/a CI) | genotypic | 1.9 | n/a |
| AMD | CFH | rs10737680 | C | A | OR (n/a CI) | genotypic | 2.7 | 7.60 |
| AMD | CFB-C2 | rs641153 | C | T | OR (n/a CI) | genotypic | 9.5 | 3.10 |

FIG. 4D

| Short Phenotype Name | Gene (or chr.loc on B36) | Genotypic risk: nonrisk homoz (NN vs NN) | Carrier Risk (RR or RN - vs NN) | Allelic Risk (R vs N) | Seminal publication | DIRECT or TAG SNP | Test SNP RR freq in HapMap CEU | Test SNP RN freq In HapMap CEU |
|---|---|---|---|---|---|---|---|---|
| AD | APOE | 1 | 6.34 (4.76, 8.44) | | Coon et al., J. Clin. Psychiatry 68:613-618 (2007) | TAG | 3% | 30% |
| BC | FGFR2 | 1.00 | | | Easton et al., Nature 447:1087-1093 (2007) | DIRECT | 22% | 40% |
| BC | TNRC9 | 1.00 | | | Easton et al., Nature 447:1087-1093 (2007) | DIRECT | 12% | 37% |
| BC | MAP3K1 | 1.00 | | | Easton et al., Nature 447:1087-1093 (2007) | TAG | 47% | 45% |
| BC | LSP1 | 1.00 | | | Easton et al., Nature 447:1087-1093 (2007) | DIRECT | 8% | 52% |
| BC | CASP8 | 1.00 | | | Cox et al., Nat. Genet 39:352-358 (2007) | TAG | 77% | 22% |
| BCERP | chr2.217614077 | 1.00 | | | Stacey et al., Nat. Genet. 39:865-869 (2007) | TAG | 40% | 45% |
| BCERP | TNRC9 | 1.00 | | | Stacey et al., Nat. Genet. 39:865-869 (2007) | DIRECT | 12% | 37% |
| BMIOB | FTO | 1.00 | | | Frayling et al., Science 316:889-894 (2007) | DIRECT | 12% | 67% |
| BMIOW | FTO | 1.00 | | | Frayling et al., Science 316:889-894 (2007) | DIRECT | 12% | 67% |
| BP | PALB2 | 1.00 | | | Wellcome Trust Case Control Consortium (WTCCC) Nature 447:661-678 (2007) | DIRECT | 67% | 27% |
| CD | chr10.101277754 | 1.00 | | | Wellcome Trust Case Control Consortium (WTCCC) Nature 447:661-678 (2007) | DIRECT | 30% | 40% |
| CD | PTGER4 | 1.00 | | | Wellcome Trust Case Control Consortium (WTCCC) Nature 447:661-678 (2007) | DIRECT | 7% | 20% |
| CD | ATG16L1 | 1.00 | | | Wellcome Trust Case Control Consortium (WTCCC) Nature 447:661-678 (2007) | DIRECT | 30% | 50% |
| CD | BSN | 1.00 | | | Wellcome Trust Case Control Consortium (WTCCC) Nature 447:661-678 (2007) | DIRECT | 3% | 42% |
| CD | IL23R | 1.00 | | | Wellcome Trust Case Control Consortium (WTCCC) Nature 447:661-678 (2007) | DIRECT | 10% | 38% |

FIG. 4E

| Short Phenotype Name | Gene (or chr.loc on B36) | Genotypic risk: nonrisk homoz (NN vs NN) | Carrier Risk (RR or RN - vs NN) | Allelic Risk (R vs N) | Seminal publication | DIRECT or TAG SNP | Test SNP RR freq in HapMap CEU | Test SNP RN freq In HapMap CEU |
|---|---|---|---|---|---|---|---|---|
| CD | IRGM | 1.00 | | | Wellcome Trust Case Control Consortium (WTCCC) Nature 447:661-678 (2007) | DIRECT | 0% | 7% |
| CD | NOD2 (CARD15) | 1.00 | | | Wellcome Trust Case Control Consortium (WTCCC) Nature 447:661-678 (2007) | DIRECT | 10% | 52% |
| CD | PTPN2 | 1.00 | | | Wellcome Trust Case Control Consortium (WTCCC) Nature 447:661-678 (2007) | DIRECT | 5% | 28% |
| CD | ZNF365 | 1.00 | | | Wellcome Trust Case Control Consortium (WTCCC) Nature 447:661-678 (2007) | DIRECT | 23% | 63% |
| CelD | IL2-IL22 locus | | | 1.42 (1.28-1.59) | van Heel et al., Nat. Genet. 39:827-829 (2007) | DIRECT | 55% | 42% |
| CelD | HLA-DQ2.5cis | | | 7.04 (6.08-8.15) | van Heel Nat Genet 39:827 (2007) | DIRECT | 0% | 18% |
| CelD | CTLA4 | | | 1.24 (1.04-1.49) | Hunt et al., Eur. J. Hum. Genet. 13:440-444 (2005) | TAG | 8% | 58% |
| EMI | THBS4 | 1.00 | | | Topol et al., Circulation 104:2641-6544 (2001) (main reference) | DIRECT | 53% | 37% |
| EMI | 9b21 | 1.00 | | | Helgadottir et al., Science 316:1491-1493 (2007): subset from McPherson et al., Science 316:1488-1491 (2007) | TAG | 22% | 55% |
| MI | 9b21 | 1.00 | | | Helgadottir et al., Science 316:1491-1493 (2007): subset from McPherson et al., Science 316:1488-1491 (2007) | TAG | 22% | 55% |
| OAK | GDF5 | 1.00 | | | Miyamoto et al., Nat. Genet. 39:529-533 (2007) | TAG | 42% | 47% |
| PC | 8q24_R1 | 1.00 | | | Yeager et., Nat. Genet. 39:64-649 (2007) | TAG | 0% | 13% |
| PC | 8q24_R3 | 1.00 | | | Yeager et., Nat. Genet. 39:64-649 (2007) | DIRECT | 18% | 55% |
| RA | PTPN22 | 1.00 | | | Wellcome Trust Case Control Consortium (WTCCC) Nature 447:661-678 (2007) | DIRECT | 0% | 28% |
| RA | MHC | 1.00 | | | Wellcome Trust Case Control Consortium (WTCCC) Nature 447:661-678 (2007) | DIRECT | 22% | 57% |
| T2D | CDKAL1 | 1.00 | | | Scott et al., Science 316:1341-1345 (2007); Zeggini Science 316:1336-1341 (2007) | DIRECT | 8% | 45% |

FIG. 4F

| Short Phenotype Name | Gene (or chr.loc on B36) | Genotypic risk: nonrisk homoz (NN vs NN) | Carrier Risk (RR or RN - vs NN) | Allelic Risk (R vs N) | Seminal publication | DIRECT or TAG SNP | Test SNP RR freq in HapMap CEU | Test SNP RN freq In HapMap CEU |
|---|---|---|---|---|---|---|---|---|
| T2D | TCF7L2 | 1.00 | | | Wellcome Trust Case Control Consortium (WTCCC) Nature 447:661-678 (2007) | DIRECT | 9% | 37% |
| T2D | CDKAL1 | 1.00 | | | Steinthorsdottir et al., Nat. Genet. 39:770-775 (2007). | DIRECT | 8% | 33% |
| T2D | CDKAL1 | 1.00 | | | Steinthorsdottir et al., Nat. Genet. 39:770-775 (2007). | DIRECT | 8% | 33% |
| T2D | CDKN2A/B | 1.00 | | | Scott et al., Science 316:1341-1345 (2007); Zeggini et al., Science 316:1336-1341 (2007) | DIRECT | 67% | 25% |
| T2D | Chr11.41871942 | 1.00 | | | Scott et al., Science 316:1341-1345 (2007); | TAG | 78% | 22% |
| T2D | FTO | 1.00 | | | Scott et al., Science 316:1341-1345 (2007); | DIRECT | 12% | 67% |
| T2D | HHEX | 1.00 | | | Scott et al., Science 316:1341-1345 (2007); | DIRECT | 32% | 48% |
| T2D | IGF2BP2 | 1.00 | | | Scott et al., Science 316:1341-1345 (2007); | DIRECT | 12% | 35% |
| T2D | KCNJ11 | 1.00 | | | Scott et al., Science 316:1341-1345 (2007); | TAG | 10% | 62% |
| T2D | PPARG | 1.00 | | | Scott et al., Science 316:1341-1345 (2007); | DIRECT | 87% | 12% |
| AMD | GRK5 | | 1.59 | | Jakobsdottir et al., Am. J. Hum. Genet. 77:389-407 (2005) | TAG | 27% | 50% |
| AMD | LOC387715 | 1.00 | | | Maller et al., Nat. Genet. 38:1055-1059 (2006) | DIRECT | 2.00% | 40% |
| AMD | CFH | 1.00 | | | Maller et al., Nat. Genet. 38:1055-1059 (2006) | DIRECT | 22% | 38% |
| AMD | CFB-C2 | 1.00 | | | Maller et al., Nat. Genet. 38:1055-1059 (2006) | TAG | 0% | 12% |

FIG. 4G

| Short Phenotype Name | Gene (or chr.loc on B36) | Test SNP-NN freq in Hap Map CEU | Test SNP-RR freq in Hap Map CHB | Test SNP-RN freq in Hap Map CHB | Test SNP-NN freq in Hap Map CHB | Test SNP RR freq in Hap Map YRI | Test SNP RN freq in Hap Map YRI | Test SNP NN freq in Hap Map YRI | Test SNP RR freq in Hap Map JAP | Test SNP RN freq in Hap Map JAP | Test SNP NN freq in Hap Map JAP |
|---|---|---|---|---|---|---|---|---|---|---|---|
| AD | APOE | 67% | 0% | 24% | 76% | 2% | 27% | 72% | 2% | 13% | 84% |
| BC | FGFR2 | 38% | 9% | 42% | 49% | 28% | 47% | 25% | 4% | 31% | 64% |
| BC | TNRC9 | 52% | 49% | 44% | 7% | 22% | 63% | 15% | 33% | 44% | 22% |
| BC | MAP3K1 | 8% | 27% | 42% | 31% | 53% | 42% | 5% | 13% | 64% | 22% |
| BC | LSP1 | 40% | 2% | 16% | 82% | 0% | 25% | 75% | 0% | 29% | 71% |
| BC | CASP8 | 2% | 100% | 0% | 0% | 2% | 37% | 62% | 100% | 0% | 0% |
| BCERP | chr2.217614077 | 15% | 0% | 27% | 73% | 67% | 27% | 7% | 4% | 13% | 82% |
| BCERP | TNRC9 | 52% | 49% | 44% | 7% | 75% | 25% | 0% | 33% | 44% | 22% |
| BMIOB | FTO | 22% | 0% | 24% | 76% | 25% | 53% | 22% | 7% | 20% | 73% |
| BMIOW | FTO | 22% | 0% | 24% | 76% | 25% | 53% | 22% | 7% | 20% | 73% |
| BP | PALB2 | 7% | 40% | 44% | 16% | 5% | 42% | 53% | 42% | 49% | 9% |
| CD | chr10.101277754 | 40% | 31% | 47% | 22% | 25% | 48% | 27% | 27% | 47% | 27% |
| CD | PTGER4 | 73% | 0% | 0% | 100% | 0% | 3% | 97% | 0% | 0% | 100% |
| CD | ATG16L1 | 20% | 16% | 47% | 38% | 8% | 38% | 53% | 0% | 33% | 67% |
| CD | BSN | 55% | 0% | 9% | 91% | | | | 0% | 16% | 84% |
| CD | IL23R | 52% | 29% | 49% | 22% | 5% | 47% | 48% | 36% | 49% | 16% |
| CD | IRGM | 93% | 13% | 49% | 38% | 7% | 43% | 50% | 21% | 39% | 41% |
| CD | NOD2 (CARD15) | 38% | 0% | 0% | 100% | 0% | 0% | 100% | 0% | 0% | 100% |
| CD | PTPN2 | 67% | 2% | 33% | 64% | 28% | 47% | 25% | 0% | 16% | 84% |
| CD | ZNF365 | 13% | 71% | 24% | 4% | 0% | 3% | 97% | 49% | 44% | 7% |
| CelD | IL2-IL22 locus | 3% | 84% | 16% | 0% | 82% | 16% | 2% | 77% | 21% | 2% |
| CelD | HLA-DQ2.5cis | 82% | 0% | 13% | 87% | 0% | 5% | 95% | 0% | 17% | 83% |

FIG. 4H

| Short Phenotype Name | Gene (or chr.loc on B36) | Test SNP-NN freq in Hap Map CEU | Test SNP-RR freq in Hap Map CHB | Test SNP-RN freq in Hap Map CHB | Test SNP-NN freq in Hap Map CHB | Test SNP RR freq in Hap Map YRI | Test SNP RN freq in Hap Map YRI | Test SNP NN freq in Hap Map YRI | Test SNP RR freq in Hap Map JAP | Test SNP RN freq in Hap Map JAP | Test SNP NN freq in Hap Map JAP |
|---|---|---|---|---|---|---|---|---|---|---|---|
| CelD | CTLA4 | 33% | 47% | 42% | 11% | 40% | 49% | 11% | 27% | 50% | 23% |
| EMI | THBS4 | 10% | 96% | 4% | 0% | 85% | 15% | 0% | 84% | 16% | 0% |
| EMI | 9p21 | 23% | 22% | 51% | 27% | 2% | 32% | 67% | 27% | 48% | 25% |
| MI | 9p21 | 23% | 22% | 51% | 27% | 2% | 32% | 67% | 27% | 48% | 25% |
| OAK | GDF5 | 12% | 58% | 36% | 7% | 17% | 60% | 23% | 51% | 44% | 4% |
| PC | 8q24_R1 | 87% | 2% | 13% | 84% | 3% | 22% | 75% | 4% | 31% | 64% |
| PC | 8q24_R3 | 27% | 11% | 56% | 33% | 97% | 3% | 0% | 11% | 46% | 43% |
| RA | PTPN22 | 72% | 0% | 0% | 100% | 18% | 43% | 38% | 0% | 0% | 100% |
| RA | MHC | 22% | 31% | 42% | 27% | 0% | 0% | 100% | 30% | 49% | 21% |
| T2D | CDKAL1 | 47% | 16% | 51% | 33%% | 48% | 37% | 15% | 16% | 44% | 40% |
| T2D | TCF7L2 | 54% | 0% | 4% | 96% | 22% | 61% | 17% | 0% | 4% | 96% |
| T2D | CDKAL1 | 58% | 22% | 49% | 29% | 42% | 43% | 15% | 23% | 48% | 30% |
| T2D | CDKAL1 | 58% | 22% | 49% | 29% | 42% | 43% | 15% | 23% | 48% | 30% |
| T2D | CDKN2A/B | 8% | 43% | 33% | 24% | 100% | 0% | 0% | 29% | 56% | 16% |
| T2D | Chr11.41871942 | 0% | 60% | 33% | 7% | 70% | 28% | 2% | 47% | 51% | 2% |
| T2D | FTO | 22% | 0% | 24% | 76% | 23% | 47% | 30% | 7% | 20% | 73% |
| T2D | HHEX | 20% | 7% | 51% | 42% | 75% | 22% | 3% | 21% | 41% | 39% |
| T2D | IGF2BP2 | 53% | 4% | 36% | 60% | 30% | 50% | 20% | 13% | 36% | 51% |
| T2D | KCNJ11 | 28% | 11% | 49% | 40% | 0% | 2% | 98% | 9% | 47% | 44% |
| T2D | PPARG | 2% | 96% | 4% | 0% | 100% | 0% | 0% | 89% | 11% | 0% |
| AMD | GRK5 | 23% | 76% | 20% | 4% | 82% | 18% | 0% | 66% | 21% | 13% |
| AMD | LOC387715 | 58% | 16% | 62% | 22% | 8% | 45% | 47% | 20% | 47% | 33% |
| AMD | CFH | 40% | 22% | 42% | 36% | 32% | 52% | 17% | 13% | 53% | 33% |
| AMD | CFB-C2 | 88% | n/a | n/a | n/a | 2% | 17% | 81% | n/a | n/a | n/a |

FIG. 4I

| Short Phenotype Name | Locus | Gene (or chr.loc on B36) | Gender Applicability (F-M-B) | TEST SNP | CHR | Test Risk allele (plus, R) | Test Non Risk allele (plus, N) | Ethnicity/ Race distr | Functional or published SNP | UNITS for effect estimate |
|---|---|---|---|---|---|---|---|---|---|---|
| PC | PC_1 \|PC_2 | 8q24_R1\|8q24_R3 | M | rs964322 6\|rs6983 267 | chr8 | C\|G | G\|T | CEU | rs1447295\|r s6983267 | OR (95% CI) |

| Gene or chr.loc on B36 | UNITS for effect estimate | 2locus_ genotypic effect RRRR | 2locus_ genotypic effect RRRN | 2locus_ genotypic effect RRNN | 2locus_ genotypic effect RNRR | 2locus_ genotypic effect RNRN | 2locus_ genotypic effect RNNN | Gene (or chr.loc on B36) |
|---|---|---|---|---|---|---|---|---|
| 8q24_R1\|8q2 4_R3 | OR (95% CI) | 3.17 (2.55, 3.94) | 2.55 (2.10, 3.09) | 2.05 (1.70, 2.46) | 2.22 (1.91, 2.57) | 1.78 (1.60, 1.98) | 1.43 (1.30, 1.57) | 8q24_R1\|8q24 _R3 |

| 2locus genotypic affect NNRR | 2locus genotypic affect NNRN | 2locus genotypic affect NNNN | Seminal publication |
|---|---|---|---|
| 1.55 (1.37, 1.75) | 1.24 (1.17, 1.32) | 1 | Yeager et, Nat. Genet. 39:64-649 (2007) |

FIG. 4J

| Short Phenotype Name | Locus | LOCUS_ETH | Gene (or chr.loc on B36) | Gender applicability (F, M, B) | TEST SNP | Test Risk allele (plus R) | Test NonRisk allele (plus N) | Ethnicity/Race-distr | Functional or published SNP |
|---|---|---|---|---|---|---|---|---|---|
| AMD | AMD_2_3_4 | AMD_2_3_4 _CEU | LOC387715\|CFH\|CFB-C2 | B | rs10490924\|rs10737680\|rs541862 | T\|C\|A | G\|A\|G | CEU | rs10490924\|rs10737680\|rs641153 |

| CHR | UNITS for effect estimate | Effect $R_1R_1R_2R_2R_3R_3$ | Effect $R_1R_1R_2R_2R_3N_3$ | Effect $R_1R_1R_2R_2N_3N_3$ | Effect $R_1R_1R_2N_2R_3R_3$ | Effect $R_1R_1R_2N_2R_3N_3$ | Effect $R_1R_1R_2N_2N_3N_3$ | Effect $R_1R_1N_2N_2R_3R_3$ | Effect $R_1N_1R_2R_2R_3R_3$ |
|---|---|---|---|---|---|---|---|---|---|
| chr10\|chr1\|chrB | OR (n/a CI) | 285 | 154 | 154 | 92.5 | 49.8 | 49.8 | 30 | 70.8 |

| Effect $R_1R_1N_2N_2R_3N_3$ | Effect $R_1R_1N_2N_2N_3N_3$ | Effect $R_1N_1R_2R_2R_3N_3$ | Effect $R_1N_1R_2R_2N_3N_3$ | Effect $R_1N_1R_2N_2R_3R_3$ | Effect $R_1N_1R_2N_2R_3N_3$ | Effect $R_1N_1R_2N_2N_3N_3$ | Effect $R_1N_1N_2N_2R_3R_3$ | Effect $N_1N_1R_2R_2N_3N_3$ | Effect $R_1N_1N_2N_2R_3N_3$ |
|---|---|---|---|---|---|---|---|---|---|
| 16.2 | 16.2 | 38.1 | 38.1 | 23 | 12.4 | 12.4 | 7.5 | 9.5 | 4 |

| Effect $R_1N_1N_2N_2N_3N_3$ | Effect $N_1N_1R_2R_2R_3R_3$ | Effect $N_1N_1R_2R_2R_3N_3$ | Effect $N_1N_1R_2N_2R_3R_3$ | Effect $N_1N_1R_2N_2R_3N_3$ | Effect $N_1N_1R_2N_2N_3N_3$ | Effect $N_1N_1N_2N_2R_3N_3$ | Effect $N_1N_1N_2N_2R_3N_3$ | Effect $N_1N_1N_2N_2N_3N_3$ | Seminal publication |
|---|---|---|---|---|---|---|---|---|---|
| 4 | 17.6 | 9.5 | 5.7 | 3.1 | 3.1 | 1.9 | 1.9 | 1 | Maller et al., Nat. Genet. 38:1055-1059 (2006) |

FIG. 4K

| Abbreviation | What Does it stand for? |
|---|---|
| CEU | European/Caucasian ethnicity |
| CHB | Chinese ethnicity |
| JAP | Japanese ethnicity |
| YRI | Yoruban ethnicity |
| R | risk allele |
| N | non-risk allele |
| CC | case control study design |
| | |
| | |
| **Ethnicity** | |
| C(H) | Han Chinese ethnicity |
| E | European |
| J | Japanese |
| L | Latine |
| NA-P | Native American-Pima Indians |
| H | Hawaiian |
| Af | African |
| As | Asian |
| | |
| **Countries** | |
| CH | Switzerland |
| Dk | Denmark |
| FI | Finland |
| GH | Ghana |
| IS | Iceland |
| IT | italy |
| KR | Korea |
| NG | Nigeria |
| NL | Netherlands |
| GB | United Kingdom |
| FR | France |
| ES | Spain |
| SE | Sweden |
| TH | Thailand |
| TW | Taiwan |
| US | United States |

FIG. 4L

| Short Phenotype Name | Phenotype | Heritability | Reference |
|---|---|---|---|
| AMD | Age Related Macular Degeneration | 0.71[1] | Haddad et al., Survey of Opthalmology, 51:316 -363 (2006) |
| AD | Alzheimer's Disease | 0.79[2] | Gatz et al., Arch of Gen. Psychiatry. 63:168-174 (2006) |
| T2D | Diabetes, Type 2 | 0.80 | van Tilburg et al., J. Med. Genet. 38:569-578 (2001) |
| EMEM | Episodic Memory (Short-term) | 0.50 | de Quervain et al., Proc. Natl. Acad. Sci. USA 103:4270-4274 (2006). |
| MI | Myocardial infarction | 0.57 (M), 0.38 (F) | Zdtavkovic. Karolinska Inst. 2006. http://diss.kib.ki.se/2006/91-7140-771-5/ |
| EMI | Myocardial infarction (early onset M<45, F<50) | 0.63 | Roberts and Stewart, Am. Heart Hosp. J. 4:222-227 (2006) |
| EMI | Myocardial infarction (early onset M<50, F<60) | 0.63 | Roberts and Stewart, Am. Heart Hosp. J. 4:222-227 (2006) |
| BMIOB | Body Mass Index, obesity endpoint (BMI 30kg/$m^2$) | 0.40 | Tambs et al., Am. J. Hum. Biol. 3:257-267 (1991) |
| BMIOW | Body mass index, overweight endpoint (BMI>25kg/m2) | 0.40 | Tambs et al., Am. J. Hum. Biol. 3:257-267 (1991) |
| BC | Breast Cancer | 0.30 | Locatelli et al., Twin Res. 7:182-191 (2004). |
| CD | Crohn's Disease (inflammatory bowel disease) | 0.58 | Breslin et al., Gut 41:557-560 (1997) |
| BP | Bipolar Disorder | 0.75 | McGuffin et al., Arch. Gen. Psych. 60:497-502 (2003) |
| BCERP | Breast Caner, ER positive | n/a | |
| OAH | osteoarthritis, hip joint | 0.61 (radiograph vs. self report) | Page et al., Twin Res. 6:147-151 (2003) |
| OAK | osteoarthritis, knee joint | 0.63 (radiograph) | Zhai et al., Osteoarthritis Cartilage 15:222-225 (2007) |
| RA | rheumatoid arthritis | 0.53 | MacGregor et al., Arthritis Rheum. 2000, 43:30-37 (2000). |
| CelD | Celiac Disease | 0.70 | Nistico et al., Gut 55:803-808 (2006) |
| PC | Prostate Cancer | 0.27 | Page et al., Prostate 33:240-245 (1997) |
| | Colorectal cancer | 0.35 | Lichtenstein et al., N. Engl. J. Med. 343:78-85 (2000) |

[1] MC-1-5 grade of maculopathy, her estimate for grades 4 and 5 "advanced disease", AMD
[2] Adjusted for age

FIG. 4M

| Phenotype | Gene | Minor allele | Ethnicity/ Race (1) | Country (2) | Functional or published SNP | Effect estimate in homoz (3) | Effect estimate in heteroz (3) | Effect estimate in carriers (3,4) | Seminal publication |
|---|---|---|---|---|---|---|---|---|---|
| Age Related Macular Degeration | CFH | C | E | US | rs1329428 | NA | NA | 6.2 (2.9, 13)* | Klein et al., Science 308:385-389 (2005) |
| Alzheimer's Disease (conditional on carrying the APOE4 allele) | GAB2 | T | E | US | rs2373115 | | | 4.06 (2.81, 14.69)* multiplied by the APOE4 risk | Reiman et al., Neuron 54:713-720 (2007) |
| Cardiac repolarization (QT interval) | NOS1AP | G | E | US | rs10494366 | | 0.1±0.6** | GG: 2.7±1.0, GT:0.1±0.6, TT:-1.3±0.6** | Arking et al., Nat. Genet. 38:644-651 (2006) |
| Cerebral infarction | PRKCH | A | As | JP, CN | rs2230500 | 1.56 (1.03, 2.37) | 1.28 (1.07, 1.54) | 1.31 (1.11, 1.56) | Kubo et al., Nat. Genet. 39:212-217 (2007). |
| Crohn's Disease (inflammatory bowel disease) | IL23R | A | E | US | rs11209026 | | | 0.26 (0.15, 0.43)* | Duerr et al., Science 314:1461-1463 (2006) |
| Diabetes, Type 1 | CTLA4 | G | E | IT | rs231775 | | | 1.79 (1.20, 2.69)* | Nistico et al., Hum. Molec. Genet. 5:1075-1080 (1996) |

FIG. 5A

| Phenotype | Gene | Minor allele | Ethnicity/ Race (1) | Country (2) | Functional or published SNP | Effect estimate in homoz (3) | Effect estimate in heteroz (3) | Effect estimate in carriers (3,4) | Seminal publication |
|---|---|---|---|---|---|---|---|---|---|
| Diabetes, Type 2 | IGF2BP2 | T | E | US | rs4402960 | | | 1.18 (1.08, 1.28)* | Scott et al., Science 316:1341-1345 (2007) |
| Diabetes, Type 2 | IGF2BP2 | T | | GB | rs4402960 | | | 1.11 (1.05, 1.16)* | Scott et al., Science 316:1341-1345 (2007) |
| Diabetes, Type 2 | KCNJ11 | T | E | US | rs5219 | | | 1.11 (1.02, 1.21)* | Scott et al., Science 316:1341-1345 (2007) |
| Diabetes, Type 2 | KCNJ11 | T | | UK | rs5219 | | | 1.13 (1.07, 1.19)* | Scott et al., Science 316:1341-1345 (2007) |
| Diabetes, Type 2 | SLC30A8 | C | E | US | rs13266634 | | | 1.18 (1.09, 1.29)* | Scott et al., Science 316:1341-1345 (2007) |
| Diabetes, Type 2 | SLC30A8 | C | | UK | rs13266634 | | | 1.12 (1.05, 1.18)* | Scott et al., Science 316:1341-1345 (2007) |
| Diabetes, Type 2 | TCF7L2 | T | E | FR | rs7903146 | 2.77 (2.27, 3.27)* | 1.65 (1.46, 1.84)* | 1.83 (1.64, 2.05)* | Sladek et al., Nature 445:881-885 (2007); Grant et al., Nat. Genet. 38:320-323 (2006) |
| Episodic Memory (short term) | WWC1 (KIBRA) | T | E | CH | rs1070145 | NA | NA | 9.4+0.2 (24%better word recall)*** | Papassotiropoulos et al., Science 314:475-478 (2006) |
| Prostate cancer | 8q24 region 1 | A | E | US | rs1447295 | 1.42 (1.29, 1.59)* | 2.23 (1.58, 3.14)* | | Yeager et al., Nat Genet. 39:645-9064 (2007); meta-analysis Witte et al., Nat. Genet. 39:579-580 (2007) |

FIG. 5B

| Phenotype | Gene | Minor allele | Ethnicity/ Race (1) | Country (2) | Functional or published SNP | Effect estimate in homoz (3) | Effect estimate in heteroz (3) | Effect estimate in carriers (3,4) | Seminal publication |
|---|---|---|---|---|---|---|---|---|---|
| Prostate cancer | 8q24 region 1 | A | | IS | rs1447295 | | | 1.71 (1.49, 1.95)* | Gudmundsson et al., Nat. Genet. 39:631-637 (2007); meta-analysis Witte et al., Nat. Genet. 39:579-580 (2007) |
| Prostate cancer | 8q24 region 1 | A | | ES | rs1447295 | | | 1.44 (1.07,1.94)* | Gudmundsson et al., Nat. Genet. 39:631-637 (2007); meta-analysis Witte et al., Nat. Genet. 39:579-580 (2007) |
| Prostate cancer | 8q24 region 1 | A | | NL | rs1447295 | | | 1.39 (1.09,1.78)* | Gudmundsson et al., Nat. Genet. 39:631-637 (2007); meta-analysis Witte et al., Nat. Genet. 39:579-580 (2007) |
| Prostate cancer | 8q24 region 1 | A | A | US | rs1447295 | | | 1.25 (1.06.1.49)* | Haiman et al., Nat. Genet. 39:638-644 (2007); meta-analysis Witte et al., Nat. Genet. 39:579-580 (2007) |
| Prostate cancer | 8q24 region 1 | A | J | US | rs1447295 | | | 1.49 (1.23,1.81)* | Haiman et al., Nat. Genet. 39:638-644 (2007); meta-analysis Witte et al., Nat. Genet. 39:579-580 (2007) |
| Prostate cancer | 8q24 region 1 | A | H | US | rs1447295 | | | 2.55 (1.33,4.89)* | Haiman et al., Nat. Genet. 39:638-644 (2007); meta-analysis Witte et al., Nat. Genet. 39:579-580 (2007) |

**FIG. 5C**

| Phenotype | Gene | Minor allele | Ethnicity/ Race (1) | Country (2) | Functional or published SNP | Effect estimate in homoz (3) | Effect estimate in heteroz (3) | Effect estimate in carriers (3,4) | Seminal publication |
|---|---|---|---|---|---|---|---|---|---|
| Prostate cancer | 8q24 region 1 | A | L | US | rs1447295 | | | 1.98 (1.49,2.61)* | Haiman et al., Nat. Genet. 39:638-644 (2007); meta-analysis Witte et al., Nat. Genet. 39:579-580 (2007) |
| Prostate cancer | 8q24 region 2/HapC | A | E | US | rs16901979 | | | 1.44 (1.21, 1.70)* | Gudmundsson et al., Nat. Genet. 39:631-637 (2007); meta-analysis Witte et al., Nat. Genet. 39:579-580 (2007) |
| Prostate cancer | 8q24 region 2/HapC | A | E | IS | rs16901979 | | | 2.08 (1.66, 2.60)* | Gudmundsson et al., Nat. Genet. 39:631-637 (2007); meta-analysis Witte et al., Nat. Genet. 39:579-580 (2007) |
| Prostate cancer | 8q24 region 2/HapC | A | E | ES | rs16901979 | | | 2.13 (1.34, 3.40)* | Gudmundsson et al., Nat. Genet. 39:631-637 (2007); meta-analysis Witte et al., Nat. Genet. 39:579-580 (2007) |
| Prostate cancer | 8q24 region 2/HapC | A | E | NL | rs16901979 | | | 1.85 (1.05, 3.27)* | Gudmundsson et al., Nat. Genet. 39:631-637 (2007); meta-analysis Witte et al., Nat. Genet. 39:579-580 (2007) |
| Prostate cancer | 8q24 region 2/HapC | A | A | US | rs16901979 | | | 1.34 (1.18, 1.53)* | Haiman et al., Nat. Genet. 39:638-644 (2007); meta-analysis Witte et al., Nat. Genet. 39:579-580 (2007) |

FIG. 5D

| Phenotype | Gene | Minor allele | Ethnicity/ Race (1) | Country (2) | Functional or published SNP | Effect estimate in homoz (3) | Effect estimate in heteroz (3) | Effect estimate in carriers (3,4) | Seminal publication |
|---|---|---|---|---|---|---|---|---|---|
| Prostate cancer | 8q24 region 2/HapC | A | J | US | rs16901979 | | | 1.78 (1.47, 2.15)* | Haiman et al., Nat. Genet. 39:638-644 (2007); meta-analysis Witte et al., Nat. Genet. 39:579-580 (2007) |
| Prostate cancer | 8q24 region 2/HapC | A | H | US | rs16901979 | | | 3.17 (1.87, 5.36)* | Haiman et al., Nat. Genet. 39:638-644 (2007); meta-analysis Witte et al., Nat. Genet. 39:579-580 (2007) |
| Prostate cancer | 8q24 region 2/HapC | A | L | US | rs16901979 | | | 1.99 (1.34, 2.96)* | Haiman et al., Nat. Genet. 39:638-644 (2007); meta-analysis Witte et al., Nat. Genet. 39:579-580 (2007) |
| Prostate cancer | 8q24 region 3 | G | E | US | rs6983267 | 1.26 (1.13, 1. 41)* | 1.58 (1.40, 1.78) * | | Yeager et al., Nat Genet. 39:645-9064 (2007); meta-analysis Witte et al., Nat. Genet. 39:579-580 (2007) |
| Prostate cancer | 8q24 region 3 | G | A | US | rs6983267 | | | 1.33 (1.17, 1.75)* | Haiman et al., Nat. Genet. 39:638-644 (2007); meta-analysis Witte et al., Nat. Genet. 39:579-580 (2007) |
| Prostate cancer | 8q24 region 3 | G | J | US | rs6983267 | | | 1.23 (1.04, 2.46)* | Haiman et al., Nat. Genet. 39:638-644 (2007); meta-analysis Witte et al., Nat. Genet. 39:579-580 (2007) |
| Prostate cancer | 8q24 region 3 | G | H | US | rs6983267 | | | 1.38 (0.89, 2.14)* | Haiman et al., Nat. Genet. 39:638-644 (2007); meta-analysis Witte et al., Nat. Genet. 39:579-580 (2007) |

FIG. 5E

| Phenotype | Gene | Minor allele | Ethnicity/ Race (1) | Country (2) | Functional or published SNP | Effect estimate in homoz (3) | Effect estimate in heteroz (3) | Effect estimate in carriers (3,4) | Seminal publication |
|---|---|---|---|---|---|---|---|---|---|
| Prostate cancer | 8q24 region 3 | G | L | US | rs6983267 | | | 1.29 (1.07, 1.56)* | Haiman et al., Nat. Genet. 39:638-644 (2007); meta-analysis Witte et al., Nat. Genet. 39:579-580 (2007) |
| Rheumatoid arthritis | PTPN22 | T | E | US | rs2476601 | 2.26 (0.56, 9.14)* | 1.69 (1.23, 2.32)* | 1.71 (1.25, 2.34)* | Begovich et al., Am. J. Hum. Genet. 75:330-337 (2004) |

FIG. 5F

| Phenotype | Gene | Direct or Tag SNP | Covered by | r2 (single or multi) | Risk Haplo/Diplotypes (Tag GT = Risk GT) | Other allele | Remarks |
|---|---|---|---|---|---|---|---|
| Age Related Macular Degeration | CFH | Tag | rs10737680 | 1 | AA=CC; AC=CT; CC=TT | T | 3.68% PP; 30%PP of >75 yo; 0.07% PI; other possible identifiers: haplotype Y402H, rs 1061170 |
| Alzheimer's Disease (conditional on carrying the APOE4 allele) | GAB2 | Direct | | | | G | |
| Cardiac repolarization (QT interval) | NOS1AP | Tag | rs12733821 | 1 | GG=GG; GC=GT; CC=TT | T | |
| Cerebral infarction | PRKCH | Tag | | | | | |
| Crohn's Disease (inflammatory bowel disease) | IL23R | Direct | | | | G | 0.18% population prevalence |
| Diabetes, Type 1 | CTLA4 | Direct | | | | A | 0.12% PP(5); 0.01% PI(6) |
| Diabetes, Type 2 | IGF2BP2 | Direct | | | | G | |
| Diabetes, Type 2 | IGF2BP2 | Direct | | | | G | |
| Diabetes, Type 2 | KCNJ11 | | | | | T | |
| Diabetes, Type 2 | KCNJ11 | | | | | T | |

FIG. 5G

| Phenotype | Gene | Direct or Tag SNP | Covered by | r2 (single or multi) | Risk Haplo/Diplotypes (Tag GT = Risk GT) | Other allele | Remarks |
|---|---|---|---|---|---|---|---|
| Diabetes, Type 2 | SLC30A8 | | | | | C | |
| Diabetes, Type 2 | SLC30A8 | | | | | C | |
| Diabetes, Type 2 | TCF7L2 | Tag | rs4506565 | 0.92 | TT=TT (9.1%); TA=TC (35.2%); AA=CC (52.3%);// TA=CC (2.3%); TT=CT (1.1%) | C | (ii); 5.88%PP; 0.29%PI; other possible identifiers: haplotype DG10S478, rs12255372 |
| Episodic Memory (short term) | WWC1 (KIBRA) | Direct | | | | C | >45 avg age of onset |
| Prostate cancer | 8q24 region 1 | Tag | rs9643226 | 1.000 | | C | 9% PAR (7) |
| Prostate cancer | 8q24 region 1 | Tag | rs9643226 | 1.000 | GG=CC; GC=CA; CC=AA | C | |
| Prostate cancer | 8q24 region 1 | Tag | rs9643226 | 1.000 | GG=CC; GC=CA; CC=AA | C | |
| Prostate cancer | 8q24 region 1 | Tag | rs9643226 | 1.000 | GG=CC; GC=CA; CC=AA | C | |
| Prostate cancer | 8q24 region 1 | Tag | rs9643226 | 1.000 | GG=CC; GC=CA; CC=AA | C | |
| Prostate cancer | 8q24 region 1 | Tag | rs9643226 | 1.000 | GG=CC; GC=CA; CC=AA | C | |
| Prostate cancer | 8q24 region 1 | Tag | rs9643226 | 1.000 | GG=CC; GC=CA; CC=AA | C | |
| Prostate cancer | 8q24 region 1 | Tag | rs9643226 | 1.000 | GG=CC; GC=CA; CC=AA | C | |
| Prostate cancer | 8q24 region 2/HapC | Direct | | | | C | |

FIG. 5H

| Phenotype | Gene | Direct or Tag SNP | Covered by | r2 (single or multi) | Risk Haplo/Diplotypes (Tag GT = Risk GT) | Other allele | Remarks |
|---|---|---|---|---|---|---|---|
| Prostate cancer | 8q24 region 2/HapC | Direct | | | | C | |
| Prostate cancer | 8q24 region 2/HapC | Direct | | | | C | |
| Prostate cancer | 8q24 region 2/HapC | Direct | | | | C | |
| Prostate cancer | 8q24 region 2/HapC | Direct | | | | C | |
| Prostate cancer | 8q24 region 2/HapC | Direct | | | | C | |
| Prostate cancer | 8q24 region 2/HapC | Direct | | | | C | |
| Prostate cancer | 8q24 region 2/HapC | Direct | | | | C | |
| Prostate cancer | 8q24 region 3 | Tag | rs10505477 | 0.935 | TT=GG; TC=GT; CC=TT | T | 21% PAR |
| Prostate cancer | 8q24 region 3 | Tag | rs10505477 | 0.935 | TT=GG; TC=GT; CC=TT | T | |
| Prostate cancer | 8q24 region 3 | Tag | rs10505477 | 0.935 | TT=GG; TC=GT; CC=TT | T | |
| Prostate cancer | 8q24 region 3 | Tag | rs10505477 | 0.935 | TT=GG; TC=GT; CC=TT | T | |
| Prostate cancer | 8q24 region 3 | Tag | rs10505477 | 0.935 | TT=GG; TC=GT; CC=TT | T | |

FIG. 5I

| Phenotype | Gene | Direct or Tag SNP | Covered by | r2 (single or multi) | Risk Haplo/Diplotypes (Tag GT = Risk GT) | Other allele | Remarks |
|---|---|---|---|---|---|---|---|
| Rheumatoid arthritis | PTPN22 | Tag | rs6679677 | | AA=TT; AC=TC; CC=CC | C | 1% PP |

Notes:

(1) Ancestry: C(H)=Han Chinese, E=European, J=Japanese, L=Latino, H=Hawaiian, A=African

(2) DK=Denmark, FI=Finland, GH=Ghana, IS=Iceland, IT=Italy, NG=Nigeria, NL=Netherlands, GB=United Kingdom, FR=France, ES=Spain, SE=Sweden, CH=Switzerland, US=United States.

(3) Due to the different study designs, effect estimates are reported as follows:

*Odds ratio (95% confidence interval).

**Difference from mean+/-standard error for each genotype level.

***Mean+/-standard error

****Hazard ratio (95% confidence interval). An overall effect estimate is reported if multiple populations are reported in the cited publication, when available.

Effect estimates adjusted for covariates are reported, when available.

(4) Carriers=Homozygotes + heterozygotes

(5) PP=population prevalence (USA=300M)

(6) PI=population incidence (USA)

(7) PAR=population attributable risk

FIG. 5J

| Phenotype | Gene | Haplotype | Identifying SNPs | Average age of onset | Population Prevalence (USA = 300M people | Population Incidence (USA) | No. copies of at-risk allele (% of population) | Estimated Relative Risk. Increase (het) | Modified age of onset (het) |
|---|---|---|---|---|---|---|---|---|---|
| Alcoholism | ALDH2 | GLU504LYS | rs671 | >30 | 5.55% (16.65M) | | | | |
| Alzheimer's Disease | ApoE | ApoE-e4 | rs11083750 | >65 | 1.47% (4M) will rise to 5% (16M) by 2050: 50% at 85 | | 73% | 0.5-1.0 | 84 |
| Breast Cancer | BRCA2 | N372H | rs144848 | | 0.08% (240k) | 0.08% (240k) | | | |
| Celiac Disease | HLA-DQA1 | 201 | rs4988889(T)+rs2858331(T) | | 0.4% (1.2M) | | | | |
| Colon Cancer | APC | I1307K | rs28933380 | | 0.05% (150k) | 0.035% (106k/yr) | | | |
| coronary heart disease | eNOS | | rs1799983 | | | | | | |
| coronary heart disease | MTHFR | | rs1801133 | | | | | | |
| coronary heart disease | APOB | Ins/Del/Sp1/EcoR1 | | | | | | | |
| Creutzfeld-Jakob | PRNP | M129V | rs1799990 | | 0% (20) | 0% (20/yr) | | | |
| Crohn's Disease (inflammatory bowel disease) | CARD15 | n/a | rs2066845 & rs2066844 | | 0.18% (540k) | | | | |
| Cystic Fibrosis | CFTR | deltaF508 | various | | 0.01% (30k) | 0.009% (2.5k) | | | |

FIG. 6A

| Phenotype | Gene | Haplotype | Identifying SNPs | Average age of onset | Population Prevalence (USA = 300M people | Population Incidence (USA) | No. copies of at-risk allele (% of population) | Estimated Relative Risk. Increase (het) | Modified age of onset (het) |
|---|---|---|---|---|---|---|---|---|---|
| Diabetes Type 1 | HLA-DR | DRB1*0301 | rs2040410 | | 0.12% (360k) | 0.01% (30k/yr) | | | |
| Diabetes Type 1 | HLA-DQ | Multiple Haplotypes (protective/not) | various | | 0.12% (360k) | 0.01% (30k/yr) | | | |
| Diabetes Type 1 | INS | class 1 VNTR | n/a | | 0.12% (360k) | 0.01% (30k/yr) | | | |
| Diabetes Type 1 | CTLA4 | | rs231775 | | 0.12% (360k) | 0.01% (30k/yr) | | | |
| Diabetes Type 1 | PTPN22 | R620W | rs2476601 | | 0.12% (360k) | 0.01% (30k/yr) | | | |
| Diabetes Type 1 | IFIH1 | A946T | rs1990760 | | 0.12% (360k) | 0.01% (30k/yr) | | | |
| Episodic Memory | CAMTA1 | | rs4908449 | | | | | | |
| Lupus | IRF5 | | rs2004640 | | 0.51% (1.53M) | | | | |
| Multiple Sclerosis | HLA-DRB | B801/DRB1/ 0301/DQA1/ 0501 | rs319763© +rs 4639334© | | 1.12% (3.6M) | | | | |
| Multiple Sclerosis | HLA-DQA1 | 102 | rs9268428© +r s6457594(a)+ RS7451962© | | 0.14% (420k) | | | | |
| Multiple Sclerosis | HLA-DRB | drb1 | RS3135388 | | 0.14% (420k) | | | | |

FIG. 6B

| Phenotype | Gene | Haplotype | Identifying SNPs | Average age of onset | Population Prevalence (USA = 300M people | Population Incidence (USA) | No. coples of at-risk allele 1% of population) | Estimation Relative Risk. Increase (het) | Modified age of onset (het) |
|---|---|---|---|---|---|---|---|---|---|
| Osteoporosis | COL1A1 | Sp1 | rs1800012 | | 10.29% (30.87M) | | | | |
| Progressive supra-nuclear palsy | MAPT | H1 | various | 60 | 0.01% (30k) | 0.004% (12k) | | | |
| Protective against Alzheimer's Disease | ApoE | ApoE-e2 | | >65 | 1.47% (4M) will rise to 5% (16M) by 2050; 50% at 85 | | | 1.0-24.0 | |
| Protective against HIV infection | CCR5 | d32 | n/a | >14 | 0.33% (990k) | 0.01% (30k) | | | |
| Psoriasis | HLA-C | 602 | rs887466(G)+ rs4379333© | | 2.02% (6.06M) | | | | |
| Rheumatoid arthritis | HLA-DRB | DRB1 | various | | 1% (3M) | | | | |
| Schizophrenia | DRD3 | SER9GLY | rs6280 | | 0.81% (2.43M) | | | | |
| Systemic Lupus SLE | HLA-DRB1 | 1501 | rs3135388 | | 0.51% (1.53M) | | | | |
| Thrombosis | factor V Leiden | R506Q | rs6025 | >50 | 0.1% (300k) | 0.1% (300k) | 96% | 1 | >50 |

FIG. 6C

| Phenotype | Gene | heterozygous for at-risk allele (% of population) | Estimated relative risk Increase (het) | Modified age of onset (het) | homozygous for at-risk allele (% of population) | Estimated relative risk Increase (homo) | Modified age of onset (homo) | Seminal Publication |
|---|---|---|---|---|---|---|---|---|
| Alcoholism | ALDH2 | | 0.2 | | | | | Yoshida et al., Am. J. Hum. Genet. 35:1107-1116 (1983) |
| Alzheimer's Disease | ApoE | 24% | 3.0-5.0 | 75 | 3% | 24 | 68 | Corder et al., Science 261:921-923 (1993) |
| Breast Cancer | BRCA2 | | 1.3 | | | | | Healey et al., Nat. Genet. 26:362-364 (2000) |
| Celiac Disease | HLA-DQA1 | | 7 | | | | | Greco et al., Gut 50:624-628 (2002) |
| Colon Cancer | APC | | 2 | | | | | Laken et al., Nat. Genet. 17:79-83 (1997) |
| coronary heart disease | eNOS | | | | | | | Casas et al., Circulation 109:1359-1365 (2004) |
| coronary heart disease | MTHFR | | | | | | | |
| coronary heart disease | APOB | | | | | | | |
| Creutzfeld-Jakob | PRNP | | 0.65 | | | | | Doh-ura et al., Biochem. Biophys. Res. Commun. 163:974-979 (1989) |
| Crohn's Disease (inflammatory bowel disease) | CARD15 | | 3-5 | | | | | Hugot et al., Nature 411:559-603 (2001) |
| Cystic Fibrosis | CFTR | | | | | | | |
| Diabetes Type 1 | HLA-DR | | 4-5 | | | | | Dunsworth et al., Clin. Genet. 21:233-236 (1982) |

FIG. 6D

| Phenotype | Gene | heterozygous for at-risk allele (% of population) | Estimated relative risk Increase (het) | Modified age of onset (het) | homozygous for at-risk allele (% of population) | Estimated relative risk Increase (homo) | Modified age of onset (homo) | Seminal Publication |
|---|---|---|---|---|---|---|---|---|
| Diabetes Type 1 | HLA-DQ | | n/a | | | | | Greenbaum et al. J. Clin. Endocr. Metab. 85:1255-1260 (2000) |
| Diabetes Type 1 | INS | | 1.5-2 | | | | | Pugliese et al., Nature 15:293-297 (1997) |
| Diabetes Type 1 | CTLA4 | | 1.4-15 | | | | | Nistico et al., Hum. Molec. Genet. 5:1075-1080 (1996) |
| Diabetes Type 1 | PTPN22 | | 1.7 | | | | | Bottini et al., Nat. Genet. 36:337-338 (2004) |
| Diabetes Type 1 | IFIH1 | | 0.8 | | | | | Smyth et al., Nat. Genet. 38:617-619 (2006) |
| Episodic Memory | CAMTA1 | | | | | | | |
| Lupus | IRF5 | | 1.8 | | | | | Graham et al., Nat. Genet. 38:550-555 (2006) |
| Multiple Sclerosis | HLA-DRB | | 2.5 | | | | | Heward et al., J. Clin. Endocr. Metab. 83:3394-3397 (1998) |
| Multiple Sclerosis | HLA-DQA1 | | 4 | | | | | Fernandez-Arquero et al., Neurology 53:1361-1363 (1999) |
| Multiple Sclerosis | HLA-DRB | | 4 | | | | | Gregersen et al., Nature 443:574-577 (2006) |

FIG. 6E

| Phenotype | Gene | heterozygous for at-risk allele (% of population) | Estimated relative risk Increase (het) | Modified age of onset (het) | homozygous for at-risk allele (% of population) | Estimated relative risk Increase (homo) | Modified age of onset (homo) | Seminal Publication |
|---|---|---|---|---|---|---|---|---|
| Osteoporosis | COL1A1 | | 1.6 | | | | | Grant et al., Nat. Genet. 14:203-205 (1996) |
| Progressive supra-nuclear palsy | MAPT | | 3.5 | | | | | Baker et al., Hum. Molec. Genet. 8:711-715 (1999) |
| Protective against Alzheimer's Disease | ApoE | | 0.6-3 | | 1% | 0.5 | >84 | Farrer et al., JAMA 278:1349-1356 (1997) |
| Protective against HIV infection | CCR5 | | n/a | | | | | Samson et al., Nature 382:722-725 (1996) |
| Psoriasis | HLA-C | | 5 | | | | | Walsh et al., Am. J. Hum. Genet. 73:580-590 (2003) |
| Rheumatoid arthritis | HLA-DRB | | 2-4 | | | | | Michou et al., Arthritis Res. Ther. 8(:R79 (2006) |
| Schizophrenia | DRD3 | | 1.7 | | | | | Crocq et al., J. Med. Genet. 29:858-860 (1992) |
| Systemic Lupus SLE | HLA-DRB1 | | 4.5 | | | | | Green et al., Ann. Hum. Genet. 50:93-96 (1986) |
| Thrombosis | factor V Leiden | 3% | 7 | <50 | <1% | 80 | <50 | Bertina et al., Nature 369:64-67 (1994) |

FIG. 6F

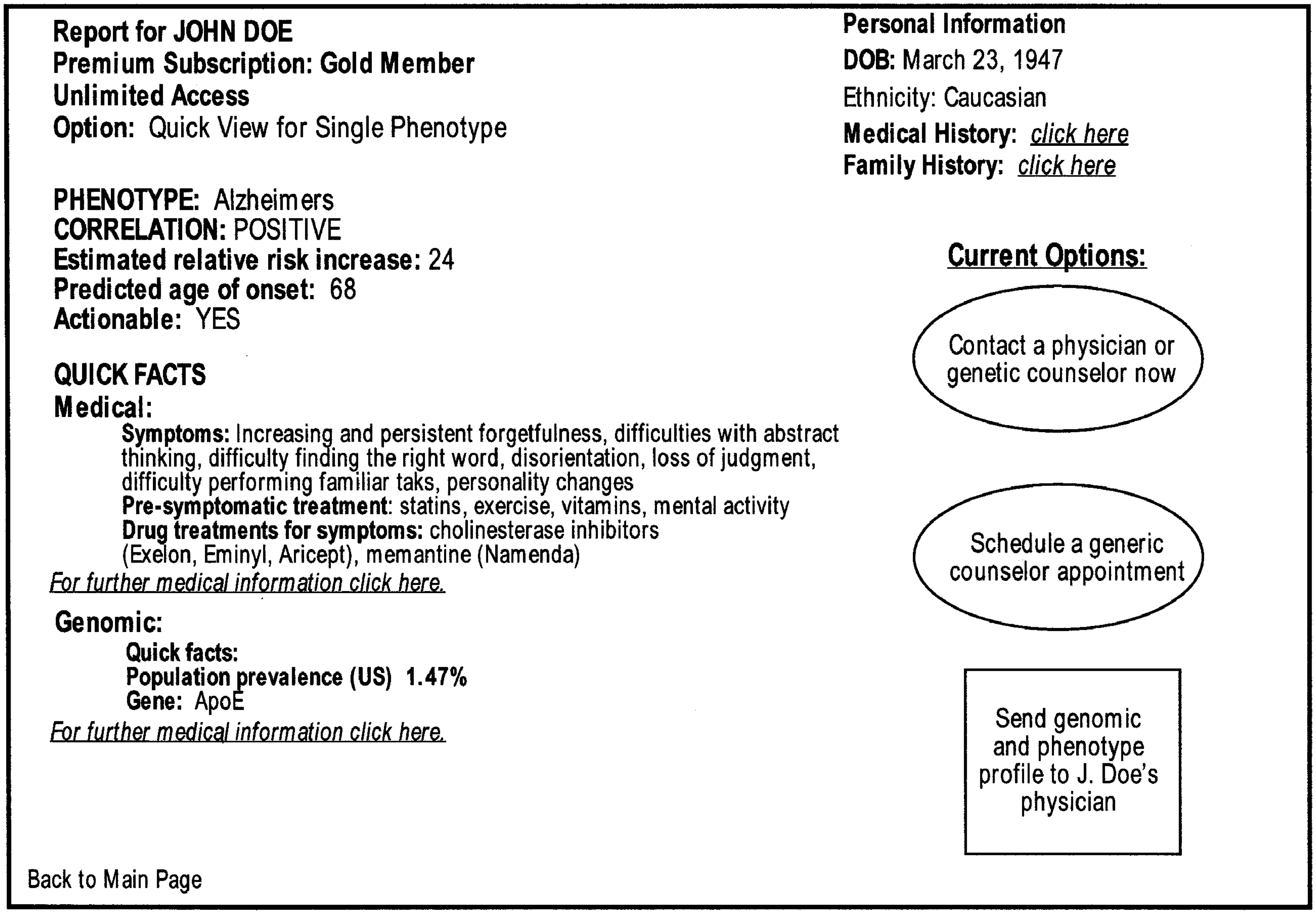

Report for JOHN DOE
Premium Subscription: Gold Member
Unlimited Access
Option: Quick View for Single Phenotype
PHENOTYPE: Alzheimers
CORRELATION: POSITIVE
Estimated relative risk increase: 24
Predicted age of onset: 68
Actionable: YES
QUICK FACTS
Medical:
Symptoms: Increasing and persistent forgetfulness, difficulties with abstract thinking, difficulty finding the right word, disorientation, loss of judgment, difficulty performing familiar taks, personality changes
Pre-symptomatic treatment: statins, exercise, vitamins, mental activity
Drug treatments for symptoms: cholinesterase inhibitors (Exelon, Eminyl, Aricept), memantine (Namenda)
For further medical information click here.
Genomic:
Quick facts:
Population prevalence (US) 1.47%
Gene: ApoE
For further medical information click here.
Back to Main Page
Personal Information
DOB: March 23, 1947
Ethnicity: Caucasian
Medical History: click here
Family History: click here
Current Options:
Contact a physician or genetic counselor now
Schedule a generic counselor appointment
Send genomic and phenotype profile to J. Doe's physician


FIG. 7

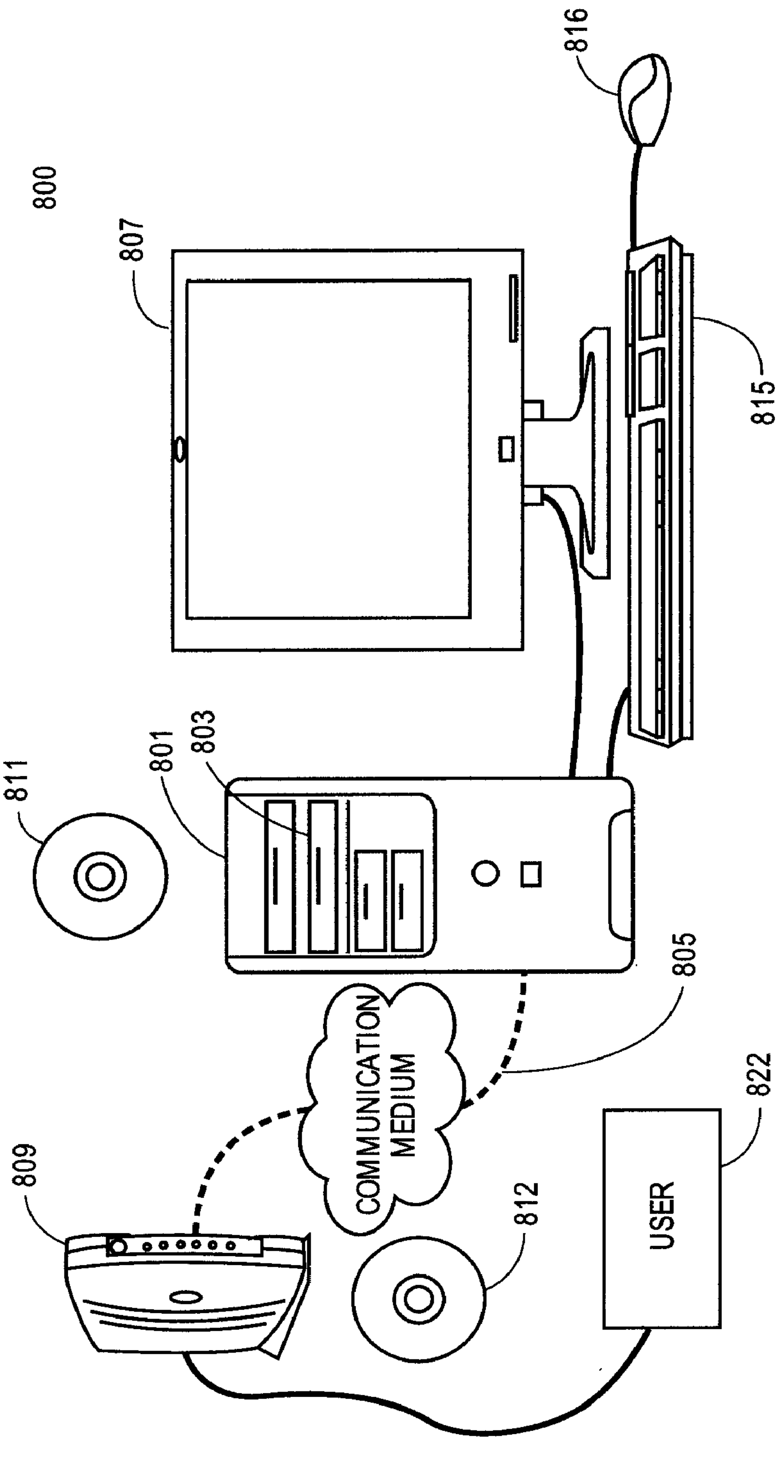
800
807
816
815
811
801
803
805
COMMUNICATION MEDIUM
809
812
822
USER

FIG. 8

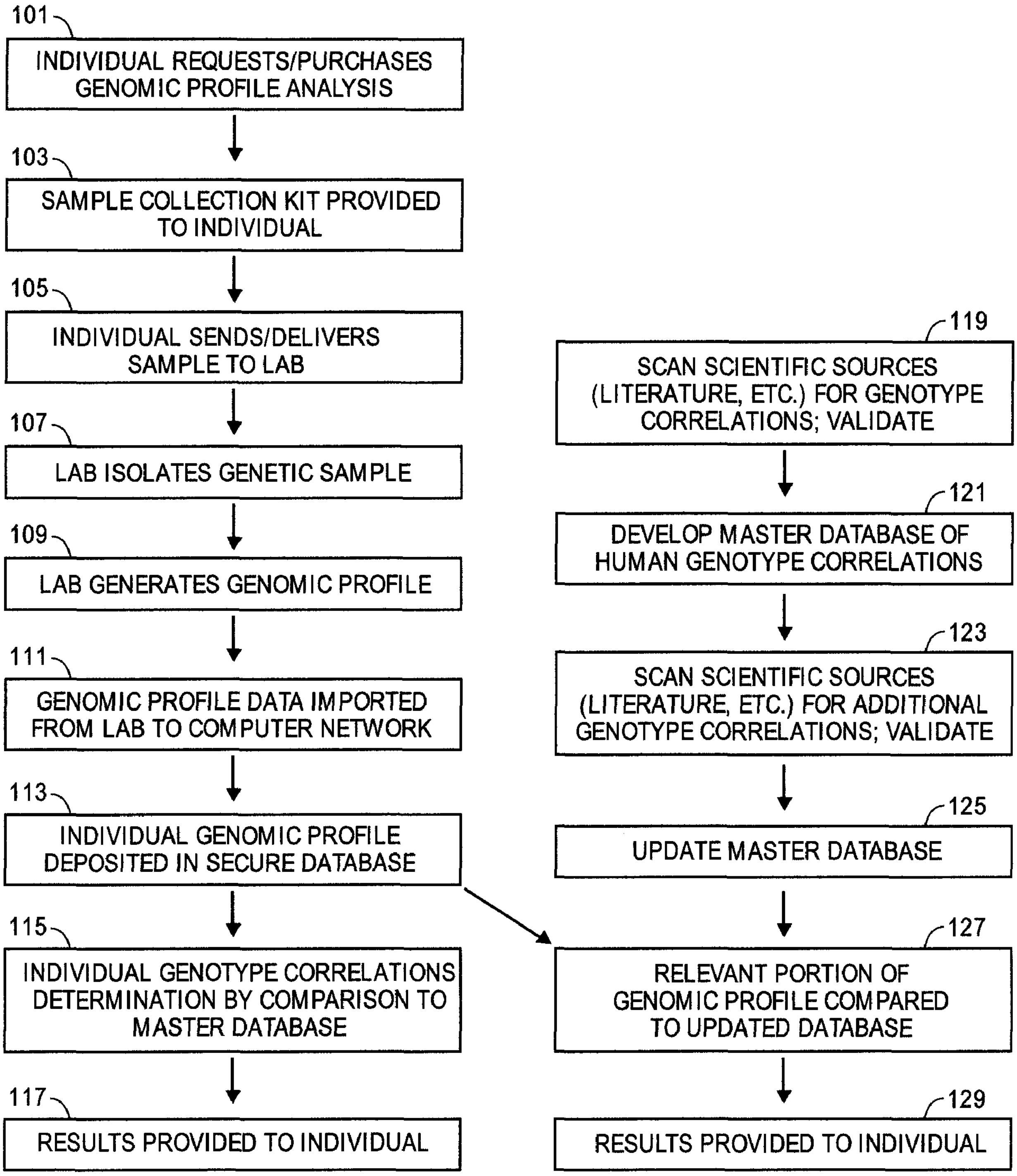
101
INDIVIDUAL REQUESTS/PURCHASES GENOMIC PROFILE ANALYSIS
103
SAMPLE COLLECTION KIT PROVIDED TO INDIVIDUAL
105
INDIVIDUAL SENDS/DELIVERS SAMPLE TO LAB
107
LAB ISOLATES GENETIC SAMPLE
109
LAB GENERATES GENOMIC PROFILE
111
GENOMIC PROFILE DATA IMPORTED FROM LAB TO COMPUTER NETWORK
113
INDIVIDUAL GENOMIC PROFILE DEPOSITED IN SECURE DATABASE
115
INDIVIDUAL GENOTYPE CORRELATIONS DETERMINATION BY COMPARISON TO MASTER DATABASE
117
RESULTS PROVIDED TO INDIVIDUAL
119
SCAN SCIENTIFIC SOURCES (LITERATURE, ETC.) FOR GENOTYPE CORRELATIONS; VALIDATE
121
DEVELOP MASTER DATABASE OF HUMAN GENOTYPE CORRELATIONS
123
SCAN SCIENTIFIC SOURCES (LITERATURE, ETC.) FOR ADDITIONAL GENOTYPE CORRELATIONS; VALIDATE
125
UPDATE MASTER DATABASE
127
RELEVANT PORTION OF GENOMIC PROFILE COMPARED TO UPDATED DATABASE
129
RESULTS PROVIDED TO INDIVIDUAL

FIG. 9

FIGURE 10

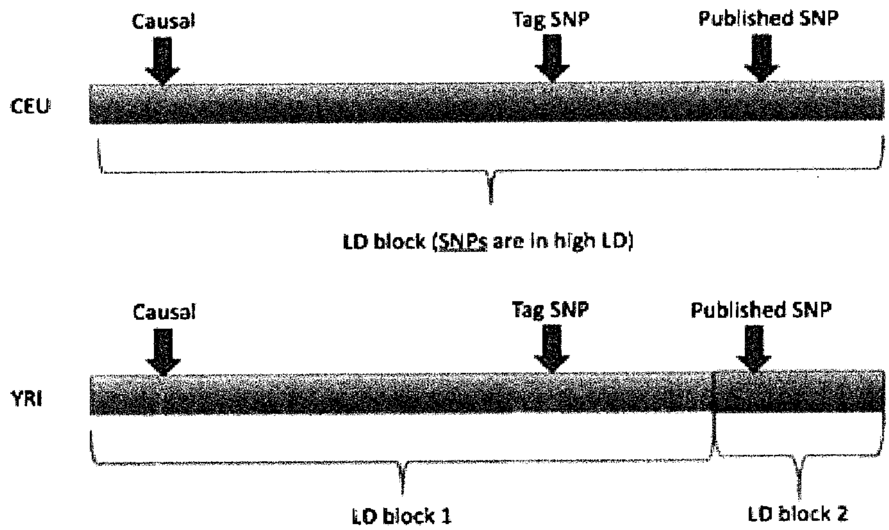
Causal
Tag SNP
Published SNP
CEU
LD block (SNPs are in high LD)
Causal
Tag SNP
Published SNP
YRI
LD block 1
LD block 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2006051763 A
- WO 2008067551 A
- US 4683202 A
- US 4683195 A
- US 4800159 A
- US 4965188 A
- US 5333675 A
- WO 8810315 A
- WO 9006995 A
- US 6410276 B
- US 4437975 A
- US 5413909 A
- US 5861245 A
- US 6124120 A
- US 6323009 B
- US 5409818 A
- US 5554517 A
- US 6063603 A
- US 5242794 A
- US 5494810 A
- US 4988617 A
- US 09854317 B
- US 16704605 A
- US 11803105 A
- US 20080131887 A


### Non-patent literature cited in the description


- **LO et al.** GABRB2 Association with Schizophrenia: Commonalities and Differences Between Ethnic Groups and Clinical Subtypes. *BIOLOGICAL PSYCHIATRY,* 16 February 2007, vol. 61 (5), 653-660
- Genome Analysis: A Laboratory Manual Series. A Laboratory Manual, Molecular Cloning: A Laboratory Manual. Cold Spring Harbor Laboratory Press, vol. I-IV
- **STRYER, L.** Biochemistry. Freeman, 1995
- **GAIT.** Oligonucleotide Synthesis: A Practical Approach. IRL Press, 1984
- **LEHNINGER.** Principles of Biochemistry. W.H. Freeman Pub,
- **BERG et al.** Biochemistry. W.H. Freeman Pub, 2002
- **SAMBROOK et al.** Molecular Cloning: A Laboratory Manual. Cold Spring Harbor Laboratory, 1989
- PCR Technology: Principles and Applications for DNA Amplification. Freeman Press, 1992
- PCR Protocols: A Guide to Methods and Applications. Academic Press, 1990
- **MATTILA et al.** *Nucleic Acids Res.,* 1991, vol. 19, 4967
- **ECKERT et al.** *PCR Methods and Applications,* 1991, vol. 1, 17
- PCR. IRL Press
- **WU ; WALLACE.** *Genomics,* 1989, vol. 4, 560
- **LANDEGREN et al.** *Science,* 1988, vol. 241, 1077
- **BARRINGER et al.** *Gene,* 1990, vol. 89, 117
- **KWOH et al.** *Proc. Natl. Acad. Sci. USA,* 1989, vol. 86, 1173-1177
- **GUATELLI et al.** *Proc. Nat. Acad. Sci. USA,* 1990, vol. 87, 1874-1878
- **DAHL et al.** *Proc. Natl. Acad. Sci,* 2004, vol. 101, 4548-4553
- **KWOK.** *Pharmocogenomics,* 2000, vol. 1, 95-100
- **OLIVIER et al.** *Nucl. Acids Res.,* 2002, vol. 30, e53
- **LIVAK et al.** *Nature Genet.,* 1995, vol. 9, 341-32
- **RANADE et al.** *Genome Res.,* 2001, vol. 11, 1262-1268
- **SANGER et al.** *Proc. Natl. Acad. Sci. USA,* 1977, vol. 74, 5463-5467
- **MARGULIES et al.** *Nature,* 2005, vol. 437, 376-380
- **EASTON et al.** *Nature,* 2007, vol. 447, 713-720
- **KLEIN et al.** *Science,* 2005, vol. 308, 385-389
- **HERBERT et al.** *Science,* 2006, vol. 312, 279-283
- **HELGADOTTIR et al.** *Science,* 2007, vol. 316, 1491-1493
- **MCPHERSON et al.** *Science,* 2007, vol. 316, 1488-1491
- **EASTON et al.** *Nature,* 2007, vol. 447, 1087-1093
- **HUNTER et al.** *Nat. Genet.,* 2007, vol. 39, 870-874

- *The International HapMap Consortium, Nature,* 2003, vol. 426, 789-796, www.hapmap.org
- *The International HapMap Consortium, Nature,* 2005, vol. 437, 1299-1320